# EUROPEAN PATENT SPECIFICATION

(11) **EP 3 561 891 B1**
(45) Date of publication and mention of the grant of the patent: **19.02.2025**
(21) Application number: 17885139.0
(22) Date of filing: 20.12.2017
(51) Int. Cl.: H10K 85/60, C09K 11/06, H10K 50/11, H10K 85/30

(54) **ORGANIC LIGHT EMITTING ELEMENT AND COMPOSITION FOR ORGANIC MATERIAL LAYER OF ORGANIC LIGHT EMITTING ELEMENT**
ORGANISCHES LICHTEMITTIERENDES ELEMENT UND ZUSAMMENSETZUNG FÜR ORGANISCHE MATERIALSCHICHT AUS ORGANISCHEM LICHTEMITTIERENDEM ELEMENT
ÉLÉMENT ÉLECTROLUMINESCENT ORGANIQUE ET COMPOSITION DESTINÉE À UNE COUCHE DE MATÉRIAU ORGANIQUE DE L'ÉLÉMENT ÉLECTROLUMINESCENT ORGANIQUE

(30) Priority: 20.12.2016 KR 20160174449
(43) Date of publication of application: 30.10.2019
(73) Proprietor: LT Materials Co., Ltd., Yongin-si, Gyeonggi-do (KR)
(72) Inventor: PARK, Geon Yu, Osan-si Gyeonggi-do 18120 (KR); KIM, Dongjun, Yongin-si Gyeonggi-do 17118 (KR); CHOI, Jin Seok, Suwon-si Gyeonggi-do 16438 (KR); CHOI, Dae Hyuk, Yongin-si Gyeonggi-do 16953 (KR); LEE, Joo Dong, Seongnam-si Gyeonggi-do 13586 (KR)
(74) Representative: Goddar, Heinz J.
(86) International application number: PCT/KR2017/015073
(87) International publication number: WO 2018/117618

(56) References cited:
- EP-A2- 3 330 342
- JP-A- 2016 149 523
- KR-A- 20140 108 637
- KR-A- 20160 045 507
- KR-A- 20160 069 934
- US-A1- 2014 008 633
- US-A1- 2014 008 643
- US-A1- 2016 072 078
- US-A1- 2016 111 657

## Description

### [Technical Field]

This application claims priority of Korean Patent Application No. 10-2016-0174449, filed with the Korean Intellectual Property Office on December 20, 2016.

The present application relates to an organic light emitting device, and a composition for an organic material layer of an organic light emitting device.

### [Background Art]

An electroluminescent device is one type of self-emissive display devices, and has an advantage of having a wide viewing angle, and a high response speed as well as having an excellent contrast.

An organic light emitting device has a structure disposing an organic thin film between two electrodes. When a voltage is applied to an organic light emitting device having such a structure, electrons and holes injected from the two electrodes bind and pair in the organic thin film, and light emits as these annihilate. The organic thin film may be formed in a single layer or a multilayer as necessary.

A material of the organic thin film may have a light emitting function as necessary. For example, as a material of the organic thin film, compounds capable of forming a light emitting layer themselves alone may be used, or compounds capable of performing a role of a host or a dopant of a host-dopant-based light emitting layer may also be used. In addition thereto, compounds capable of performing roles of hole injection, hole transfer, electron blocking, hole blocking, electron transfer, electron injection and the like may also be used as a material of the organic thin film.

US2016072078 (A1) discloses organic light emitting device comprising a light emitting layer comprising a mixture of a hole transporting and electron transporting hosts.

Development of an organic thin film material has been continuously required for enhancing performance, lifetime or efficiency of an organic light emitting device.

### [Disclosure]

### [Technical Problem]

Researches for an organic light emitting device comprising a compound capable of satisfying conditions required for materials usable in an organic light emitting device, for example, a proper energy level, electrochemical stability, thermal stability and the like, and having a chemical structure that may perform various roles required in an organic light emitting device depending on substituents have been required.

### [Technical Solution]

One embodiment of the present application provides an organic light emitting device according to the claims comprising an anode, a cathode and a light emitting layer provided between the anode and the cathode,wherein the light emitting layer comprises a hetero-cyclic compound represented by the following Chemical Formula 1 and a hetero-cyclic compound represented by the following Chemical Formula 2 at the same time.

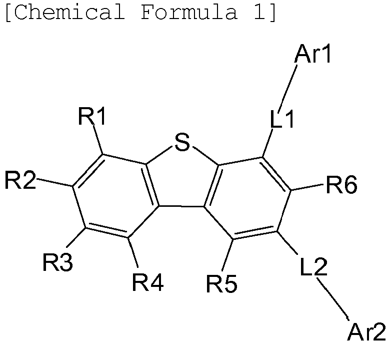

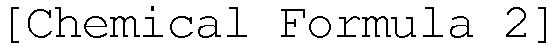

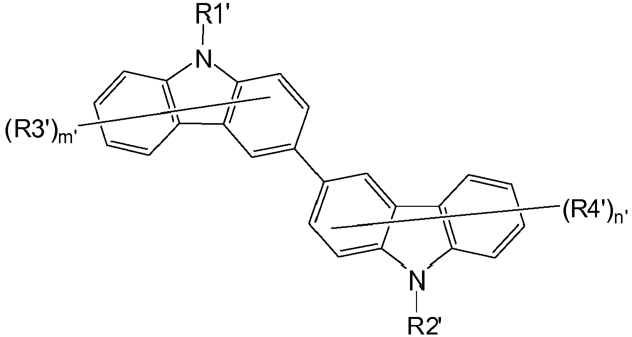

In Chemical Formulae 1 and 2,
L1 and L2 are the same as or different from each other, and each independently a direct bond, or a substituted or unsubstituted C₆ to C₆₀ arylene group,
Ar1 is a pyridine group unsubstituted or substituted with a phenyl group; a triazine group unsubstituted or substituted with one or more substituents selected from the group consisting of a phenyl group, a biphenyl group, a dibenzothiophene group and a dimethylfluorene group; a quinoline group unsubstituted or substituted with a phenyl group; a quinazoline group unsubstituted or substituted with a phenyl group or a biphenyl group; a phenanthroline group; or a benzimidazole group unsubstituted or substituted with a phenyl group.
Ar2 is represented by any one of the following Chemical Formulae 3 and 4, 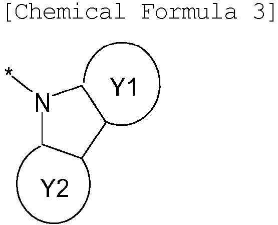 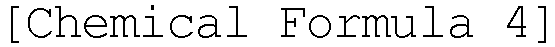 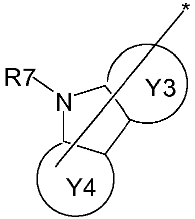
Y1 to Y4 are the same as or different from each other, and each independently a substituted or unsubstituted C₆ to C₆₀ aromatic hydrocarbon ring; or a substituted or unsubstituted C₂ to C₆₀ aromatic heteroring,
R1 to R7, R3' and R4' are the same as or different from each other, and each independently selected from the group consisting of hydrogen; deuterium; a halogen group; -CN; a substituted or unsubstituted C₁ to C₆₀ alkyl group; a substituted or unsubstituted C₂ to C₆₀ alkenyl group; a substituted or unsubstituted C₂ to C₆₀ alkynyl group; a substituted or unsubstituted C₁ to C₆₀ alkoxy group; a substituted or unsubstituted C₃ to C₆₀ cycloalkyl group; a substituted or unsubstituted C₂ to C₆₀ heterocycloalkyl group; a substituted or unsubstituted C₆ to C₆₀ aryl group; a substituted or unsubstituted C₂ to C₆₀ heteroaryl group; -SiRR'R"; - P(=O)RR'; and an amine group unsubstituted or substituted with a C₁ to C₂₀ alkyl group, a substituted or unsubstituted C₆ to C₆₀ aryl group, or a C₂ to C₆₀ heteroaryl group, or two or more groups adjacent to each other bond to each other to form a substituted or unsubstituted aliphatic or aromatic hydrocarbon ring,
R1' is represented by -(L1')p'-(Ar1')q', and R2' is represented by -(L2')r'-(Ar2')s',
L1' and L2' are each independently a direct bond, or a substituted or unsubstituted C₆ to C₆₀ arylene group,
Ar1' and Ar2' are each independently -CN; a substituted or unsubstituted C₁ to C₆₀ alkyl group; -SiRR'R"; a substituted or unsubstituted C₆ to C₆₀ aryl group, wherein Chemical Formula 3 is represented by any one of the following structural formulae: 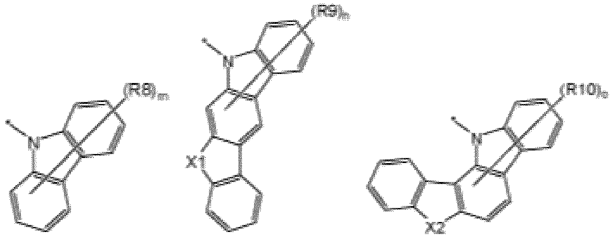 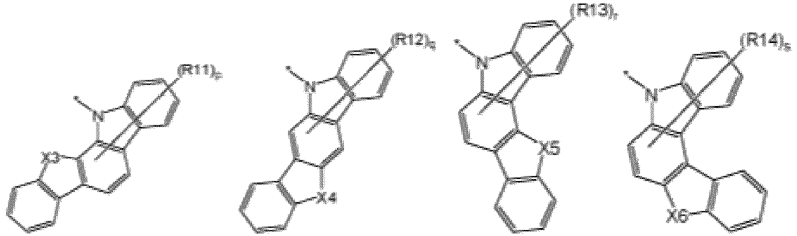
in the structural formulae, X1 to X6 are the same as or different from each other, and each independently NR, S, O or CR'R"; R8 to R14 are the same as or different from each other, and each independently selected from the group consisting of hydrogen; deuterium; a halogen group; -CN; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -SiRR'R"; -P(=O)RR'; and an amine group unsubstituted or substituted with a C1 to C20 alkyl group, a substituted or unsubstituted C6 to C60 aryl group, or a C2 to C60 heteroaryl group, or two or more groups adjacent to each other bond to each other to form a substituted or unsubstituted aliphatic or aromatic hydrocarbon ring;
R, R' and R" are the same as or different from each other, and each independently hydrogen; deuterium; -CN; a substituted or unsubstituted C₁ to C₆₀ alkyl group; a substituted or unsubstituted C₃ to C₆₀ cycloalkyl group; a substituted or unsubstituted C₆ to C₆₀ aryl group; or a substituted or unsubstituted C₂ to C₆₀ heteroaryl group,
p' and r' are each independently an integer of 0 to 4,
q' and s' are each independently an integer of 1 to 4, and
m' and n' are each independently an integer of 0 to 7,
m is an integer of 0 to 8, and n, o, p, q, r and s are each independently an integer of 0 to 6.

Another embodiment of the present application provides a composition for an organic material layer of an organic light emitting device comprising the hetero-cyclic compound represented by Chemical Formula 1 and the compound represented by Chemical Formula 2 at the same time.

### [Advantageous Effects]

A hetero-cyclic compound according to one embodiment of the present application can be used as a material of an organic material layer of an organic light emitting device. The hetero-cyclic compound can be used as a material of a hole injection layer, a hole transfer layer, a light emitting layer, an electron transfer layer, an electron injection layer, a charge generation layer or the like in an organic light emitting device. Particularly, the hetero-cyclic compound represented by Chemical Formula 1 and the compound represented by Chemical Formula 2 can be used as a material of a light emitting layer of an organic light emitting device at the same time. In addition, using the hetero-cyclic compound represented by Chemical Formula 1 and the hetero-cyclic compound represented by Chemical Formula 2 in an organic light emitting device at the same time lowers a driving voltage of the device, enhances light efficiency, and can enhance a lifetime property of the device with thermal stability of the compound.

### [Description of Drawings]

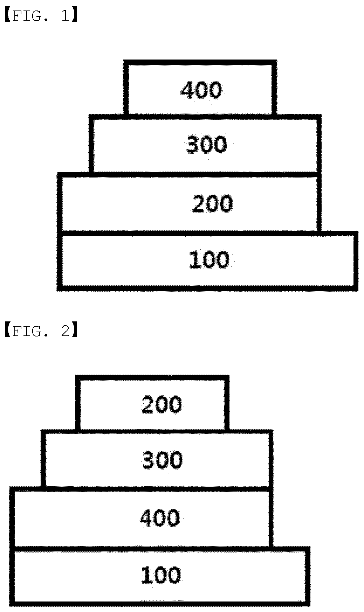

FIG. 1 to FIG. 3 are diagrams each schematically illustrating a lamination structure of an organic light emitting device according to one embodiment of the present application.

### <Reference Numeral>

100: Substrate
200: Anode
300: Organic Material Layer
301: Hole Injection Layer
302: Hole Transfer Layer
303: Light Emitting Layer
304: Hole Blocking Layer
305: Electron Transfer Layer
306: Electron Injection Layer
400: Cathode

### [Best Mode for Disclosure]

Hereinafter, the present application will be described in detail.

An organic light emitting device according to one embodiment of the present application comprises an anode, a cathode, and one or more organic material layers provided between the anode and the cathode, and one or more layers of the organic material layers comprise the hetero-cyclic compound represented by Chemical Formula 1 and the compound represented by Chemical Formula 2.

In Chemical Formulae 3 and 4, * represents a position linked to L2 of Chemical Formula 1.

According to the present application, Chemical Formula 3 is represented by any one of the following structural formulae.

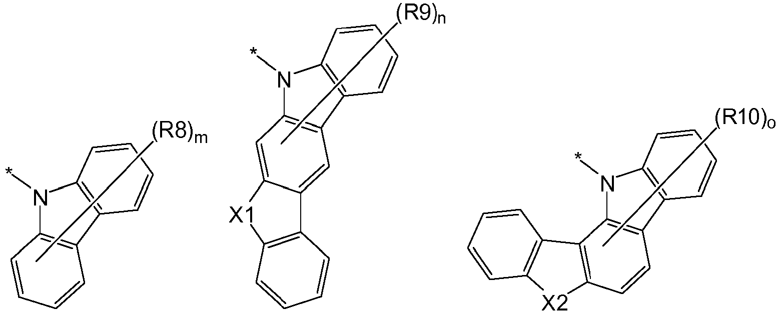

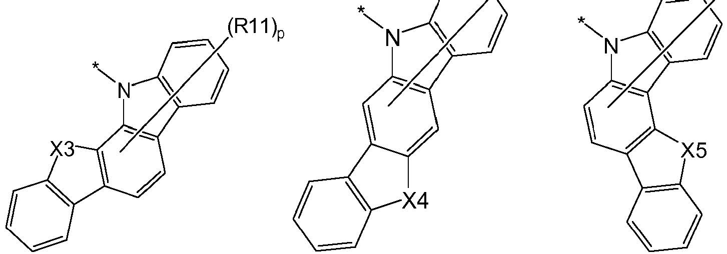

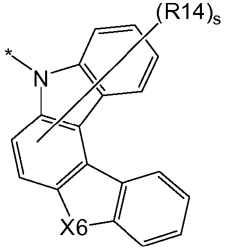

In the structural formulae, X1 to X6 are the same as or different from each other, and each independently NR, S, O or CR'R",
R8 to R14 are the same as or different from each other, and each independently selected from the group consisting of hydrogen; deuterium; a halogen group; -CN; a substituted or unsubstituted C₁ to C₆₀ alkyl group; a substituted or unsubstituted C₂ to C₆₀ alkenyl group; a substituted or unsubstituted C₂ to C₆₀ alkynyl group; a substituted or unsubstituted C₁ to C₆₀ alkoxy group; a substituted or unsubstituted C₃ to C₆₀ cycloalkyl group; a substituted or unsubstituted C₂ to C₆₀ heterocycloalkyl group; a substituted or unsubstituted C₆ to C₆₀ aryl group; a substituted or unsubstituted C₂ to C₆₀ heteroaryl group; -SiRR'R"; -P(=O)RR'; and an amine group unsubstituted or substituted with a C₁ to C₂₀ alkyl group, a substituted or unsubstituted C₆ to C₆₀ aryl group, or a C₂ to C₆₀ heteroaryl group, or two or more groups adjacent to each other bond to each other to form a substituted or unsubstituted aliphatic or aromatic hydrocarbon ring,
R, R' and R" are the same as or different from each other, and each independently hydrogen; deuterium; -CN; a substituted or unsubstituted C₁ to C₆₀ alkyl group; a substituted or unsubstituted C₃ to C₆₀ cycloalkyl group; a substituted or unsubstituted C₆ to C₆₀ aryl group; or a substituted or unsubstituted C₂ to C₆₀ heteroaryl group, and
m is an integer of 0 to 8, and n, o, p, q, r and s are each independently an integer of 0 to 6.

According to one embodiment of the present application, Chemical Formula 4 may be represented by any one of the following structural formulae.

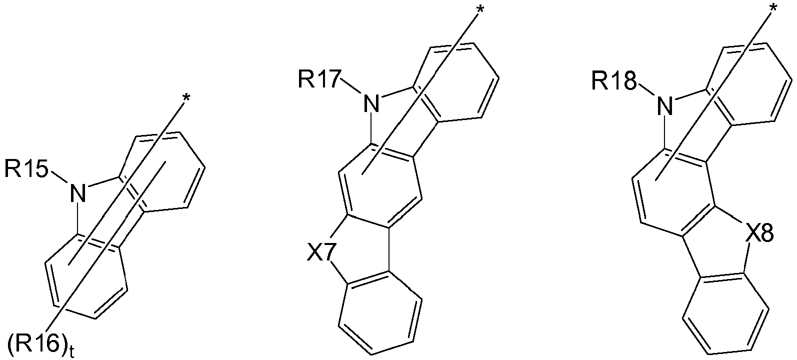

In the structural formulae, X7 and X8 are the same as or different from each other, and each independently NR, S, O or CR'R",
R15 to R18 are the same as or different from each other, and each independently selected from the group consisting of hydrogen; deuterium; a halogen group; -CN; a substituted or unsubstituted C₁ to C₆₀ alkyl group; a substituted or unsubstituted C₂ to C₆₀ alkenyl group; a substituted or unsubstituted C₂ to C₆₀ alkynyl group; a substituted or unsubstituted C₁ to C₆₀ alkoxy group; a substituted or unsubstituted C₃ to C₆₀ cycloalkyl group; a substituted or unsubstituted C₂ to C₆₀ heterocycloalkyl group; a substituted or unsubstituted C₆ to C₆₀ aryl group; a substituted or unsubstituted C₂ to C₆₀ heteroaryl group; -SiRR'R"; -P(=O)RR'; and an amine group unsubstituted or substituted with a C₁ to C₂₀ alkyl group, a substituted or unsubstituted C₆ to C₆₀ aryl group, or a C₂ to C₆₀ heteroaryl group, or two or more groups adjacent to each other bond to each other to form a substituted or unsubstituted aliphatic or aromatic hydrocarbon ring,
R, R' and R" are the same as or different from each other, and each independently hydrogen; deuterium; -CN; a substituted or unsubstituted C₁ to C₆₀ alkyl group; a substituted or unsubstituted C₃ to C₆₀ cycloalkyl group; a substituted or unsubstituted C₆ to C₆₀ aryl group; or a substituted or unsubstituted C₂ to C₆₀ heteroaryl group, and
t is an integer of 0 to 7.

According to one embodiment of the present application, L1 and L2 are the same as or different from each other, and may be each independently a direct bond, or a substituted or unsubstituted C₆ to C₆₀ arylene group.

According to another embodiment, L1 and L2 are the same as or different from each other, and may be each independently a direct bond, or a substituted or unsubstituted C₆ to C₄₀ arylene group.

According to another embodiment, L1 and L2 are the same as or different from each other, and may be each independently a direct bond, or a substituted or unsubstituted C₆ to C₂₀ arylene group.

According to another embodiment, L1 and L2 are the same as or different from each other, and may be each independently a direct bond, or a C₆ to C₂₀ arylene group.

According to another embodiment, L1 and L2 are the same as or different from each other, and may be each independently a direct bond or a monocyclic arylene group.

According to another embodiment, L1 and L2 are the same as or different from each other, and may be each independently a direct bond or a phenylene group.

Ar1 is a pyridine group unsubstituted or substituted with a phenyl group; a triazine group unsubstituted or substituted with one or more substituents selected from the group consisting of a phenyl group, a biphenyl group, a dibenzothiophene group and a dimethylfluorene group; a quinoline group unsubstituted or substituted with a phenyl group; a quinazoline group unsubstituted or substituted with a phenyl group or a biphenyl group; a phenanthroline group; or a benzimidazole group unsubstituted or substituted with a phenyl group.

According to another embodiment, Ar1 may be a pyridine group unsubstituted or substituted with a phenyl group; a triazine group unsubstituted or substituted with one or more substituents selected from the group consisting of a phenyl group, a biphenyl group, a dibenzothiophene group and a dimethylfluorene group; a quinoline group unsubstituted or substituted with a phenyl group; a quinazoline group unsubstituted or substituted with a phenyl group or a biphenyl group; a phenanthroline group; or a benzimidazole group unsubstituted or substituted with a phenyl group.

In one embodiment of the present application, Ar1 may be substituted again with NO₂ or CN.

According to one embodiment of the present application, Y1 to Y4 are the same as or different from each other, and may be each independently a substituted or unsubstituted C₆ to C₆₀ aromatic hydrocarbon ring; or a substituted or unsubstituted C₂ to C₆₀ aromatic heteroring.

According to another embodiment, Y1 to Y4 are the same as or different from each other, and may be each independently a substituted or unsubstituted C₆ to C₄₀ aromatic hydrocarbon ring; or a substituted or unsubstituted C₂ to C₄₀ aromatic heteroring.

According to another embodiment, Y1 to Y4 are the same as or different from each other, and may be each independently a C₆ to C₄₀ aromatic hydrocarbon ring unsubstituted or substituted with one or more substituents selected from the group consisting of a C₆ to C₄₀ aryl group and a C₂ to C₄₀ heteroaryl group; or a C₂ to C₄₀ aromatic heteroring unsubstituted or substituted with a C₆ to C₄₀ aryl group.

According to another embodiment, Y1 to Y4 are the same as or different from each other, and may be each independently a C₆ to C₄₀ aromatic hydrocarbon ring unsubstituted or substituted with one or more substituents selected from the group consisting of a phenyl group, a biphenyl group, a naphthyl group, a carbazole group, a dibenzothiophene group and a dibenzofuran group; or a C₂ to C₄₀ aromatic heteroring unsubstituted or substituted with a phenyl group.

According to another embodiment, Y1 to Y4 are the same as or different from each other, and may be each independently a benzene ring unsubstituted or substituted with one or more substituents selected from the group consisting of a phenyl group, a biphenyl group, a naphthyl group, a carbazole group, a dibenzothiophene group and a dibenzofuran group; a dimethylfluorene group; a carbazole group unsubstituted or substituted with a phenyl group; a dibenzothiophene group; or a dibenzofuran group.

In one embodiment of the present application, Y1 to Y4 may be unsubstituted or substituted again with a phenyl group.

In one embodiment of the present application, R7 may be a substituted or unsubstituted C₆ to C₃₀ aryl group.

In another embodiment, R7 may be a C₆ to C₃₀ aryl group.

In another embodiment, R7 may be a phenyl group.

According to one embodiment of the present application, Chemical Formula 1 may be represented by any one of the following Chemical Formulae 5 to 10.

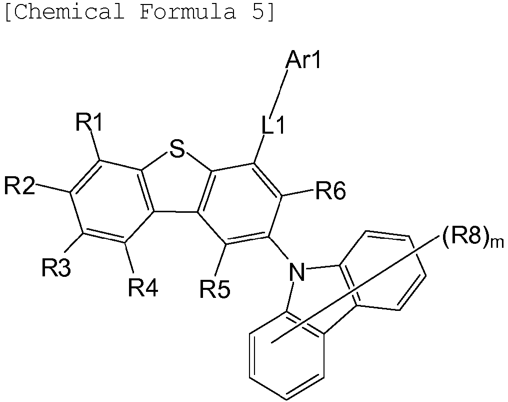

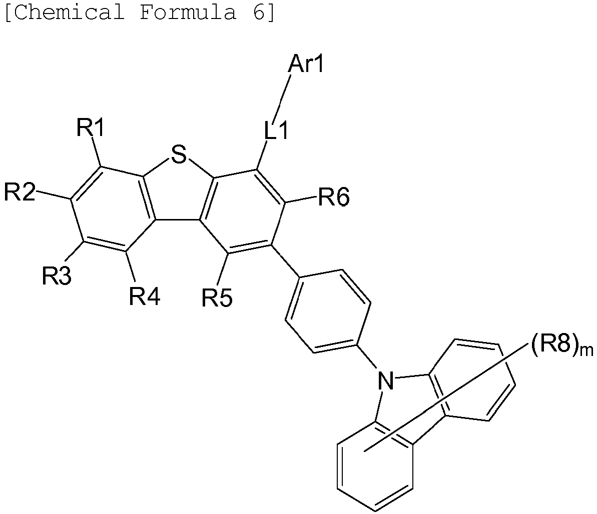

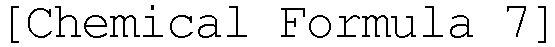

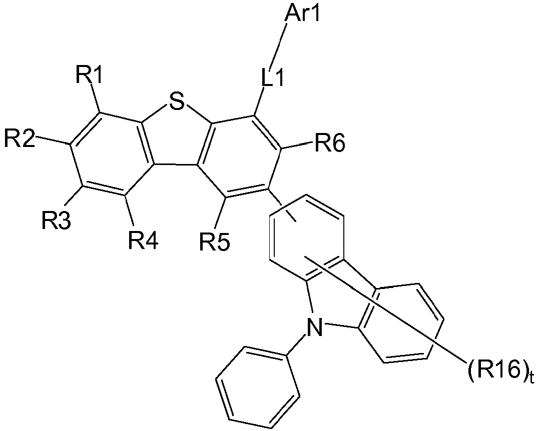

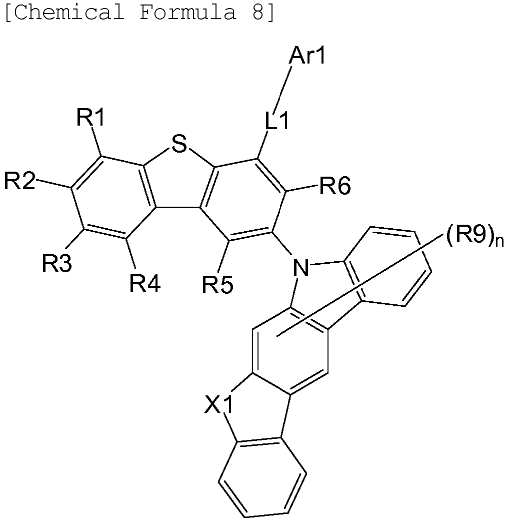

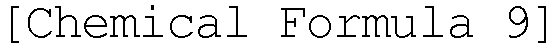

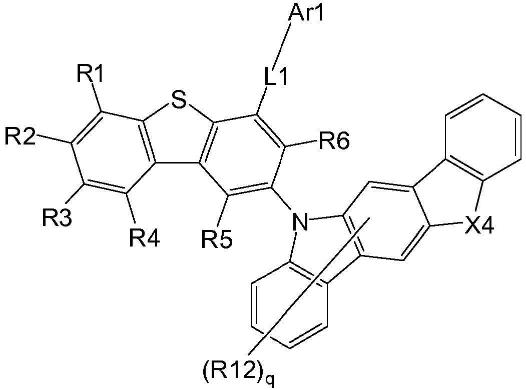

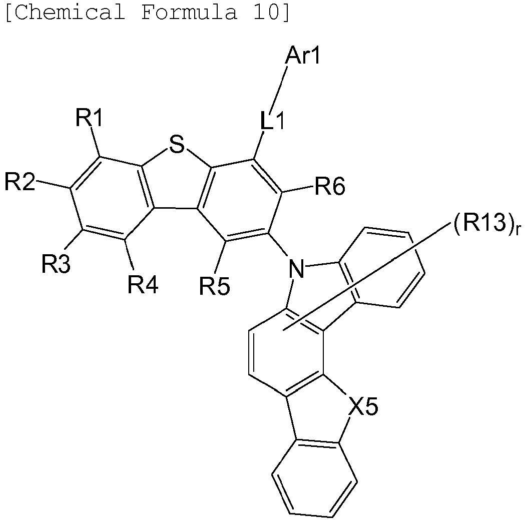

In Chemical Formulae 5 to 10, R1 to R6, R8, R9, R12, R13, R16, L1, Ar1, X1, X4, X5, m, n, q, r, and t have the same definitions as in Chemical Formula 1 and the structural formulae.

In one embodiment of the present application, R1 to R6 of Chemical Formula 1 may be each independently hydrogen or deuterium.

In one embodiment of the present application, R8 to R18 of the structural formulae may be each independently hydrogen; deuterium; a substituted or unsubstituted C₆ to C₆₀ aryl group; or a substituted or unsubstituted C₂ to C₆₀ heteroaryl group.

In another embodiment, R8 to R18 of the structural formulae may be each independently hydrogen; a substituted or unsubstituted C₆ to C₄₀ aryl group; or a substituted or unsubstituted C₂ to C₄₀ heteroaryl group.

In another embodiment, R8 to R18 of the structural formulae may be each independently hydrogen; a C₆ to C₄₀ aryl group; or a C₂ to C₄₀ heteroaryl group unsubstituted or substituted with a C₆ to C₄₀ aryl group.

In another embodiment, R8 to R18 of the structural formulae may be each independently hydrogen; a phenyl group; a biphenyl group; a naphthyl group; a carbazole group unsubstituted or substituted with a phenyl group; a dibenzothiophene group; or a dibenzofuran group.

In one embodiment of the present application, R, R' and R" of Chemical Formula 1 are the same as or different from each other, and each independently hydrogen; a substituted or unsubstituted C₁ to C₆₀ alkyl group; or a substituted or unsubstituted C₆ to C₆₀ aryl group.

In another embodiment, R, R' and R" of Chemical Formula 1 are the same as or different from each other, and each independently a substituted or unsubstituted C₁ to C₃₀ alkyl group; or a substituted or unsubstituted C₆ to C₃₀ aryl group.

In another embodiment, R, R' and R" of Chemical Formula 1 are the same as or different from each other, and each independently a C₁ to C₃₀ alkyl group; or a C₆ to C₃₀ aryl group.

In another embodiment, R, R' and R" of Chemical Formula 1 are the same as or different from each other, and each independently a methyl group; or a phenyl group.

In one embodiment of the present application, R3' and R4' of Chemical Formula 2 may be each independently hydrogen or deuterium.

In one embodiment of the present application, Ar1' and Ar2' of Chemical Formula 2 may be each independently -CN; a substituted or unsubstituted C₁ to C₆₀ alkyl group; -SiRR'R"; or a substituted or unsubstituted C₆ to C₆₀ aryl group.

In another embodiment, Ar1' and Ar2' of Chemical Formula 2 may be each independently -CN; a substituted or unsubstituted C₁ to C₄₀ alkyl group; -SiRR'R"; or a substituted or unsubstituted C₆ to C₄₀ aryl group.

In another embodiment, Ar1' and Ar2' of Chemical Formula 2 may be each independently -CN; a C₁ to C₄₀ alkyl group; - SiRR'R"; or a C₆ to C₄₀ aryl group.

In another embodiment, Ar1' and Ar2' of Chemical Formula 2 may be each independently -CN; a methyl group; a phenyl group; a biphenyl group; a naphthyl group; a dimethylfluorene group; a fluorene group; a diphenylfluorene group; a spirobifluorene group; a triphenylene group; or -SiRR'R".

In one embodiment of the present application, L1' and L2' may be each independently a direct bond, or a substituted or unsubstituted C₆ to C₆₀ arylene group.

In another embodiment, L1' and L2' may be each independently a direct bond or a C₆ to C₆₀ arylene group.

In another embodiment, L1' and L2' may be each independently a direct bond or a C₆ to C₄₀ arylene group.

In another embodiment, L1' and L2' may be each independently a direct bond; a phenylene group; or a biphenylene group.

In the present application, substituents of Chemical Formulae 1 and 2 may be more specifically described as follows.

In the present specification, the term "substituted or unsubstituted" means being substituted with one or more substituents selected from the group consisting of deuterium; a halogen group; -CN; a C₁ to C₆₀ alkyl group; a C₂ to C₆₀ alkenyl group; a C₂ to C₆₀ alkynyl group; a C₃ to C₆₀ cycloalkyl group; a C₂ to C₆₀ heterocycloalkyl group; a C₆ to C₆₀ aryl group; a C₂ to C₆₀ heteroaryl group; -SiRR'R"; -P(=O)RR'; a C₁ to C₂₀ alkylamine group; a C₆ to C₆₀ arylamine group; and a C₂ to C₆₀ heteroarylamine group, or being unsubstituted, or being substituted with a substituent bonding two or more of the above-mentioned substituents, or being substituted, or being substituted with a substituent linking two or more substituents selected from among the above-mentioned substituents, or being unsubstituted. For example, "a substituent linking two or more substituents" may comprise a biphenyl group. In other words, a biphenyl group may be an aryl group, or may be interpreted as a substituent linking two phenyl groups. The additional substituents may be further substituted. R, R' and R" are the same as or different from each other, and each independently hydrogen; deuterium; -CN; a substituted or unsubstituted C₁ to C₆₀ alkyl group; a substituted or unsubstituted C₃ to C₆₀ cycloalkyl group; a substituted or unsubstituted C₆ to C₆₀ aryl group; or a substituted or unsubstituted C₂ to C₆₀ heteroaryl group.

According to one embodiment of the present application, the "substituted or unsubstituted" means being substituted with one or more substituents selected from the group consisting of deuterium, a halogen group, -CN, SiRR'R", P(=O)RR', a C₁ to C₂₀ linear or branched alkyl group, a C₆ to C₆₀ aryl group and a C₂ to C₆₀ heteroaryl group, or being unsubstituted, and
R, R' and R" are the same as or different from each other, and each independently hydrogen; deuterium; -CN; a C₁ to C₆₀ alkyl group unsubstituted or substituted with deuterium, a halogen group, -CN, a C₁ to C₂₀ alkyl group, a C₆ to C₆₀ aryl group and a C₂ to C₆₀ heteroaryl group; a C₃ to C₆₀ cycloalkyl group unsubstituted or substituted with deuterium, halogen, - CN, a C₁ to C₂₀ alkyl group, a C₆ to C₆₀ aryl group and a C₂ to C₆₀ heteroaryl group; a C₆ to C₆₀ aryl group unsubstituted or substituted with deuterium, halogen, -CN, a C₁ to C₂₀ alkyl group, a C₆ to C₆₀ aryl group and a C₂ to C₆₀ heteroaryl group; or a C₂ to C₆₀ heteroaryl group unsubstituted or substituted with deuterium, halogen, -CN, a C₁ to C₂₀ alkyl group, a C₆ to C₆₀ aryl group and a C₂ to C₆₀ heteroaryl group.

The term "substituted" means a hydrogen atom bonding to a carbon atom of a compound is changed to another substituent, and the position of substitution is not limited as long as it is a position at which the hydrogen atom is substituted, that is, a position at which a substituent can substitute, and when two or more substituents substitute, the two or more substituents may be the same as or different from each other.

In the present specification, the halogen may be fluorine, chlorine, bromine or iodine.

In the present specification, the alkyl group comprises linear or branched having 1 to 60 carbon atoms, and may be further substituted with other substituents. The number of carbon atoms of the alkyl group may be from 1 to 60, specifically from 1 to 40 and more specifically from 1 to 20. Specific examples thereof may comprise a methyl group, an ethyl group, a propyl group, an n-propyl group, an isopropyl group, a butyl group, an n-butyl group, an isobutyl group, a tert-butyl group, a sec-butyl group, a 1-methyl-butyl group, a 1-ethylbutyl group, a pentyl group, an n-pentyl group, an isopentyl group, a neopentyl group, a tert-pentyl group, a hexyl group, an n-hexyl group, a 1-methylpentyl group, a 2-methylpentyl group, a 4-methyl-2-pentyl group, a 3,3-dimethylbutyl group, a 2-ethylbutyl group, a heptyl group, an n-heptyl group, a 1-methylhexyl group, a cyclopentylmethyl group, a cyclohexylmethyl group, an octyl group, an n-octyl group, a tert-octyl group, a 1-methylheptyl group, a 2-ethylhexyl group, a 2-propylpentyl group, an n-nonyl group, a 2,2-dimethylheptyl group, a 1-ethyl-propyl group, a 1,1-dimethyl-propyl group, an isohexyl group, a 2-methylpentyl group, a 4-methylhexyl group, a 5-methylhexyl group and the like, but are not limited thereto.

In the present specification, the alkenyl group comprises linear or branched having 2 to 60 carbon atoms, and may be further substituted with other substituents. The number of carbon atoms of the alkenyl group may be from 2 to 60, specifically from 2 to 40 and more specifically from 2 to 20. Specific examples thereof may comprise a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, a styrenyl group and the like, but are not limited thereto.

In the present specification, the alkynyl group comprises linear or branched having 2 to 60 carbon atoms, and may be further substituted with other substituents. The number of carbon atoms of the alkynyl group may be from 2 to 60, specifically from 2 to 40 and more specifically from 2 to 20.

In the present specification, the cycloalkyl group comprises monocyclic or multicyclic having 3 to 60 carbon atoms, and may be further substituted with other substituents. Herein, the multicyclic means a group in which the cycloalkyl group is directly linked to or fused with other cyclic groups. Herein, the other cyclic groups may be a cycloalkyl group, but may also be different types of cyclic groups such as a heterocycloalkyl group, an aryl group and a heteroaryl group. The number of carbon groups of the cycloalkyl group may be from 3 to 60, specifically from 3 to 40 and more specifically from 5 to 20. Specific examples thereof may comprise a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, a cyclooctyl group and the like, but are not limited thereto.

In the present specification, the heterocycloalkyl group comprises O, S, Se, N or Si as a heteroatom, comprises monocyclic or multicyclic having 2 to 60 carbon atoms, and may be further substituted with other substituents. Herein, the multicyclic means a group in which the heterocycloalkyl group is directly linked to or fused with other cyclic groups. Herein, the other cyclic groups may be a heterocycloalkyl group, but may also be different types of cyclic groups such as a cycloalkyl group, an aryl group and a heteroaryl group. The number of carbon atoms of the heterocycloalkyl group may be from 2 to 60, specifically from 2 to 40 and more specifically from 3 to 20.

In the present specification, the aryl group comprises monocyclic or multicyclic having 6 to 60 carbon atoms, and may be further substituted with other substituents. Herein, the multicyclic means a group in which the aryl group is directly linked to or fused with other cyclic groups. Herein, the other cyclic groups may be an aryl group, but may also be different types of cyclic groups such as a cycloalkyl group, a heterocycloalkyl group and a heteroaryl group. The aryl group comprises a spiro group. The number of carbon atoms of the aryl group may be from 6 to 60, specifically from 6 to 40 and more specifically from 6 to 25. Specific examples of the aryl group may comprise a phenyl group, a biphenyl group, a triphenyl group, a naphthyl group, an anthryl group, a chrysenyl group, a phenanthrenyl group, a perylenyl group, a fluoranthenyl group, a triphenylenyl group, a phenalenyl group, a pyrenyl group, a tetracenyl group, a pentacenyl group, a fluorenyl group, an indenyl group, an acenaphthylenyl group, a benzofluorenyl group, a spirobifluorenyl group, a 2,3-dihydro-1H-indenyl group, a fused ring thereof, and the like, but are not limited thereto.

In the present specification, the spiro group is a group comprising a spiro structure, and may have 15 to 60 carbon atoms. For example, the spiro group may comprise a structure in which a 2,3-dihydro-1H-indene group or a cyclohexane group spiro bonds to a fluorenyl group. Specifically, the following spiro group may comprise any one of the groups having the following structural formulae.

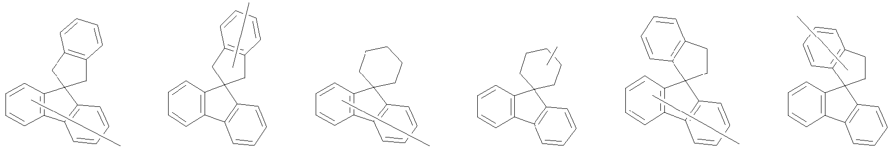

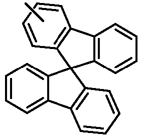

In the present specification, the heteroaryl group comprises O, S, Se, N or Si as a heteroatom, comprises monocyclic or multicyclic having 2 to 60 carbon atoms, and may be further substituted with other substituents. Herein, the multicyclic means a group in which the heteroaryl group is directly linked to or fused with other cyclic groups. Herein, the other cyclic groups may be a heteroaryl group, but may also be different types of cyclic groups such as a cycloalkyl group, a heterocycloalkyl group and an aryl group. The number of carbon atoms of the heteroaryl group may be from 2 to 60, specifically from 2 to 40 and more specifically from 3 to 25. Specific examples of the heteroaryl group may comprise a pyridyl group, a pyrrolyl group, a pyrimidyl group, a pyridazinyl group, a furanyl group, a thiophene group, an imidazolyl group, a pyrazolyl group, an oxazolyl group, an isoxazolyl group, a thiazolyl group, an isothiazolyl group, a triazolyl group, a furazanyl group, an oxadiazolyl group, a thiadiazolyl group, a dithiazolyl group, a tetrazolyl group, a pyranyl group, a thiopyranyl group, a diazinyl group, an oxazinyl group, a thiazinyl group, a dioxynyl group, a triazinyl group, a tetrazinyl group, a quinolyl group, an isoquinolyl group, a quinazolinyl group, an isoquinazolinyl group, a qninozolinyl group, a naphthyridyl group, an acridinyl group, a phenanthridinyl group, an imidazopyridinyl group, a diazanaphthalenyl group, a triazaindene group, an indolyl group, an indolizinyl group, a benzothiazolyl group, a benzoxazolyl group, a benzimidazolyl group, a benzothiophene group, a benzofuran group, a dibenzothiophene group, a dibenzofuran group, a carbazolyl group, a benzocarbazolyl group, a dibenzocarbazolyl group, a phenazinyl group, a dibenzosilole group, spirobi(dibenzosilole), a dihydrophenazinyl group, a phenoxazinyl group, a phenanthridyl group, an imidazopyridinyl group, a thienyl group, an indolo[2,3-a]carbazolyl group, an indolo[2,3-b]carbazolyl group, an indolinyl group, a 10,11-dihydro-dibenzo[b,f]azepine group, a 9,10-dihydroacridinyl group, a phenanthrazinyl group, a phenothiazinyl group, a phthalazinyl group, a naphthyridinyl group, a phenanthrolinyl group, a benzo[c][1,2,5]thiadiazolyl group, a 5,10-dihydrobenzo[b,e][1,4]azasilinyl, a pyrazolo[1,5-c]quinazolinyl group, a pyrido[1,2-b]indazolyl group, a pyrido[1,2-a]imidazo[1,2-e]indolinyl group, a 5,11-dihydroindeno[1,2-b]carbazolyl group and the like, but are not limited thereto.

In the present specification, the amine group may be selected from the group consisting of a monoalkylamine group; a monoarylamine group; a monoheteroarylamine group; -NH₂; a dialkylamine group; a diarylamine group; a diheteroarylamine group; an alkylarylamine group; an alkylheteroarylamine group; and an arylheteroarylamine group, and although not particularly limited thereto, the number of carbon atoms is preferably from 1 to 30. Specific examples of the amine group may comprise a methylamine group, a dimethylamine group, an ethylamine group, a diethylamine group, a phenylamine group, a naphthylamine group, a biphenylamine group, a dibiphenylamine group, an anthracenylamine group, a 9-methyl-anthracenylamine group, a diphenylamine group, a phenylnaphthylamine group, a ditolylamine group, a phenyltolylamine group, a triphenylamine group, a biphenylnaphthylamine group, a phenylbiphenylamine group, a biphenylfluorenylamine group, a phenyltriphenylenylamine group, a biphenyltriphenylenylamine group and the like, but are not limited thereto.

In the present specification, the arylene group means the aryl group having two bonding sites, that is, a divalent group. Descriptions on the aryl group provided above may be applied thereto except for each being a divalent. In addition, the heteroarylene group means the heteroaryl group having two bonding sites, that is, a divalent group. Descriptions on the heteroaryl group provided above may be applied thereto except for each being a divalent.

According to one embodiment of the present application, Chemical Formula 1 may be represented by any one of the following compounds, but is not limited thereto.

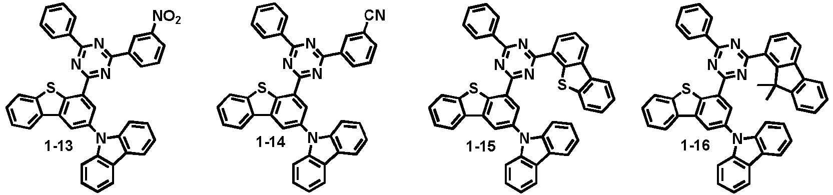

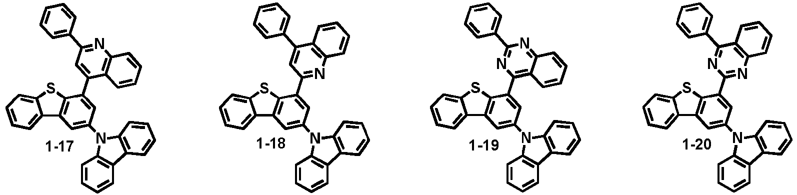

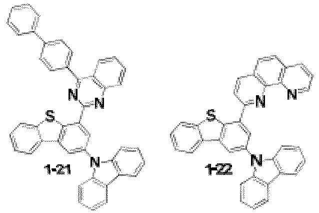

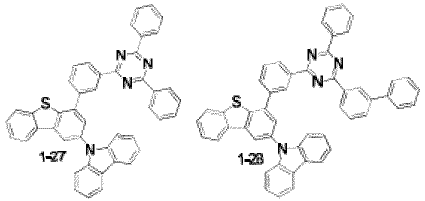

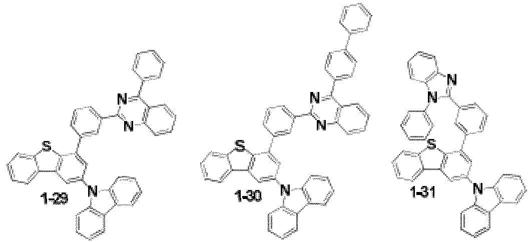

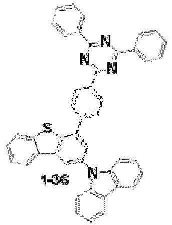

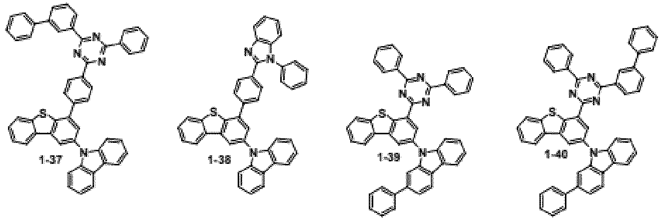

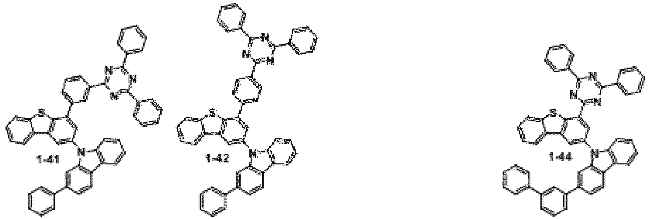

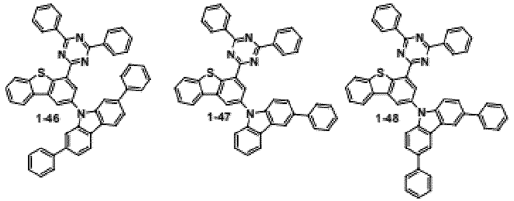

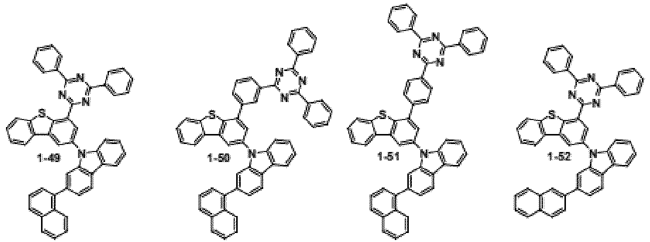

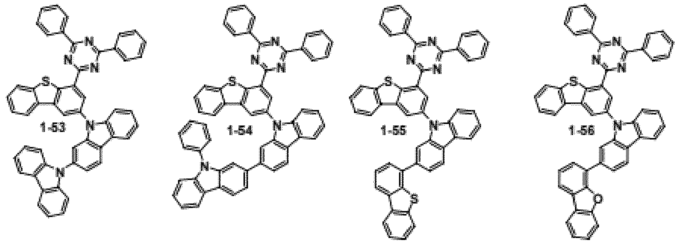

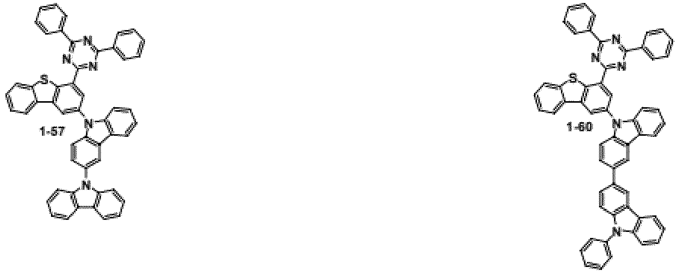

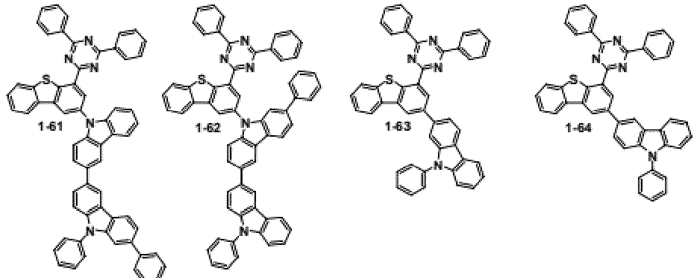

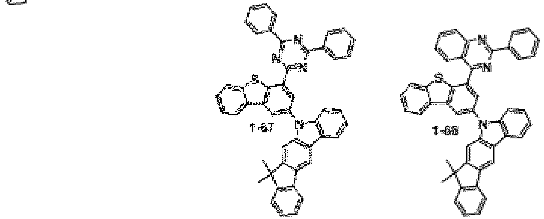

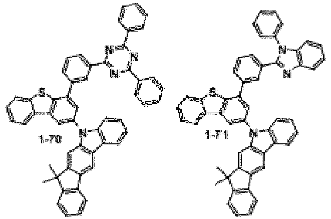

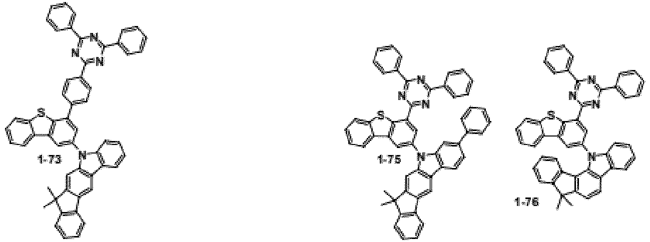

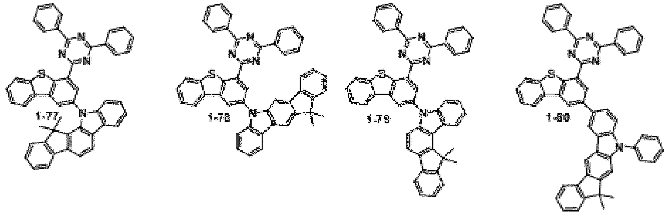

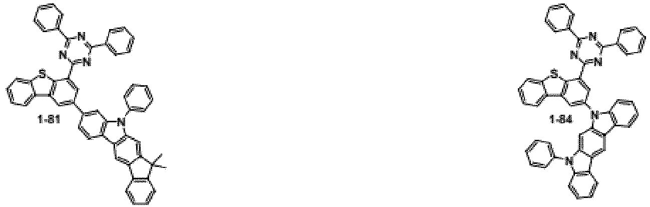

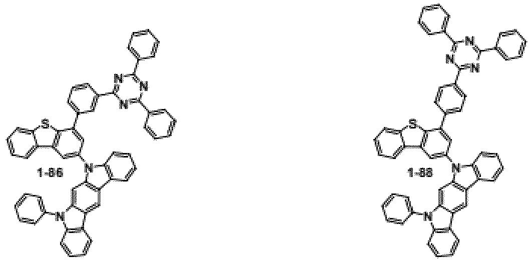

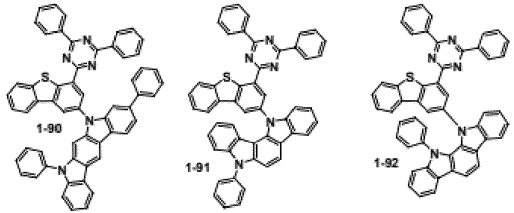

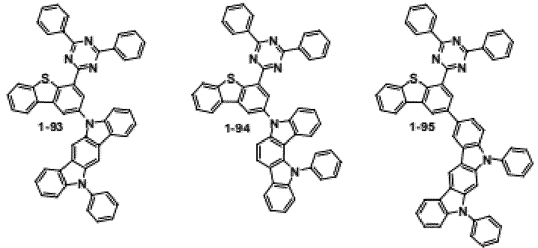

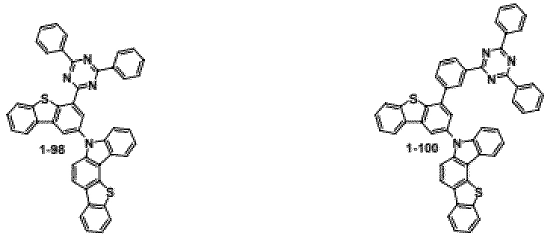

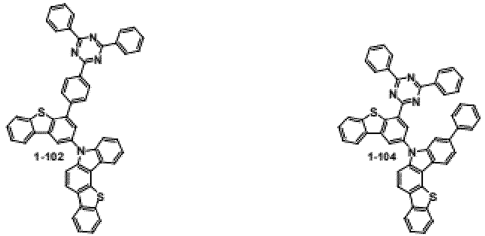

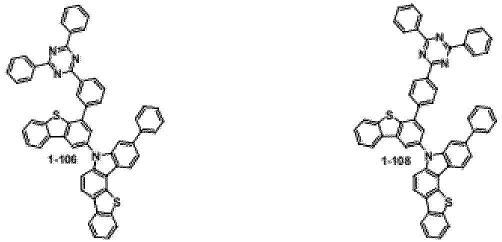

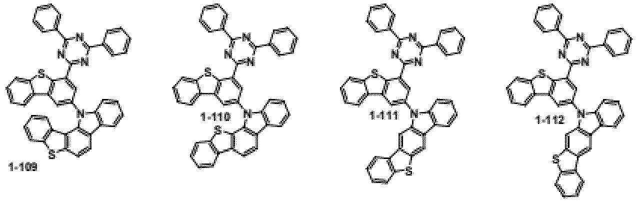

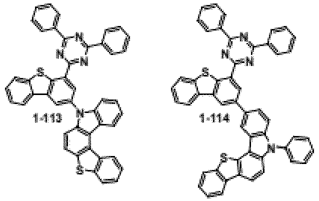

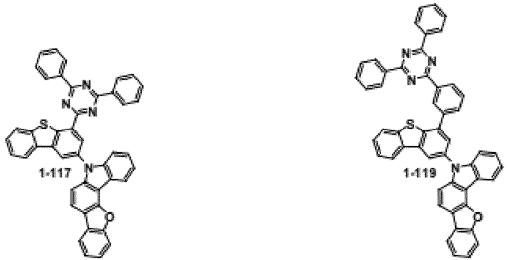

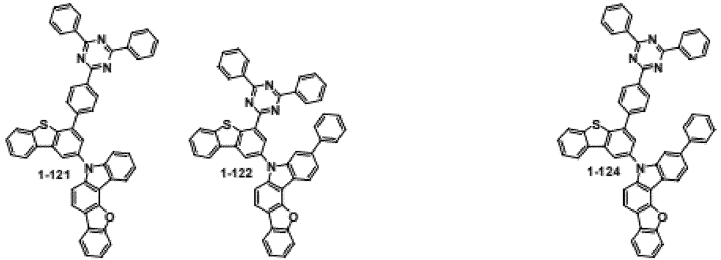

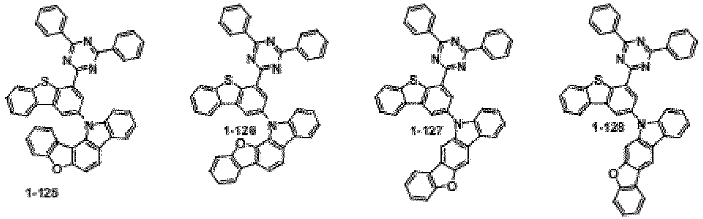

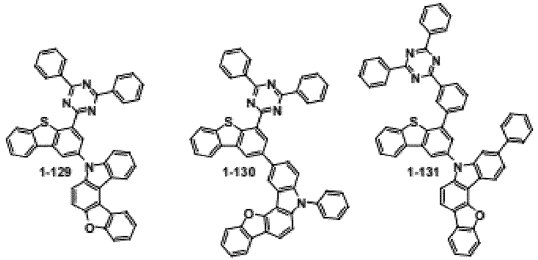

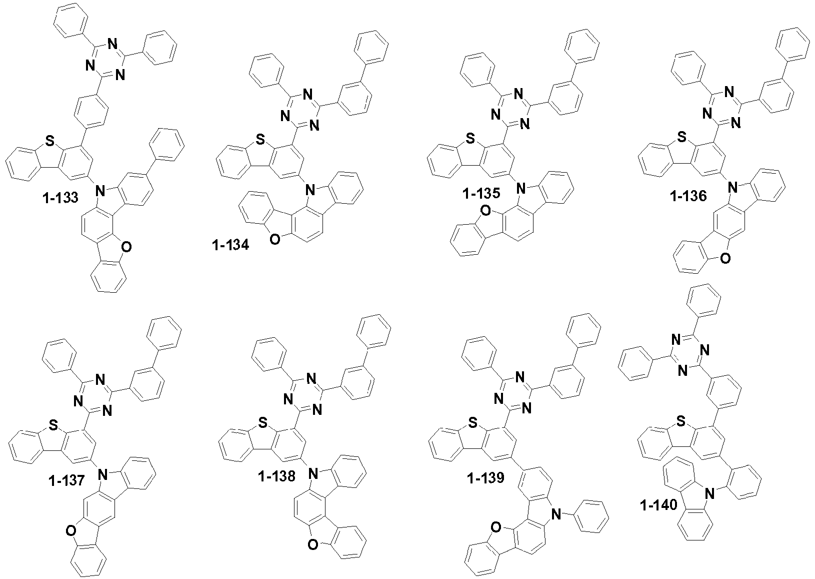

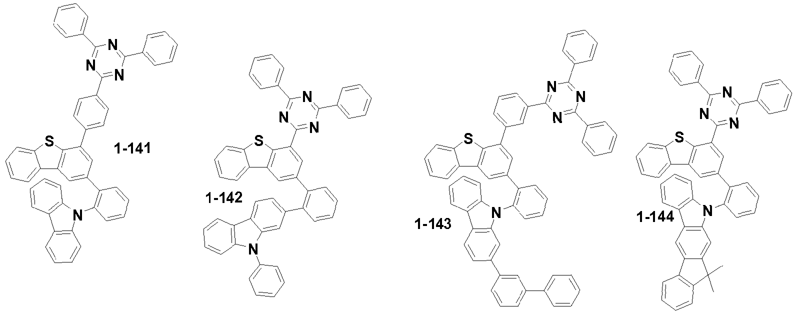

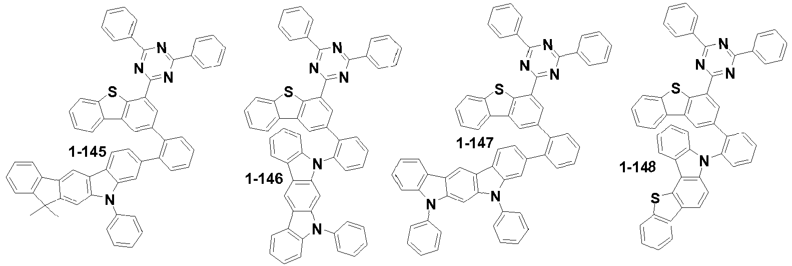

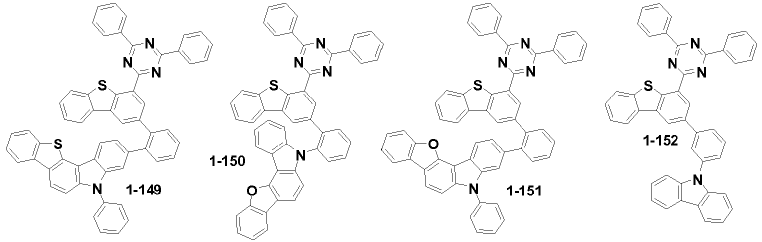

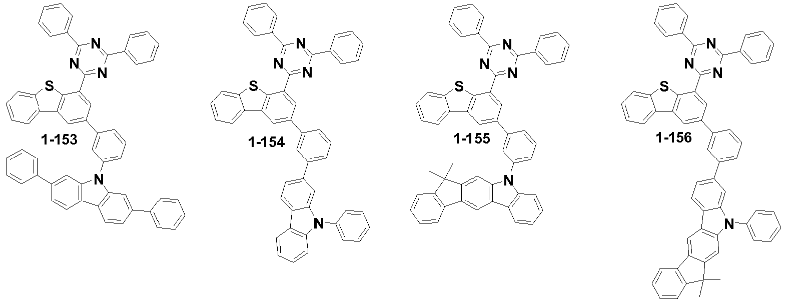

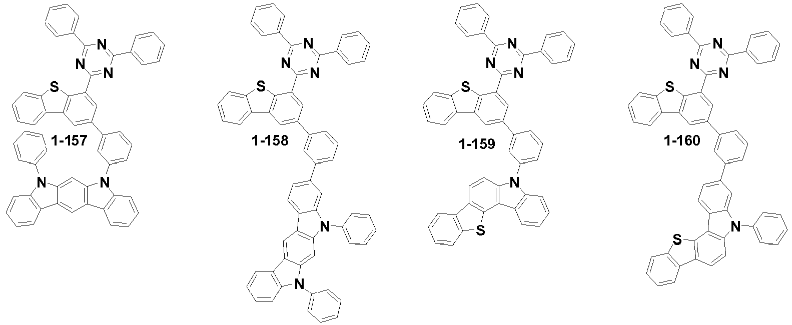

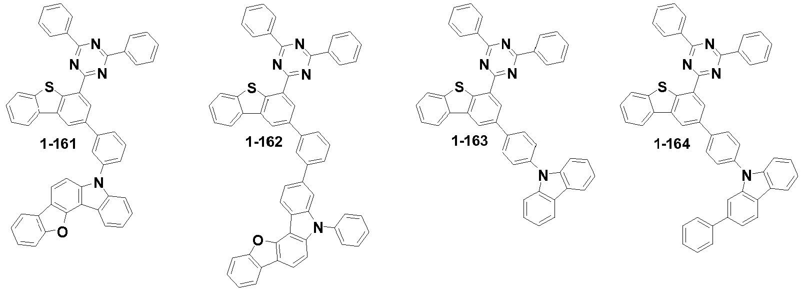

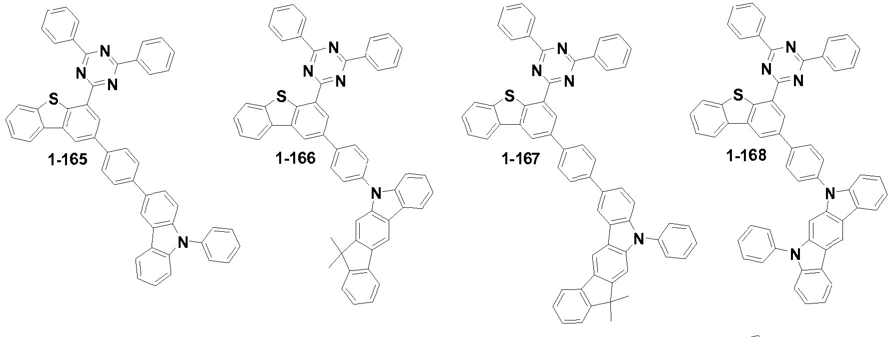

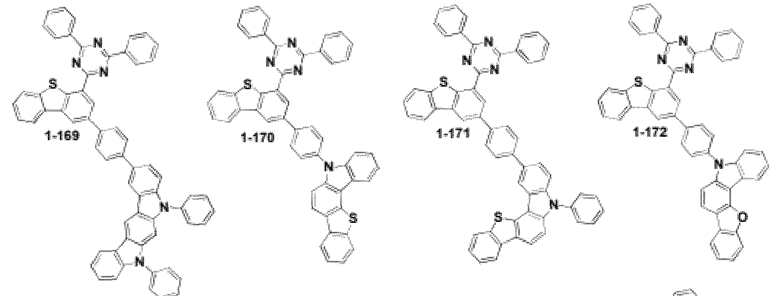

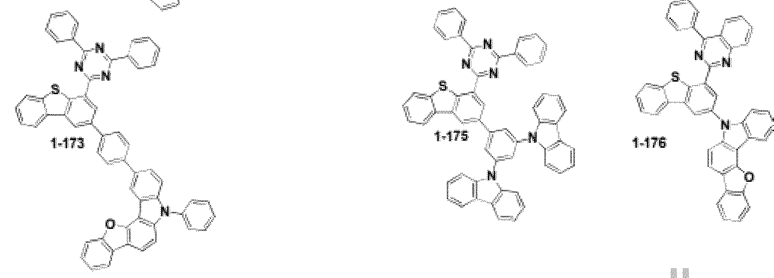

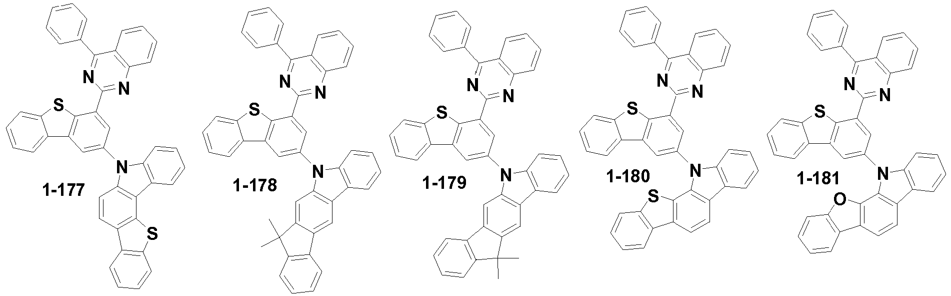

According to one embodiment of the present application, Chemical Formula 2 may be represented by any one of the following compounds, but is not limited thereto.

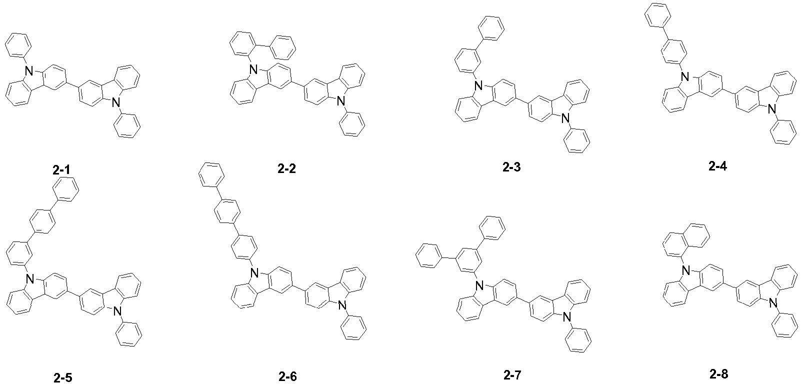

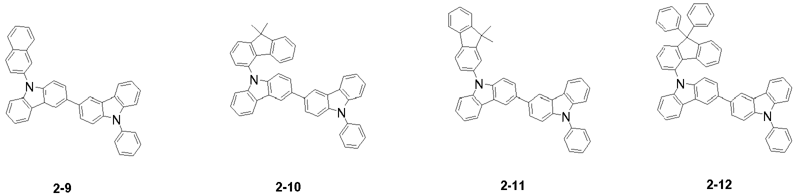

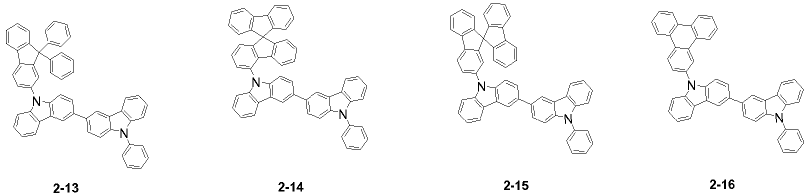

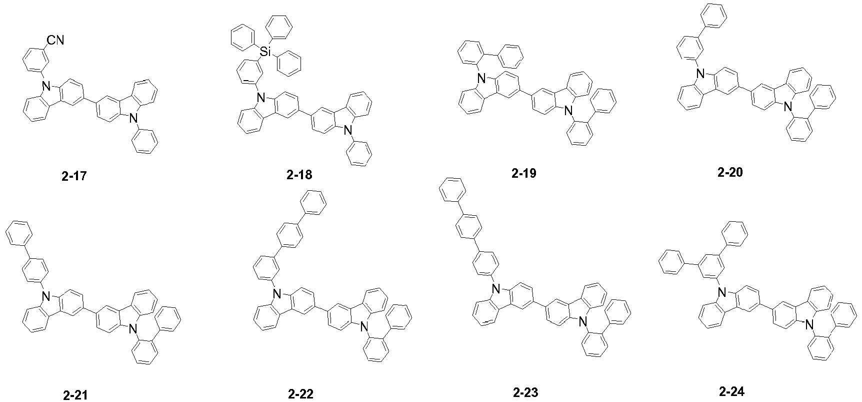

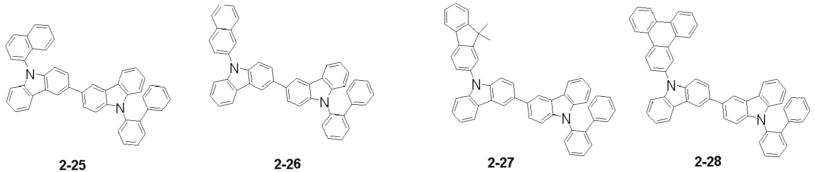

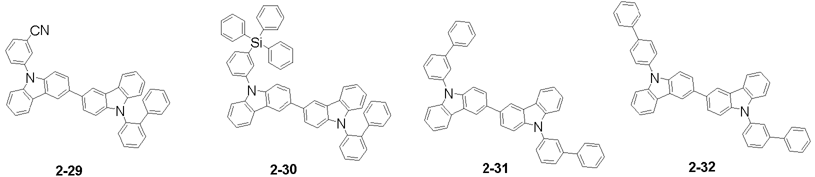

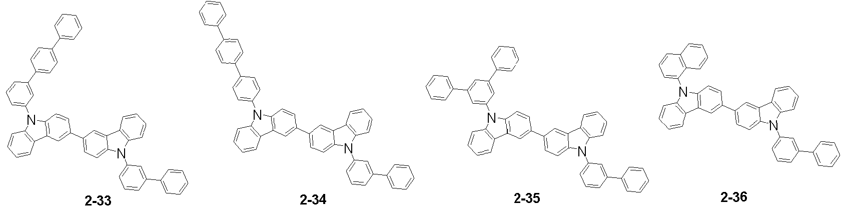

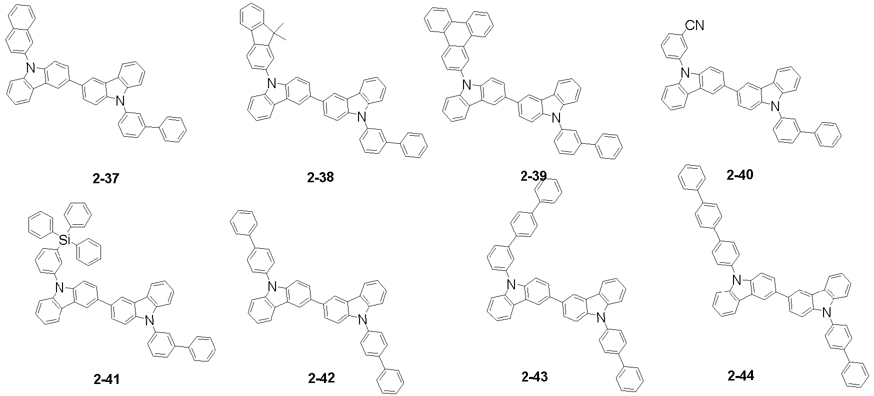

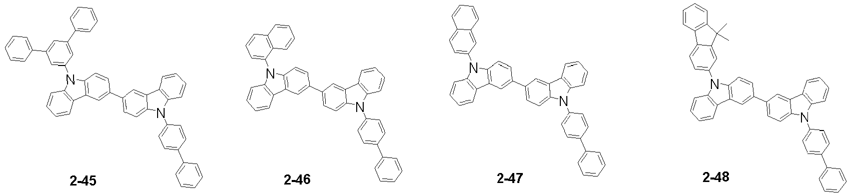

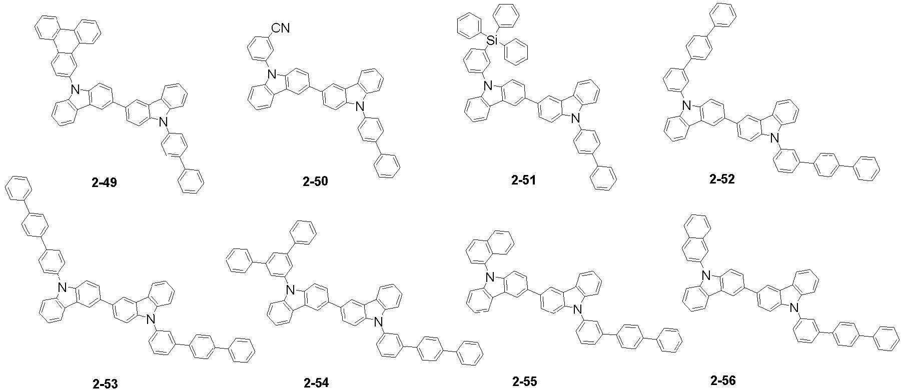

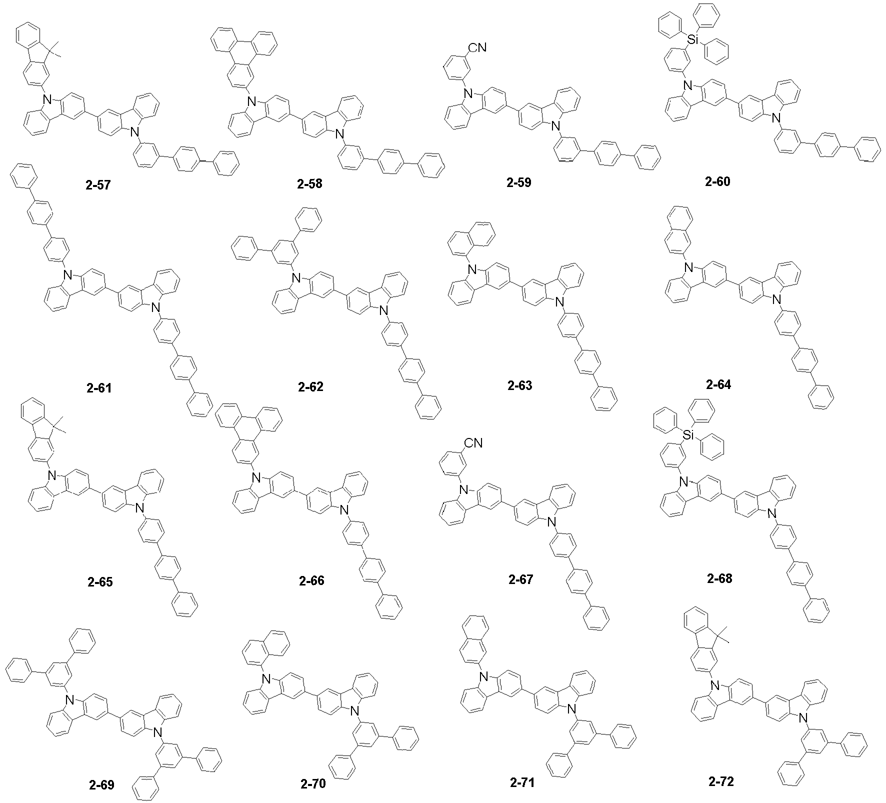

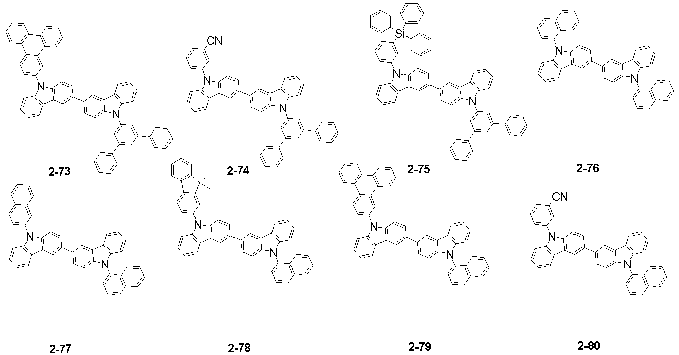

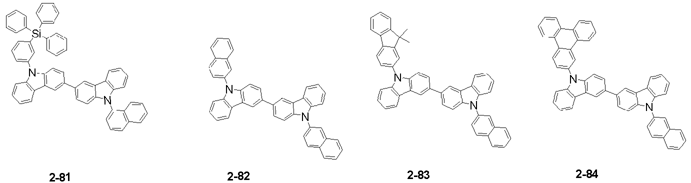

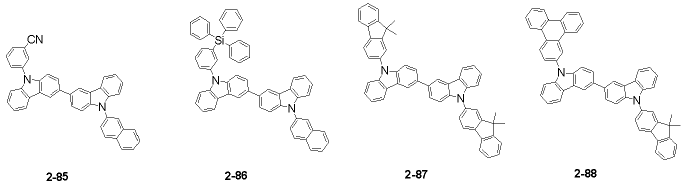

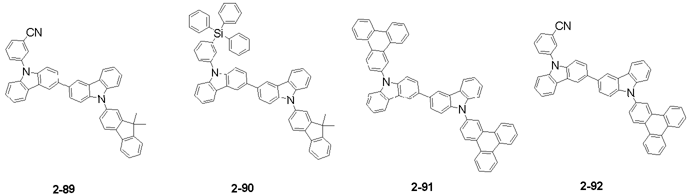

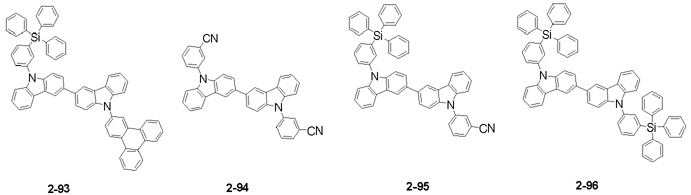

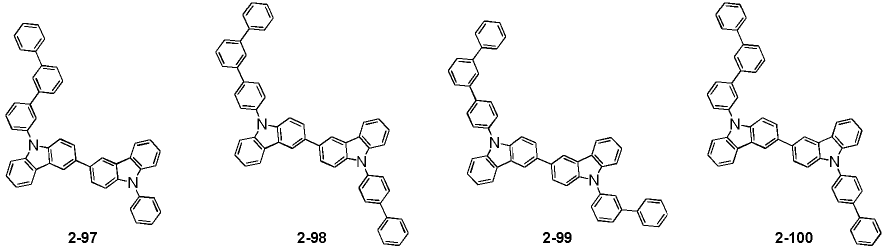

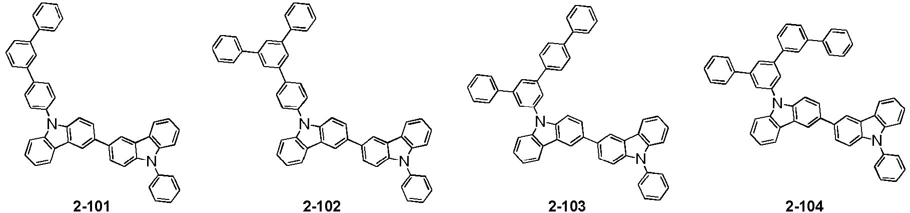

In addition, by introducing various substituents to the structures of Chemical Formulae 1 and 2, compounds having unique properties of the introduced substituents may be synthesized. For example, by introducing substituents normally used as hole injection layer materials, hole transfer layer materials, light emitting layer materials, electron transfer layer materials and charge generation layer materials used for manufacturing an organic light emitting device to the core structure, materials satisfying conditions required for each organic material layer may be synthesized.

In addition, by introducing various substituents to the structures of Chemical Formulae 1 and 2, the energy band gap may be finely controlled, and meanwhile, properties at interfaces between organic materials are enhanced, and material applications may become diverse.

Meanwhile, the hetero-cyclic compound has excellent thermal stability with a high glass transition temperature (Tg). Such an increase in the thermal stability becomes an important factor in providing driving stability to a device.

The hetero-cyclic compound according to one embodiment of the present application may be prepared through a multistep chemical reaction. Some intermediate compounds are prepared first, and the compound of Chemical Formula 1 or 2 may be prepared from the intermediate compounds. More specifically, the hetero-cyclic compound according to one embodiment of the present application may be prepared based on preparation examples to be described below.

Another embodiment of the present application provides a composition for an organic material layer of an organic light emitting device comprising the hetero-cyclic compound represented by Chemical Formula 1 and the compound represented by Chemical Formula 2 at the same time.

Specific descriptions on the hetero-cyclic compound represented by Chemical Formula 1, and the compound represented by Chemical Formula 2 are the same as the descriptions provided above.

A weight ratio of the hetero-cyclic compound represented by Chemical Formula 1:the compound represented by Chemical Formula 2 in the composition may be from 1:10 to 10:1, from 1:8 to 8:1, from 1:5 to 5:1, or from 1:2 to 2:1, but is not limited thereto.

The composition may be used when forming an organic material of an organic light emitting device, and particularly, may be more preferably used when forming a host of a light emitting layer.

The composition has a form of simply mixing two or more compounds, and materials in a powder state may be mixed before forming an organic material layer of an organic light emitting device, or compounds in a liquid state may be mixed at an appropriate temperature or higher. The composition is in a solid state at a temperature below a melting point of each material, and may maintain a liquid state when adjusting a temperature.

The organic light emitting device according to one embodiment of the present application may be manufactured using common organic light emitting device manufacturing methods and materials except that one or more organic material layers are formed using the hetero-cyclic compound represented by Chemical Formula 1 and the hetero-cyclic compound represented by Chemical Formula 2 described above.

The hetero-cyclic compound represented by Chemical Formula 1 and the hetero-cyclic compound represented by Chemical Formula 2 may be formed into an organic material layer through a solution coating method as well as a vacuum deposition method when manufacturing the organic light emitting device. Herein, the solution coating method means spin coating, dip coating, inkjet printing, screen printing, a spray method, roll coating and the like, but is not limited thereto.

Specifically, the organic light emitting device according to one embodiment of the present application comprises an anode, a cathode, and one or more organic material layers provided between the anode and the cathode, wherein one or more layers of the organic material layers comprise the hetero-cyclic compound represented by Chemical Formula 1 and the hetero-cyclic compound represented by Chemical Formula 2.

FIGs. 1 to 3 illustrate a lamination order of electrodes and organic material layers of an organic light emitting device according to one embodiment of the present application. However, the scope of the present application is not limited to these diagrams, and structures of organic light emitting devices known in the art may also be used in the present application.

FIG. 1 illustrates an organic light emitting device in which an anode (200), an organic material layer (300) and a cathode (400) are consecutively laminated on a substrate (100). However, the structure is not limited to such a structure, and as illustrated in FIG. 2, an organic light emitting device in which a cathode, an organic material layer and an anode are consecutively laminated on a substrate may also be obtained.

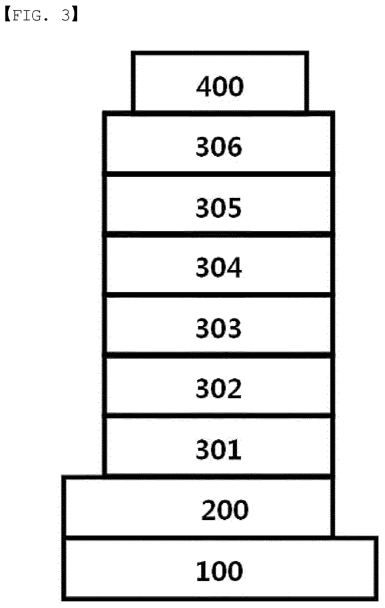

FIG. 3 illustrates a case of the organic material layer being a multilayer. The organic light emitting device according to FIG. 3 comprises a hole injection layer (301), a hole transfer layer (302), a light emitting layer (303), a hole blocking layer (304), an electron transfer layer (305) and an electron injection layer (306). However, the scope of the present application is not limited to such a lamination structure, and as necessary, other layers except the light emitting layer may not be included, and other necessary functional layers may be further included.

The organic light emitting device according to the present specification may be manufactured using materials and methods known in the art except that one or more layers of the organic material layers comprise the hetero-cyclic compound represented by Chemical Formula 1 and the hetero-cyclic compound represented by Chemical Formula 2.

The organic material layer comprising the hetero-cyclic compound represented by Chemical Formula 1 and the hetero-cyclic compound represented by Chemical Formula 2 may further comprise other materials as necessary.

The hetero-cyclic compound represented by Chemical Formula 1 and the hetero-cyclic compound represented by Chemical Formula 2 may be used as a material of a charge generation layer in the organic light emitting device.

The hetero-cyclic compound represented by Chemical Formula 1 and the hetero-cyclic compound represented by Chemical Formula 2 may be used as a material of an electron transfer layer, a hole blocking layer, a light emitting layer or the like in the organic light emitting device. As one example, the hetero-cyclic compound represented by Chemical Formula 1 and the hetero-cyclic compound represented by Chemical Formula 2 may be used as a material of an electron transfer layer, a hole transfer layer or a light emitting layer in the organic light emitting device.

In addition, the hetero-cyclic compound represented by Chemical Formula 1 and the compound represented by Chemical Formula 2 may be used as a material of a light emitting layer in the organic light emitting device. As one example, the hetero-cyclic compound represented by Chemical Formula 1 and the compound represented by Chemical Formula 2 may be used as a phosphorescent host material of a light emitting layer in the organic light emitting device.

In the organic light emitting device according to one embodiment of the present application, materials other than the hetero-cyclic compound of Chemical Formula 1 and the hetero-cyclic compound represented by Chemical Formula 2 are illustrated below, however, these are for illustrative purposes only and not for limiting the scope of the present application, and may be replaced by materials known in the art.

As the anode material, materials having relatively large work function may be used, and transparent conductive oxides, metals, conductive polymers or the like may be used. Specific examples of the anode material comprise metals such as vanadium, chromium, copper, zinc and gold, or alloys thereof; metal oxides such as zinc oxide, indium oxide, indium tin oxide (ITO) and indium zinc oxide (IZO); combinations of metals and oxides such as ZnO:Al or SnO₂:Sb; conductive polymers such as poly(3-methylthiophene), poly[3,4-(ethylene-1,2-dioxy)thiophene] (PEDOT), polypyrrole and polyaniline, and the like, but are not limited thereto.

As the cathode material, materials having relatively small work function may be used, and metals, metal oxides, conductive polymers or the like may be used. Specific examples of the cathode material comprise metals such as magnesium, calcium, sodium, potassium, titanium, indium, yttrium, lithium, gadolinium, aluminum, silver, tin and lead, or alloys thereof; multilayer structure materials such as LiF/Al or LiO₂/Al, and the like, but are not limited thereto.

As the hole injection material, known hole injection materials may be used, and for example, phthalocyanine compounds such as copper phthalocyanine disclosed in US Patent No. 4,356,429, or starburst-type amine derivatives such as tris(4-carbazoyl-9-ylphenyl)amine (TCTA), 4,4',4"-tri[phenyl(m-tolyl)amino]triphenylamine (m-MTDATA) or 1,3,5-tris[4-(3-methylphenylphenylamino)phenyl]benzene (m-MTDAPB) described in the literature [Advanced Material, 6, p.677 (1994)], polyaniline/dodecylbenzene sulfonic acid, poly(3,4-ethylenedioxythiophene)/poly(4-styrenesulfonate), polyaniline/camphor sulfonic acid or polyaniline/poly(4-styrene-sulfonate) that are conductive polymers having solubility, and the like, may be used.

As the hole transfer material, pyrazoline derivatives, arylamine-based derivatives, stilbene derivatives, triphenyldiamine derivatives and the like may be used, and low molecular or high molecular materials may also be used.

As the electron transfer material, metal complexes of oxadiazole derivatives, anthraquinodimethane and derivatives thereof, benzoquinone and derivatives thereof, naphthoquinone and derivatives thereof, anthraquinone and derivatives thereof, tetracyanoanthraquinodimethane and derivatives thereof, fluorenone derivatives, diphenyldicyanoethylene and derivatives thereof, diphenoquinone derivatives, 8-hydroxyquinoline and derivatives thereof, and the like, may be used, and high molecular materials may also be used as well as low molecular materials.

As examples of the electron injection material, LiF is typically used in the art, however, the present application is not limited thereto.

As the light emitting material, red, green or blue light emitting materials may be used, and as necessary, two or more light emitting materials may be mixed and used. Herein, two or more light emitting materials may be used by being deposited as individual sources of supply or by being premixed and deposited as one source of supply. In addition, fluorescent materials may also be used as the light emitting material, however, phosphorescent materials may also be used. As the light emitting material, materials emitting light by bonding electrons and holes injected from an anode and a cathode, respectively, may be used alone, however, materials having a host material and a dopant material involved in light emission together may also be used.

When mixing light emitting material hosts, same series hosts may be mixed, or different series hosts may be mixed. For example, any two or more types of materials among n-type host materials or p-type host materials may be selected, and used as a host material of a light emitting layer.

The organic light emitting device according to one embodiment of the present application may be a top-emission type, a bottom-emission type or a dual-emission type depending on the materials used.

The hetero-cyclic compound according to one embodiment of the present application may also be used in an organic electronic device comprising an organic solar cell, an organic photo conductor, an organic transistor and the like under a similar principle used in the organic light emitting device.

### [Mode for Disclosure]

Hereinafter, the present specification will be described in more detail with reference to examples, however, these are for illustrative purposes only, and the scope of the present application is not limited thereto.

### <Example>

### <Preparation Example 1> Preparation of Compound 1-39

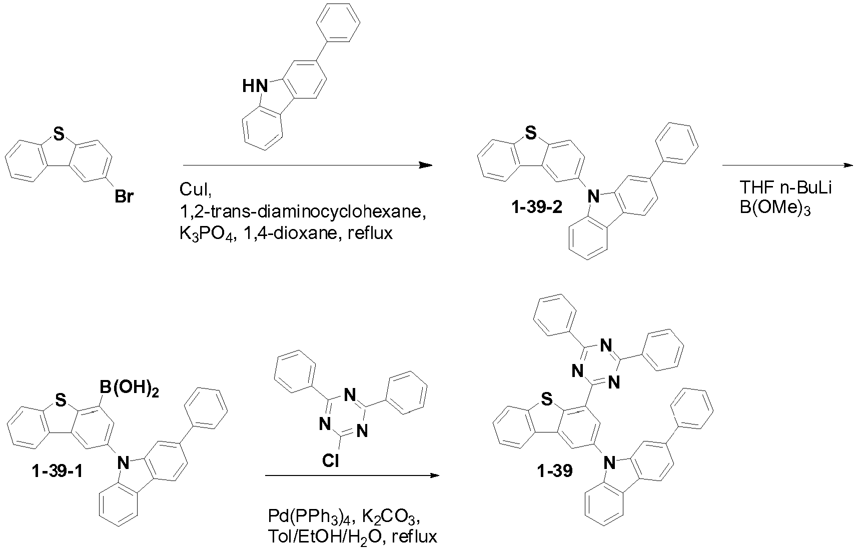

### 1) Preparation of Compound 1-39-2

After dissolving 2-bromodibenzo[b,d]thiophene (5.0 g, 19.0 mM), 2-phenyl-9H-carbazole (3.8 g, 15.8 mM), CuI (3.0 g, 15.8 mM), trans-1,2-diaminocyclohexane (1.9 mL, 15.8 mM) and K₃PO₄ (3.3 g, 31.6 mM) in 1,4-oxane (100 mL), the result was refluxed for 24 hours. After the reaction was completed, the result was extracted using distilled water and DCM at room temperature, the organic layer was dried with MgSO₄, and then the solvent was removed using a rotary evaporator. The reaction material was purified using column chromatography (DCM:Hex=1:3), and recrystallized using methanol to obtain target Compound 1-39-2 (5.7 g, 85%).

### 2) Preparation of Compound 1-39-1

2.5 M n-BuLi (7.4 mL, 18.6 mM) was added dropwise to a mixed solution of Compound 1-39-2 (6.1 g, 14.3 mM) and THF (100 mL) at -78°C, and the result was stirred for 1 hour at room temperature. To the reaction mixture, trimethyl borate (B(OMe)₃, 4.8 mL, 42.9 mM) was added dropwise, and the result was stirred for 2 hours at room temperature. After the reaction was completed, the result was extracted using distilled water and DCM at room temperature, the organic layer was dried with MgSO₄, and then the solvent was removed using a rotary evaporator. The reaction material was purified using column chromatography (DCM:MeOH=100:3), and recrystallized using DCM to obtain target Compound 1-39-1 (4.7 g, 70%).

### 3) Preparation of Compound 1-39

After dissolving Compound 1-39-1 (8.9 g, 19.0 mM), 2-chloro-4,6-diphenyl-1,3,5-triazine (5.1 g, 19.0 mM), Pd(PPh₃)₄ (1.1 g, 0.95 mM) and K₂CO₃ (5.2 g, 38.0 mM) in toluene/EtOH/H₂O (100/20/20 mL), the result was refluxed for 12 hours. After the reaction was completed, the result was extracted using distilled water and DCM at room temperature, the organic layer was dried with MgSO₄, and then the solvent was removed using a rotary evaporator. The reaction material was purified using column chromatography (DCM:Hex=1:3), and recrystallized using methanol to obtain target Compound 1-39 (8.7 g, 70%).

Target Compound A was synthesized in the same manner as in Preparation Example 1 except that Intermediate A of the following Table 1 was used instead of 2-phenyl-9H-carbazole, and Intermediate B of the following Table 1 was used instead of 2-chloro-4,6-diphenyl-1,3,5-triazine.

**[Table 1]**

| Compo und Numbe r | Intermediate A | Intermediate B | Target Compound A | Total Yield |
|---|---|---|---|---|
| 1-40 | 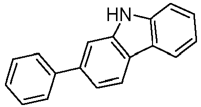 | 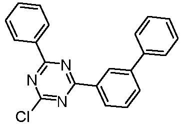 | 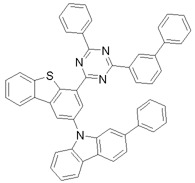 | 41% |
| 1-67 | 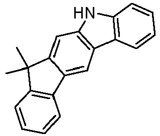 | 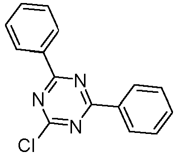 | 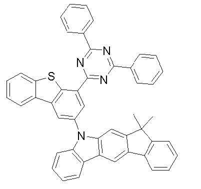 | 42% |
| 1-46 | 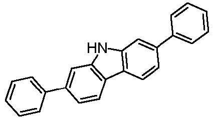 | | 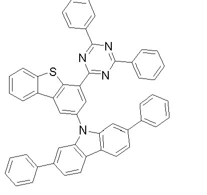 | 43% |
| 1-41 | 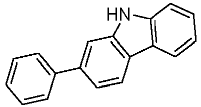 | 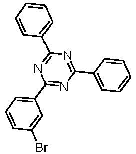 | 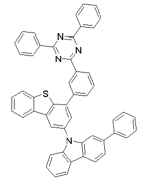 | 41% |

### <Preparation Example 2> Synthesis of Compound 2-3

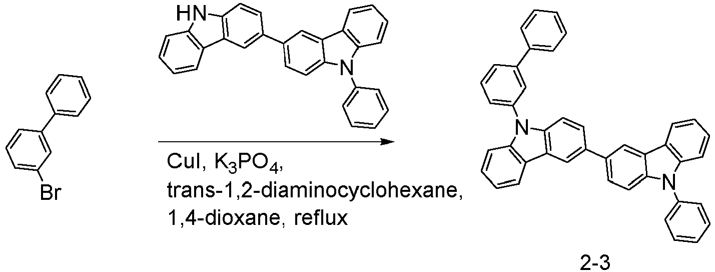

### 1) Preparation of Compound 2-3

After dissolving 3-bromo-1,1'-biphenyl (3.7 g, 15.8 mM), 9-phenyl-9H,9'H-3,3'-bicarbazole (6.5 g, 15.8 mM), CuI (3.0 g, 15.8 mM), trans-1,2-diaminocyclohexane (1.9 mL, 15.8 mM) and K₃PO₄ (3.3 g, 31.6 mM) in 1,4-oxane (100 mL), the result was refluxed for 24 hours. After the reaction was completed, the result was extracted using distilled water and DCM at room temperature, the organic layer was dried with MgSO₄, and then the solvent was removed using a rotary evaporator. The reaction material was purified using column chromatography (DCM:Hex=1:3), and recrystallized using methanol to obtain target Compound 2-3 (7.5 g, 85%).

Target Compound A was synthesized in the same manner as in Preparation Example 2 except that Intermediate A of the following Table 2 was used instead of 3-bromo-1,1'-biphenyl, and Intermediate B of the following Table 2 was used instead of 9-phenyl-9H,9'H-3,3'-bicarbazole.

**[Table 2]**

| Compo und Numbe r | Intermediate A | Intermediate B | Target Compound A | Total Yield |
|---|---|---|---|---|
| 2-4 | 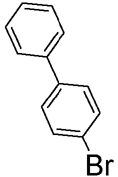 | 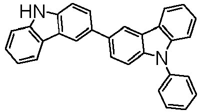 | 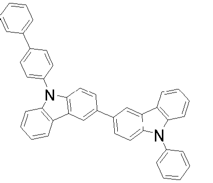 | 83% |
| 2-7 | 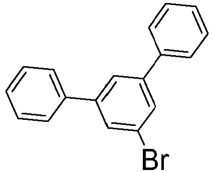 | | 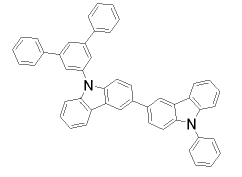 | 84% |
| 2-31 | 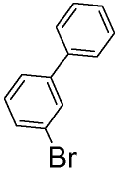 | 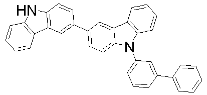 | 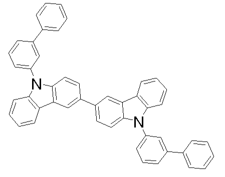 | 81% |
| 2-32 | 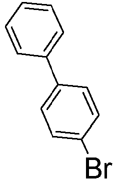 | | 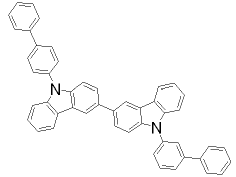 | 80% |
| 2-42 | 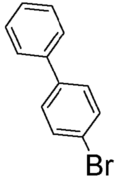 | 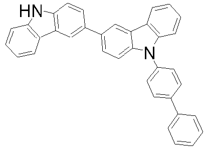 | 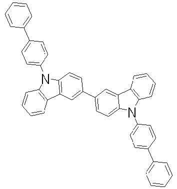 | 82% |

Compounds were prepared in the same manner as in the preparation examples, and the synthesis identification results are shown in the following Tables 3 to 5.

**[Table 3]**

| Compound Number | ¹H NMR (CDCl₃, 200 Mz) |
|---|---|
| 1-39 | δ =9.35 (1H, s), 8.90(4H, d), 8.64(1H, s), 8.30~8.20 (3H, m), 8.13 (1H, d), 7.71(1H, s), 7.66~7.45(14H, m), 7.38∼7.33 (3H, m) |
| 1-40 | δ =9.38(1H, s), 9.24(1H, s), 8.87(3H, d), 8.64(1H, s), 8.30~8.21(3H, m), 8.08(1H, d), 7.90(1H, d), 7.80(2H, d), 7.72~7.32(19H, d) |
| 1-41 | δ =9.30(1H, s), 8.90(1H, d), 8.80(4H, d), 8.43(1H, s), 8.26~8.20(3H, m), 8.03(1H, d), 7.93(1H, d), 7.87(1H, |
| | s), 7.77(1H, t), 7.72 (1H, s), 7.67(1H, d), 7.60~7.47(11H, m), 7.41~7.29(4H, m) |
| 1-46 | δ =8.62(1H, d), 8.45(1H, d), 8.36~8.31(5H, m), 8.22(1H, m), 8.00(1H, s), 7.93~7.91(2H, m), 7.77~7.74(7H, m), 7.50~7.41(14H, m) |
| 1-67 | δ =9.39(1H, s), 8.90(4H, d), 8.68(1H, s), 8.54 (1H, s), 8.30(1H, d), 8.25(1H, d), 8.15(1H, d), 7.91 (1H, d), 7.64~7.52(10H, m), 7.47~7.35(4H, m), 7.29(1H, t), 1.53∼1.50 (6H, d) |
| 2-3 | δ =8.55(1H, d), 8.30(1H, d), 8.21~8.13(3H, m), 7.99~7.89(4H, m), 7.77~7.35(17H, m), 7.20~7.16(2H, m) |
| 2-4 | δ =8.55(1H, d), 8.30(1H, d), 8.19~8.13(2H, m), 7.99~7.89(8H, m), 7.77~7.75(3H, m), 7.62~7.35(11H, m), 7.20~7.16(2H, m) |
| 2-7 | δ =8.55(1H, d), 8.31~8.30(3H, d), 8.19~8.13(2H, m), 7.99~7.89(5H, m), 7.77~7.75(5H, m), 7.62~7.35(14H, m), 7.20~7.16(2H, m) |
| 2-31 | δ =8.55(1H, d), 8.30(1H, d), 8.21~8.13(4H, m), 7.99~7.89(4H, m), 7.77~7.35(20H, m), 7.20~7.16(2H, m) |
| 2-32 | δ =8.55(1H, d), 8.30(1H, d), 8.21~8.13(3H, m), 7.99~7.89(8H, m), 7.77~7.35(17H, m), 7.20~7.16(2H, m) |

**[Table 4]**

| Compound | FD-MS | Compound | FD-MS |
|---|---|---|---|
| 1-2 | m/z=579.18 (C₄₀H₂₅N₃S=579.72) | 1-11 | m/z=580.17 (C₃₉H₂₄N₄S=580.71) |
| 1-12 | m/z=656.20 (C₄₅H₂₈N4S=656.81) | 1-17 | m/z=552.17 (C₃₉H₂₄N₂S=552.69) |
| 1-23 | m/z=655.21 (C₄₆H₂₉N₃S=655.81) | 1-27 | m/z=656.20 (C₄₅H₂₈N₄S=656.80) |
| 1-33 | m/z=655.21 (C₄₆H₂₉N₃S=655.81) | 1-36 | m/z=656.20 (C₄₅H₂₈N₄S=656.80) |
| 1-39 | m/z=656.20 (C₄₅H₂₈N4S=656.80) | 1-40 | m/z=732.23 (C₅₁H₃₂N₄S=732.90) |
| 1-41 | m/z=732.23 (C₅₁H₃₂N₄S=732.89) | 1-42 | m/z=732.23 (C₅₁H₃₂N₄S=732.89) |
| 1-46 | m/z=732.23 (C₅₁H₃₂N₄S=732.89) | 1-64 | m/z=656.20 (C₄₅H₂₈N₄S=656.80) |
| 1-65 | m/z=695.24 (C₄₉H₃₃N₃S=695.87) | 1-66 | m/z=695.24 (C₄₉H₃₃N₃S=695.87) |
| 1-67 | m/z=696.23 (C₄₈H₃₂N₄S=696.86) | 1-68 | m/z=669.22 (C₄₇H₃₁N₃S=669.83) |
| 1-69 | m/z=771.27 (C₅₅H₃₇N₃S=771.97) | 1-70 | m/z=772.27 (C₅₄H₃₆N₄S=772.96) |
| 1-71 | m/z=733.26 (C₅₂H₃₅N₃S=733.93) | 1-72 | m/z=772.27 (C₅₄H₃₆N₄S=772.96) |
| 1-78 | m/z=696.23 (C₄₈H₃₂N₄S=696.86) | 1-82 | m/z=744.23 (C₅₂H₃₂N₄S=744.90) |
| 1-83 | m/z=744.23 (C₅₂H₃₂N₄S=744.90) | 1-84 | m/z=745.23 (C₅₁H₃₁N₅S=745.89) |
| 1-93 | m/z=745.23 (C₅₁H₃₁N₅S=745.89) | 1-96 | m/z=685.16 (C₄₆H₂₇N₃S₂=685.86) |
| 1-98 | m/z=686.16 (C₄₅H₂₆N₄S₂=686.84) | 1-99 | m/z=761.20 (C₅₁H₃₁N₃S₂=761.95) |
| 1-100 | m/z=762.19 (C₅₁H₃₀N₄S₂=762.94) | 1-110 | m/z=686.16 (C₄₅H₂₆N₄S₂=686.84) |
| 1-117 | m/z=670.18 (C₄₅H₂₆N₄OS=670.78) | 1-119 | m/z=746.21 (C₅₁H₃₀N₄OS=746.88) |
| 1-126 | m/z=670.18 (C₄₅H₂₆N₄OS=670.78) | 1-163 | m/z=656.80 (C₄₅H₂₈N₄S=656.20) |
| 1-170 | m/z=762.19 (C₅₁H₃₀N₄S₂=762.94) | 1-172 | m/z=746.21 (C₅₁H₃₀N₄OS=746.88) |
| 1-176 | m/z=643.17 (C₄₄H₂₅N₃OS=643.75) | 1-177 | m/z=659.15 (C₄₄H₂₅N₃S₂=659.82) |
| 1-178 | m/z=669.22 (C₄₇H₃₁N₃S=669.83) | 1-179 | m/z=669.22 (C₄₇H₃₁N₃S=669.83) |
| 1-180 | m/z=659.15 (C₄₄H₂₅N₃S₂=659.82) | 1-181 | m/z=643.17 (C₄₄H₂₅N₃OS=643.75) |

**[Table 5]**

| Compound | FD-Mass | Compound | FD-Mass |
|---|---|---|---|
| 2-1 | m/z=484.19 (C₃₆H₂₄N₂=484.60) | 2-2 | m/z=560.23 (C₄₂H₂₈N₂=560.70) |
| 2-3 | m/z=560.23 (C₄₂H₂₈N₂=560.70) | 2-4 | m/z=560.23 (C₄₂H₂₈N₂=560.70) |
| 2-5 | m/z=636.26 (C₄₈H₃₂N₂=636.80) | 2-6 | m/z=636.26 (C₄₈H₃₂N₂=636.80) |
| 2-7 | m/z=636.26 (C₄₈H₃₂N₂=636.80) | 2-8 | m/z=534.21 (C₄₀H₂₆N₂=534.66) |
| 2-9 | m/z=534.21 (C₄₀H₂₆N₂=534.66) | 2-10 | m/z=600.26 (C₄₅H₃₂N₂=600.76) |
| 2-11 | m/z=600.26 (C₄₅H₃₂N₂=600.76) | 2-12 | m/z=724.29 (C₅₅H₃₆N₂=724.91) |
| 2-13 | m/z=724.29 (C₅₅H₃₆N₂=724.91) | 2-14 | m/z=722.27 (C₅₅H₃₄N₂=722.89) |
| 2-15 | m/z=722.27 (C₅₅H₃₄N₂=722.89) | 2-16 | m/z=634.24 (C₄₈H₃₀N₂=634.78) |
| 2-17 | m/z=509.19 (C₃₇H₂₃N₃=509.61) | 2-18 | m/z=742.28 (C₅₄H₃₈N₂Si=743.00) |
| 2-19 | m/z=636.26 (C₄₈H₃₂N₂=636.80) | 2-20 | m/z=636.26 (C₄₈H₃₂N₂=636.80) |
| 2-21 | m/z=636.26 (C₄₈H₃₂N₂=636.80) | 2-22 | m/z=712.29 (C₅₄H₃₆N₂=712. 90) |
| 2-23 | m/z=712.29 (C₅₄H₃₆N₂=712. 90) | 2-24 | m/z=712.29 (C₅₄H₃₆N₂=712. 90) |
| 2-25 | m/z=610.24 (C₄₆H₃₀N₂=610.76) | 2-26 | m/z=610.24 (C₄₆H₃₀N₂=610.76) |
| 2-27 | m/z=676.29 (C₅₁H₃₆N₂=676.86) | 2-28 | m/z=710.27 (C₅₄H₃₄N₂=710.88) |
| 2-29 | m/z=585.22 (C₄₃H₂₇N₃=585.71) | 2-30 | m/z=818.31 (C₆₀H₄₂N₂Si=819.10) |
| 2-31 | m/z=636.26 (C₄₈H₃₂N₂=636.80) | 2-32 | m/z=636.26 (C₄₈H₃₂N₂=636.80) |
| 2-33 | m/z=712.29 (C₅₄H₃₆N₂=712.90) | 2-34 | m/z=712.29 (C₅₄H₃₆N₂=712.90) |
| 2-35 | m/z=712.29 (C₅₄H₃₆N₂=712.90) | 2-36 | m/z=610.24 (C₄₆H₃₀N₂=610.76) |
| 2-37 | m/z=610.24 (C₄₆H₃₀N₂=610.76) | 2-38 | m/z=676.29 (C₅₁H₃₆N₂=676.86) |
| 2-39 | m/z=710.27 (C₅₄H₃₄N₂=710.88) | 2-40 | m/z=585.22 (C₄₃H₂₇N₃=585.71) |
| 2-41 | m/z=818.31 (C₆₀H₄₂N₂Si=819.10) | 2-42 | m/z=636.26 (C₄₈H₃₂N₂=636.80) |
| 2-43 | m/z=712.29 (C₅₄H₃₆N₂=712.90) | 2-44 | m/z=712.29 (C₅₄H₃₆N₂=712.90) |
| 2-45 | m/z=712.29 (C₅₄H₃₆N₂=712.90) | 2-46 | m/z=610.24 (C₄₆H₃₀N₂=610.76) |
| 2-47 | m/z=610.24 (C₄₆H₃₀N₂=610.76) | 2-48 | m/z=676.29 (C₅₁H₃₆N₂=676.86) |
| 2-49 | m/z=710.27 (C₅₄H₃₄N₂=710.88) | 2-50 | m/z=585.22 (C₄₃H₂₇N₃=585.71) |
| 2-51 | m/z=818.31 (C₆₀H₄₂N₂Si=819.10) | 2-52 | m/z=788.32 (C₆₀H₄₀N₂=788. 99) |
| 2-53 | m/z=788.32 (C₆₀H₄₀N₂=788.99) | 2-54 | m/z=788.32 (C₆₀H₄₀N₂=788.99) |
| 2-55 | m/z=686.27 (C₅₂H₃₄N₂=686.86) | 2-56 | m/z=686.27 (C₅₂H₃₄N₂=686.86) |
| 2-57 | m/z=752.32 (C₅₇H₄₀N₂=752.96) | 2-58 | m/z=786.30 (C₆₀H₃₈N₂=786.98) |
| 2-59 | m/z=661.25 (C₄₉H₃₁N₃=661.81) | 2-60 | m/z=894.34 (C₆₆H₄₆N₂Si=895.19) |
| 2-61 | m/z=788.32 (C₆₀H₄₀N₂=788. 99) | 2-62 | m/z=788.32 (C₆₀H₄₀N₂=788.99) |
| 2-63 | m/z=686.27 (C₅₂H₃₄N₂=686.86) | 2-64 | m/z=686.27 (C₅₂H₃₄N₂=686.86) |
| 2-65 | m/z=752.32 (C₅₇H₄₀N₂=752.96) | 2-66 | m/z=786.30 (C₆₀H₃₈N₂=786.98) |
| 2-67 | m/z=661.25 (C₄₉H₃₁N₃=661.81) | 2-68 | m/z=894.34 (C₆₆H₄₆N₂Si=895.19) |
| 2-69 | m/z=788.32 (C₆₀H₄₀N₂=788.99) | 2-70 | m/z=686.27 (C₅₂H₃₄N₂=686.86) |
| 2-71 | m/z=686.27 (C₅₂H₃₄N₂=686.86) | 2-72 | m/z=752.32 (C₅₇H₄₀N₂=752.96) |
| 2-73 | m/z=786.30 (C₆₀H₃₈N₂=786.98) | 2-74 | m/z=661.25 (C₄₉H₃₁N₃=661.81) |
| 2-75 | m/z=894.34 (C₆₆H₄₆N₂Si=895.19) | 2-76 | m/z=584.23 (C₄₄H₂₈N₂=584.72) |
| 2-77 | m/z=584.23 (C₄₄H₂₈N₂=584.72) | 2-78 | m/z=650.27 (C₄₉H₃₄N₂=650.83) |
| 2-79 | m/z=684.26 (C₅₂H₃₂N₂=684.84) | 2-80 | m/z=559.20 (C₄₁H₂₅N₃=559.67) |
| 2-81 | m/z=792.30 (C₅₈H₄₀N₂Si=793.06) | 2-82 | m/z=584.23 (C₄₄H₂₈N₂=584.72) |
| 2-83 | m/z=650.27 (C₄₉H₃₄N₂=650.83) | 2-84 | m/z=684.26 (C₅₂H₃₂N₂=684.84) |
| 2-85 | m/z=559.20 (C₄₁H₂₅N₃=559.67) | 2-86 | m/z=792.30 (C₅₈H₄₀N₂Si=793.06) |
| 2-87 | m/z=716.32 (C₅₄H₄₀N₂=716.93) | 2-88 | m/z=750.30 (C₅₇H₃₈N₂=750.94) |
| 2-89 | m/z=625.25 (C₄₆H₃₁N₃=625.77) | 2-90 | m/z=858.34 (C₆₃H₄₆N₂Si=859.16) |
| 2-91 | m/z=784.29 (C₆₀H₃₆N₂=784.96) | 2-92 | m/z=659.24 (C₄₉H₂₉N₃=659.79) |
| 2-93 | m/z=892.33 (C₆₆H₄₄N₂Si=893.18) | 2-94 | m/z=534.18 (C₃₈H₂₂N₄=534.62) |
| 2-95 | m/z=767.28 (C₅₅H₃₇N₃Si=768.01) | 2-96 | m/z=1000.37 (C₇₂H₅₂N₂Si₂=1001.39) |

Table 3 shows NMR values, and Tables 4 and 5 show field desorption mass spectrometry (FD-MS) measurement values.

### <Experimental Example 1> Manufacture of Organic Light Emitting Device

A glass substrate on which ITO was coated as a thin film to a thickness of 1500 Å was cleaned with distilled water ultrasonic waves. After the cleaning with distilled water was finished, the substrate was ultrasonic cleaned with solvents such as acetone, methanol and isopropyl alcohol, then dried, and UVO treatment was carried out for 5 minutes in a UV cleaner using UV. After that, the substrate was transferred to a plasma cleaner (PT), and plasma treatment was carried out under vacuum for ITO work function and remaining film removal, and the substrate was transferred to a thermal deposition apparatus for organic deposition.

On the transparent ITO electrode (anode), a hole injection layer 4,4',4"-tris[2-naphthyl(phenyl)amino]triphenylamine (2-TNATA) and a hole transfer layer N,N'-di(1-naphthyl)-N,N'-diphenyl-(1,1'-biphenyl)-4,4'-diamine (NPB), which are common layers, were formed.

A light emitting layer was thermal vacuum deposited thereon as follows. As the light emitting layer, a host was deposited to 400 Å in one supply source after premixing one type of compound described in Chemical Formula 1 and one type of compound described in Chemical Formula 2, and a green phosphorescent dopant was deposited by 7% doping Ir(ppy)₃. After that, BCP was deposited to 60 Å as a hole blocking layer, and Alq₃ was deposited to 200 Å thereon as an electron transfer layer. Lastly, an electron injection layer was formed on the electron transfer layer by depositing lithium fluoride (LiF) to a thickness of 10 Å, and then a cathode was formed on the electron injection layer by depositing an aluminum (Al) cathode to a thickness of 1,200 Å to manufacture an organic electroluminescent device.

Meanwhile, all the organic compounds required to manufacture the OLED device were vacuum sublimation purified under 10⁻⁶ torr to 10⁻⁸ torr by each material to be used in the OLED manufacture.

Driving voltage and light emission efficiency of the organic electroluminescent devices according to Experimental Example 1 are as shown in the following Table 6.

For the organic electroluminescent devices manufactured as above, electroluminescent light emission (EL) characteristics were measured using M7000 manufactured by McScience Inc., and with the measurement results, T₉₀ when standard luminance was 6,000 cd/m² was measured using a lifetime test system (M6000) manufactured by McScience Inc..

Properties of the organic electroluminescent devices of the present disclosure are as shown in the following Table 6.

**[Table 6]**

| | Light Emittin g Layer Compoun d | Rati o | Drivin g Voltag e (V) | Efficienc y (cd/A) | Color Coordinat e (x, y) | Lifetim e (T₉₀) |
|---|---|---|---|---|---|---|
| Example 1 | 1-39: 2-3 | 1:8 | 4.73 | 54.2 | (0.233, 0.714) | 180 |
| Example 2 | | 1:5 | 4.71 | 57.2 | (0.243, 0.714) | 182 |
| Example 3 | | 1:2 | 4.35 | 79.2 | (0.241, 0.714) | 301 |
| Example 4 | | 1: 1 | 4.41 | 75.8 | (0.231, 0.711) | 289 |
| Example 5 | | 2:1 | 4.67 | 71.2 | (0.251, 0.714) | 241 |
| Example 6 | | 5:1 | 4.32 | 68.3 | (0.241, 0.711) | 171 |
| Example 7 | | 8:1 | 4.21 | 67.0 | (0.247, 0.727) | 162 |
| Example 8 | 1-40: 2-4 | 1:2 | 4.33 | 74.2 | (0.241, 0.714) | 261 |
| Example 9 | | 1:1 | 4.42 | 72.2 | (0.231, 0.711) | 251 |
| Example 10 | | 2:1 | 4.66 | 71.2 | (0.251, 0.714) | 227 |
| Example 11 | 1-41: 2-7 | 1:2 | 4.38 | 76.4 | (0.241, 0.711) | 240 |
| Example 12 | | 1: 1 | 4.45 | 72.8 | (0.251, 0.714) | 229 |
| Example 13 | | 2:1 | 4.66 | 71.1 | (0.241, 0.711) | 201 |
| Example 14 | 1-46: 2-31 | 1:2 | 4.33 | 75.2 | (0.247, 0.727) | 267 |
| Example 15 | | 1: 1 | 4.48 | 70.2 | (0.241, 0.714) | 246 |
| Example 16 | | 2:1 | 4.69 | 69.2 | (0.231, 0.711) | 219 |
| Example 17 | 1-67: 2-32 | 1:2 | 4.31 | 79.2 | (0.246, 0.717) | 283 |
| Example 18 | | 1: 1 | 4.42 | 75.7 | (0.251, 0.714) | 261 |
| Example 19 | | 2:1 | 4.66 | 71.1 | (0.241, 0.711) | 236 |
| Comparativ e Example 1 | 1-39 | - | 4.14 | 68.9 | (0.246, 0.717) | 140 |
| Comparativ e Example 2 | 1-40 | - | 4.26 | 67.6 | (0.255, 0.698) | 131 |
| Comparativ e Example 3 | 1-41 | - | 4.64 | 63.9 | (0.236, 0.696) | 120 |
| Comparativ e Example 4 | 1-46 | - | 4.54 | 65.9 | (0.246, 0.686) | 126 |
| Comparativ e Example 5 | 1-67 | - | 4.32 | 65.9 | (0.265, 0.716) | 135 |
| Comparativ e Example 6 | 2-3 | - | 4.75 | 51.2 | (0.254, 0.724) | 59 |
| Comparativ e Example 7 | 2-4 | - | 4.83 | 50.9 | (0.233, 0.703) | 41 |
| Comparativ e Example 8 | 2-7 | - | 4.73 | 52.2 | (0.234, 0.714) | 51 |
| Comparativ e Example 9 | 2-31 | - | 4.81 | 49.9 | (0.243, 0.693) | 52 |
| Comparativ e Example 10 | 2-32 | - | 4.74 | 55.2 | (0.251, 0.724) | 43 |

The organic light emitting device of the present disclosure comprises a light emitting layer using a host and a phosphorescent dopant, and by the host being formed with a host compound (p-n type) mixing two or more compounds, the organic light emitting device of the present disclosure is effective in obtaining a more superior lifetime property compared to existing organic light emitting devices comprising a host compound formed with a single compound.

Particularly, the p-n type host of the present disclosure has an advantage of increasing light emission properties by controlling a ratio of the host, and this is a result obtained by a proper combination of a p host with favorable hole mobility and an n host with favorable electron mobility.

In addition, the light emitting host formed with plural types of compounds was formed with one deposition supply source after premixing the compounds, and then deposited. Herein, thin film uniformity and thin film properties may be improved by avoiding deposition of several times, and as a result, a device with a simplified process, reduced costs, and improved efficiency and lifetime may be formed.

## Claims

1. An organic light emitting device comprising:
an anode (200);
a cathode (400); and
a light emitting layer (303) provided between the anode and the cathode,
wherein the light emitting layer comprises a hetero-cyclic compound represented by the following Chemical Formula 1 and a compound represented by the following Chemical Formula 2 at the same time: 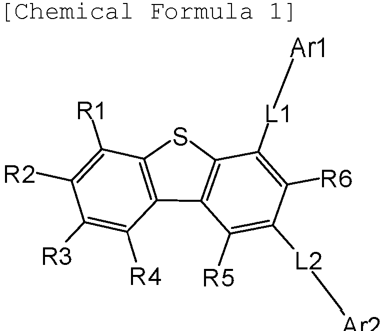 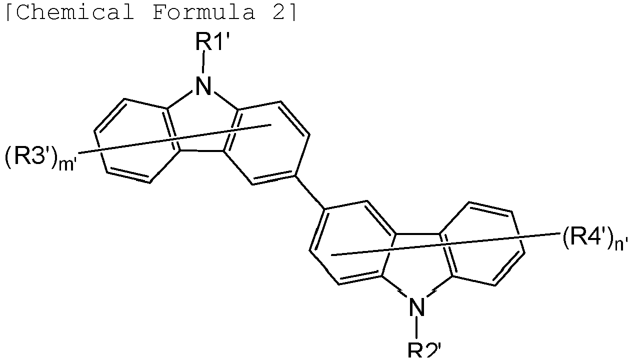
in Chemical Formulae 1 and 2,
L1 and L2 are the same as or different from each other, and each independently a direct bond, or a substituted or unsubstituted C₆ to C₆₀ arylene group;
Ar1 is a pyridine group unsubstituted or substituted with a phenyl group; a triazine group unsubstituted or substituted with one or more substituents selected from the group consisting of a phenyl group, a biphenyl group, a dibenzothiophene group and a dimethylfluorene group; a quinoline group unsubstituted or substituted with a phenyl group; a quinazoline group unsubstituted or substituted with a phenyl group or a biphenyl group; a phenanthroline group; or a benzimidazole group unsubstituted or substituted with a phenyl group,
Ar2 is represented by any one of the following Chemical Formulae 3 and 4; 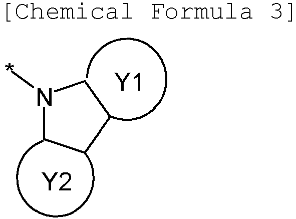 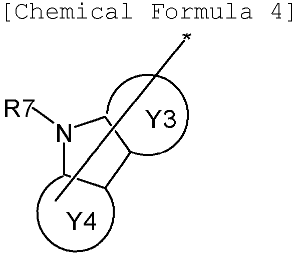
Y1 to Y4 are the same as or different from each other, and each independently a substituted or unsubstituted C₆ to C₆₀ aromatic hydrocarbon ring; or a substituted or unsubstituted C₂ to C₆₀ aromatic heteroring;
R1 to R7, R3' and R4' are the same as or different from each other, and each independently selected from the group consisting of hydrogen; deuterium; a halogen group; -CN; a substituted or unsubstituted C₁ to C₆₀ alkyl group; a substituted or unsubstituted C₂ to C₆₀ alkenyl group; a substituted or unsubstituted C₂ to C₆₀ alkynyl group; a substituted or unsubstituted C₁ to C₆₀ alkoxy group; a substituted or unsubstituted C₃ to C₆₀ cycloalkyl group; a substituted or unsubstituted C₂ to C₆₀ heterocycloalkyl group; a substituted or unsubstituted C₆ to C₆₀ aryl group; a substituted or unsubstituted C₂ to C₆₀ heteroaryl group; - SiRR'R"; -P(=O)RR' ; and an amine group unsubstituted or substituted with a C₁ to C₂₀ alkyl group, a substituted or unsubstituted C₆ to C₆₀ aryl group, or a C₂ to C₆₀ heteroaryl group, or two or more groups adjacent to each other bond to each other to form a substituted or unsubstituted aliphatic or aromatic hydrocarbon ring;
R1' is represented by -(L1')p'-(Ar1')q', and R2' is represented by -(L2')r'-(Ar2')s';
L1' and L2' are each independently a direct bond, or a substituted or unsubstituted C₆ to C₆₀ arylene group;
Ar1' and Ar2' are each independently -CN; a substituted or unsubstituted C₁ to C₆₀ alkyl group; -SiRR'R"; a substituted or unsubstituted C6 to C60 aryl group,
wherein Chemical Formula 3 is represented by any one of the following structural formulae: 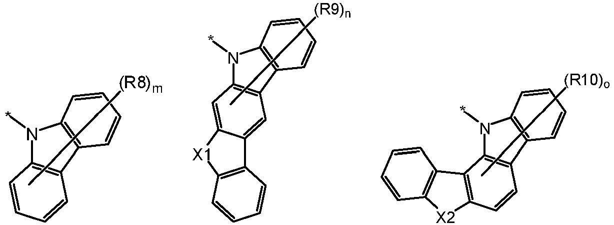 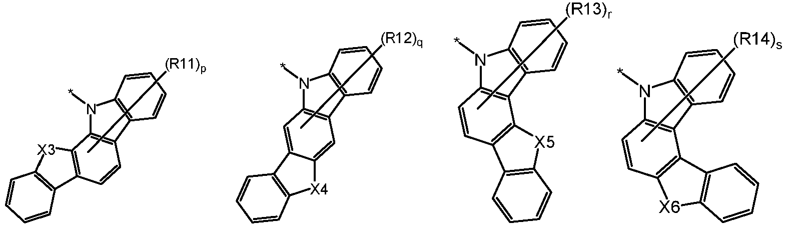
in the structural formulae, X1 to X6 are the same as or different from each other, and each independently NR, S, O or CR'R";
R8 to R14 are the same as or different from each other, and each independently selected from the group consisting of hydrogen; deuterium; a halogen group; -CN; a substituted or unsubstituted C₁ to C₆₀ alkyl group; a substituted or unsubstituted C₂ to C₆₀ alkenyl group; a substituted or unsubstituted C₂ to C₆₀ alkynyl group; a substituted or unsubstituted C₁ to C₆₀ alkoxy group; a substituted or unsubstituted C₃ to C₆₀ cycloalkyl group; a substituted or unsubstituted C₂ to C₆₀ heterocycloalkyl group; a substituted or unsubstituted C₆ to C₆₀ aryl group; a substituted or unsubstituted C₂ to C₆₀ heteroaryl group; -SiRR'R"; -P(=O)RR' ; and an amine group unsubstituted or substituted with a C₁ to C₂₀ alkyl group, a substituted or unsubstituted C₆ to C₆₀ aryl group, or a C₂ to C₆₀ heteroaryl group, or two or more groups adjacent to each other bond to each other to form a substituted or unsubstituted aliphatic or aromatic hydrocarbon ring;
R, R' and R" are the same as or different from each other, and each independently hydrogen; deuterium; -CN; a substituted or unsubstituted C₁ to C₆₀ alkyl group; a substituted or unsubstituted C₃ to C₆₀ cycloalkyl group; a substituted or unsubstituted C₆ to C₆₀ aryl group; or a substituted or unsubstituted C₂ to C₆₀ heteroaryl group;
p' and r' are each independently an integer of 0 to 4;
q' and s' are each independently an integer of 1 to 4; and
m' and n' are each independently an integer of 0 to 7,
m is an integer of 0 to 8, and n, o, p, q, r and s are each independently an integer of 0 to 6.

2. The organic light emitting device of Claim 1, wherein Chemical Formula 4 is represented by any one of the following structural formulae: 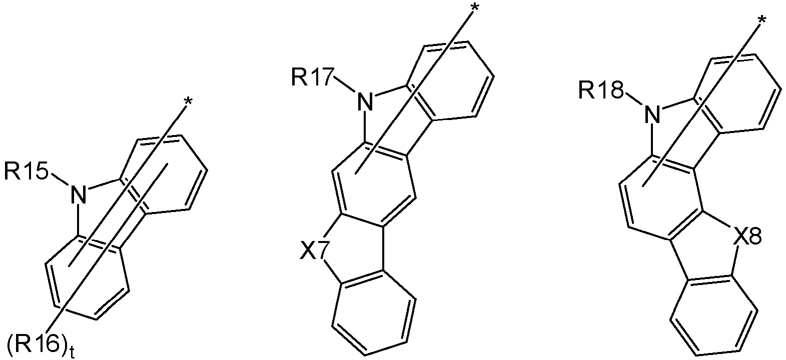
in the structural formulae, X7 and X8 are the same as or different from each other, and each independently NR, S, O or CR'R";
R15 to R18 are the same as or different from each other, and each independently selected from the group consisting of hydrogen; deuterium; a halogen group; -CN; a substituted or unsubstituted C₁ to C₆₀ alkyl group; a substituted or unsubstituted C₂ to C₆₀ alkenyl group; a substituted or unsubstituted C₂ to C₆₀ alkynyl group; a substituted or unsubstituted C₁ to C₆₀ alkoxy group; a substituted or unsubstituted C₃ to C₆₀ cycloalkyl group; a substituted or unsubstituted C₂ to C₆₀ heterocycloalkyl group; a substituted or unsubstituted C₆ to C₆₀ aryl group; a substituted or unsubstituted C₂ to C₆₀ heteroaryl group; -SiRR'R"; -P(=O)RR'; and an amine group unsubstituted or substituted with a C₁ to C₂₀ alkyl group, a substituted or unsubstituted C₆ to C₆₀ aryl group, or a C₂ to C₆₀ heteroaryl group, or two or more groups adjacent to each other bond to each other to form a substituted or unsubstituted aliphatic or aromatic hydrocarbon ring;
R, R' and R" are the same as or different from each other, and each independently hydrogen; deuterium; -CN; a substituted or unsubstituted C₁ to C₆₀ alkyl group; a substituted or unsubstituted C₃ to C₆₀ cycloalkyl group; a substituted or unsubstituted C₆ to C₆₀ aryl group; or a substituted or unsubstituted C₂ to C₆₀ heteroaryl group; and
t is an integer of 0 to 7.

3. The organic light emitting device of Claim 1, wherein Chemical Formula 1 is represented by any one of the following Chemical Formulae 5 to 10: 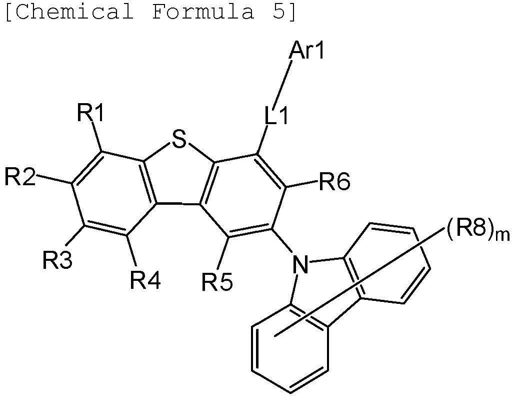 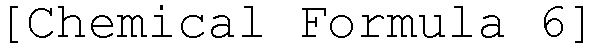 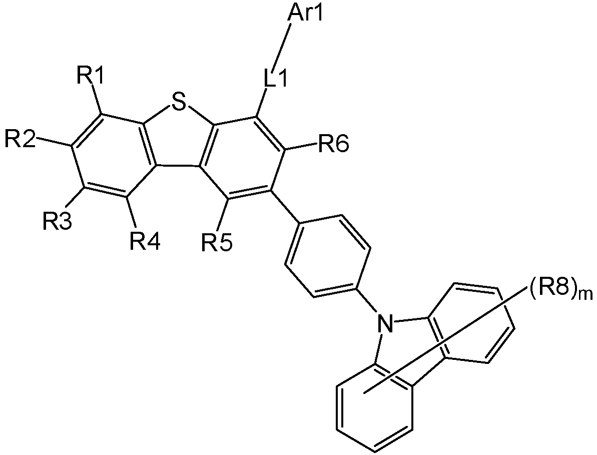 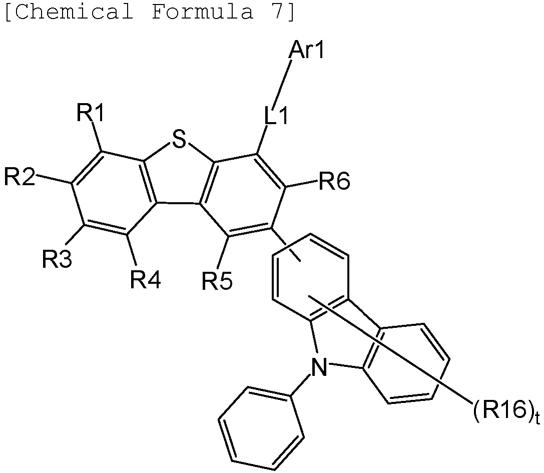 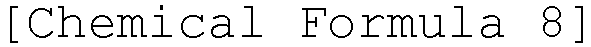 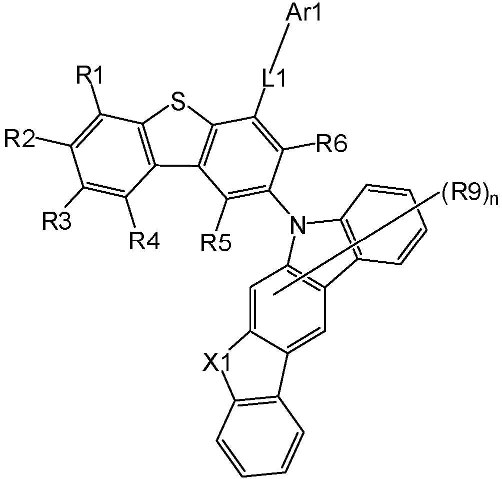 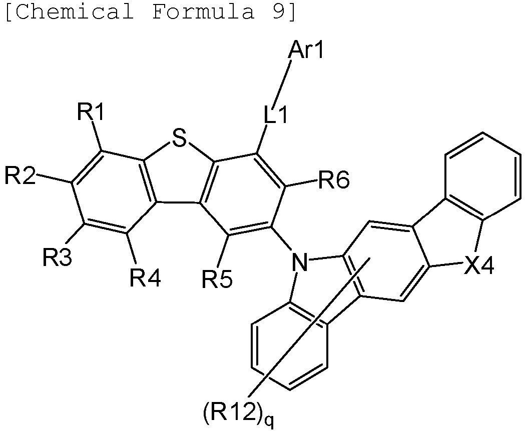 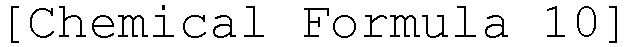 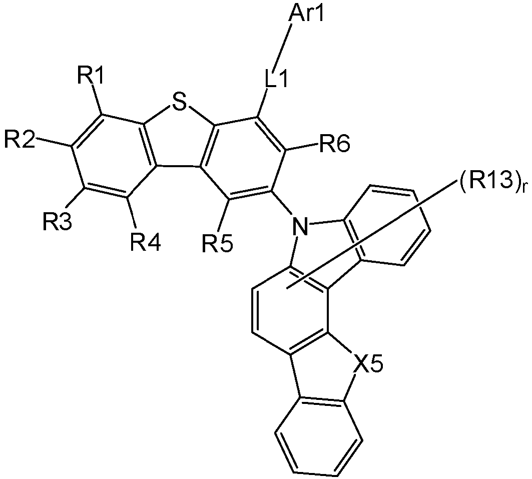 in Chemical Formulae 5 to 10,
R1 to R6, L1 and Ar1 have the same definitions as in Chemical Formula 1;
X1, X4 and X5 are the same as or different from each other, and each independently NR, S, O or CR'R";
R8, R9, R12, R13 and R16 are the same as or different from each other, and each independently selected from the group consisting of hydrogen; deuterium; a halogen group; -CN; a substituted or unsubstituted C₁ to C₆₀ alkyl group; a substituted or unsubstituted C₂ to C₆₀ alkenyl group; a substituted or unsubstituted C₂ to C₆₀ alkynyl group; a substituted or unsubstituted C₁ to C₆₀ alkoxy group; a substituted or unsubstituted C₃ to C₆₀ cycloalkyl group; a substituted or unsubstituted C₂ to C₆₀ heterocycloalkyl group; a substituted or unsubstituted C₆ to C₆₀ aryl group; a substituted or unsubstituted C₂ to C₆₀ heteroaryl group; - SiRR'R"; -P(=O)RR'; and an amine group unsubstituted or substituted with a C₁ to C₂₀ alkyl group, a substituted or unsubstituted C₆ to C₆₀ aryl group, or a C₂ to C₆₀ heteroaryl group, or two or more groups adjacent to each other bond to each other to form a substituted or unsubstituted aliphatic or aromatic hydrocarbon ring;
R, R' and R" are the same as or different from each other, and each independently hydrogen; deuterium; -CN; a substituted or unsubstituted C₁ to C₆₀ alkyl group; a substituted or unsubstituted C₃ to C₆₀ cycloalkyl group; a substituted or unsubstituted C₆ to C₆₀ aryl group; or a substituted or unsubstituted C₂ to C₆₀ heteroaryl group; and
m is an integer of 0 to 8, n, q and r are each independently an integer of 0 to 6, and t is an integer of 0 to 7.

4. The organic light emitting device of Claim 1, wherein Chemical Formula 1 is represented by any one of the following compounds:

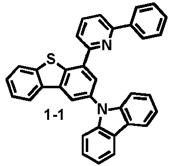

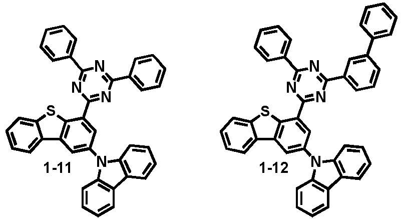

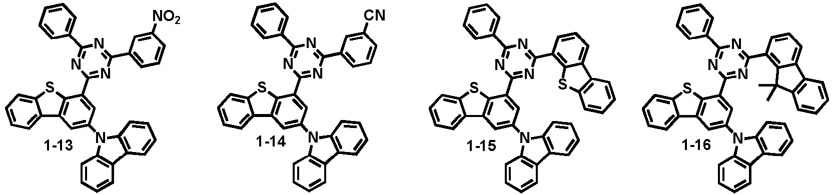

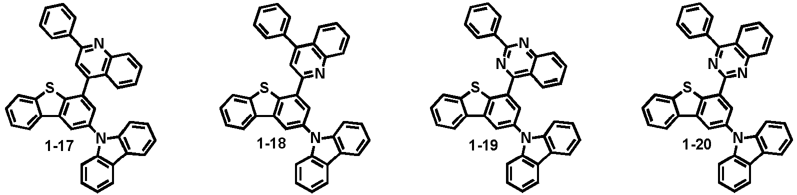

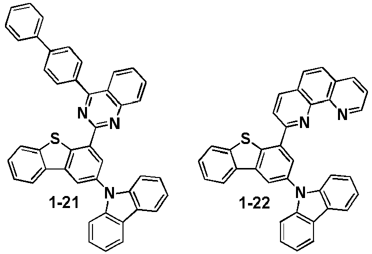

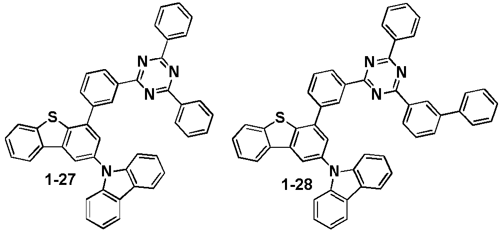

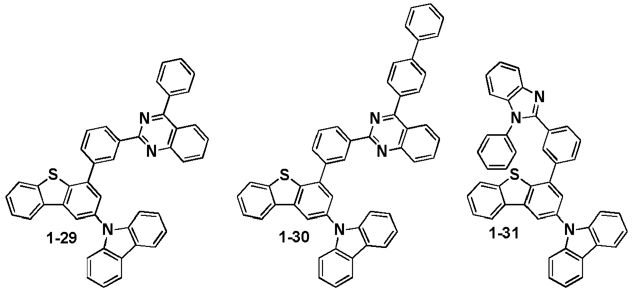

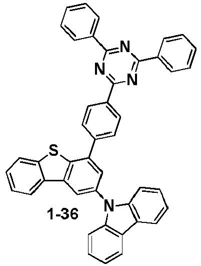

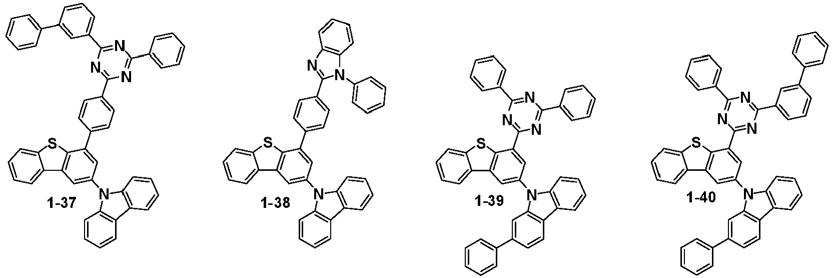

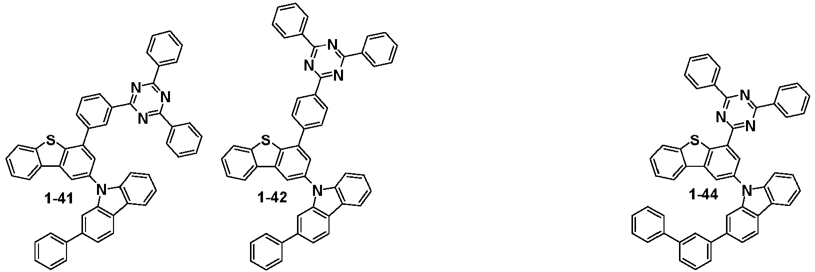

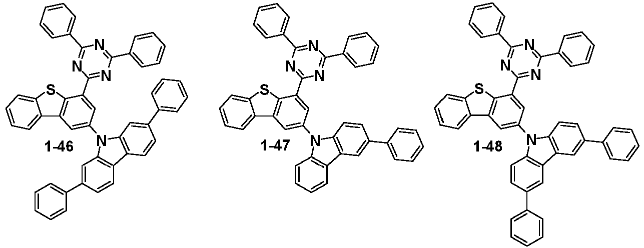

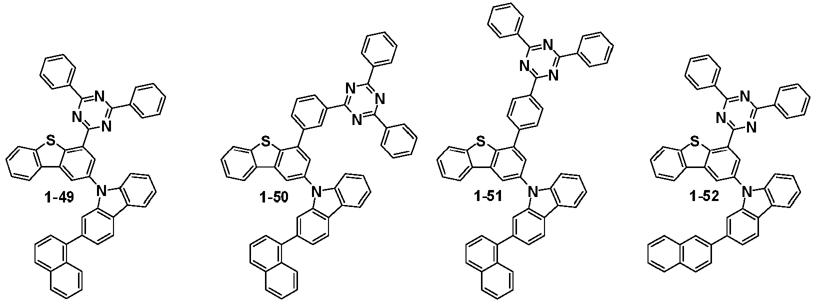

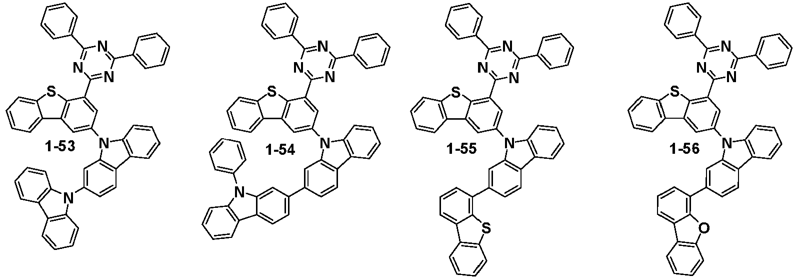

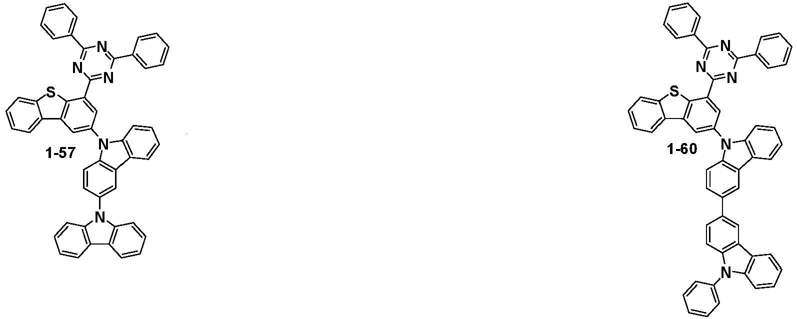

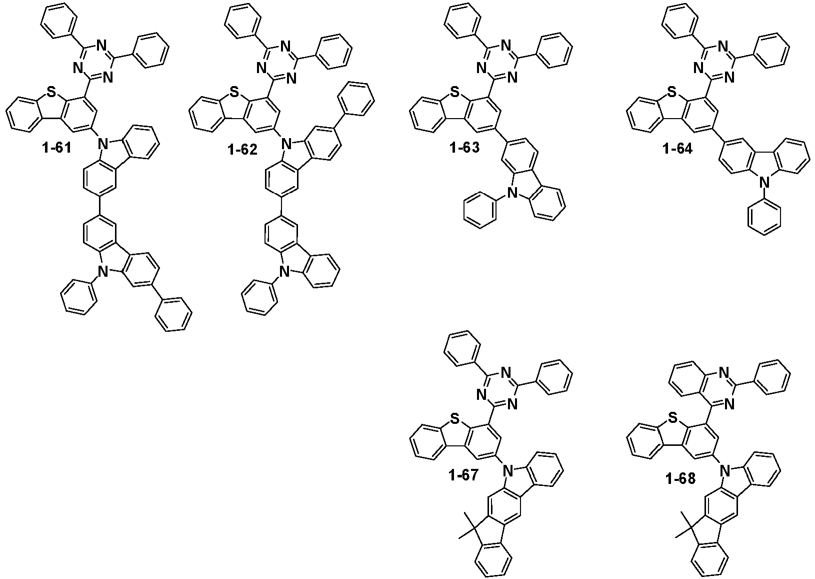

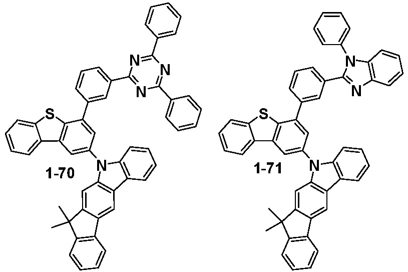

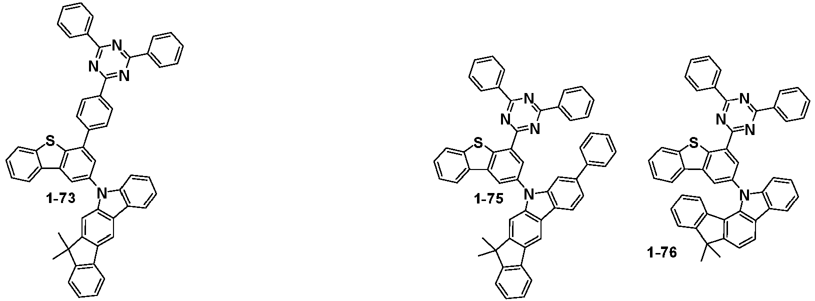

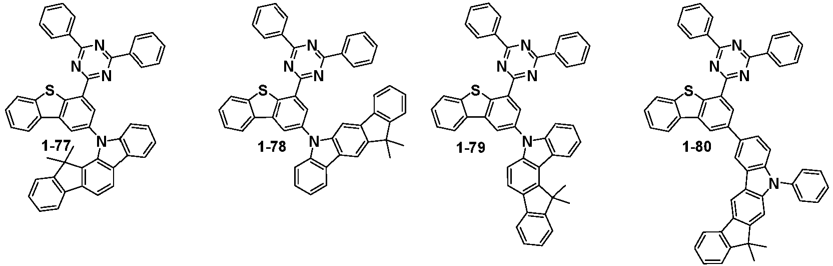

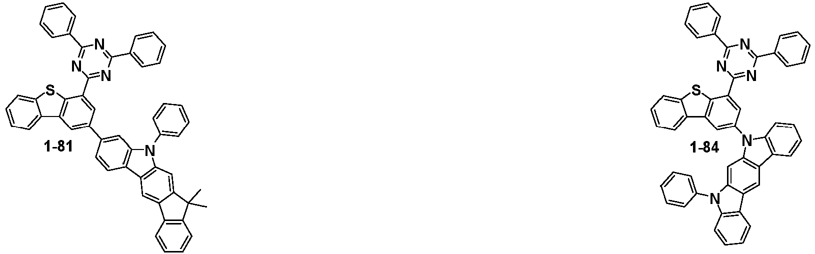

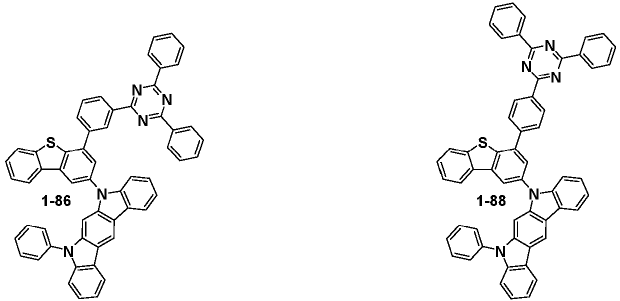

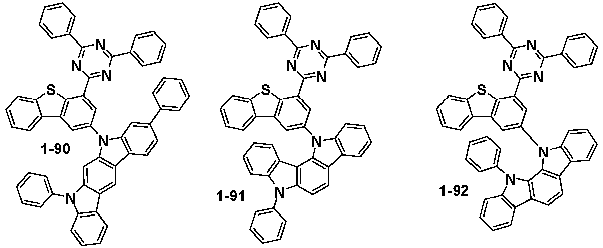

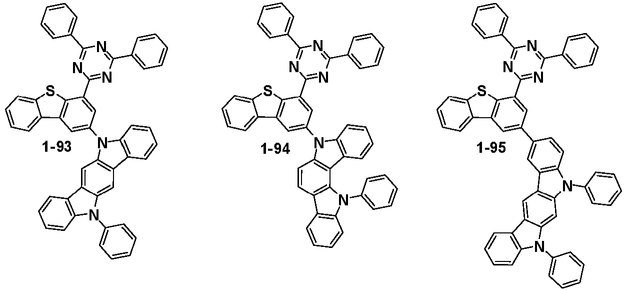

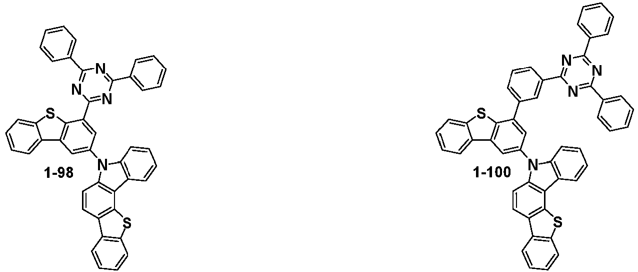

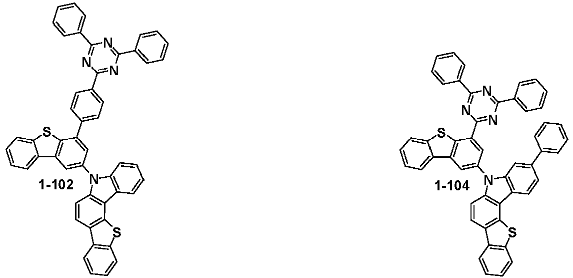

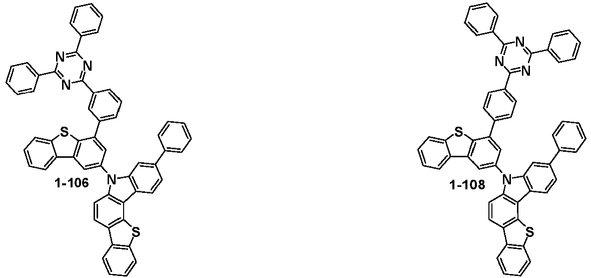

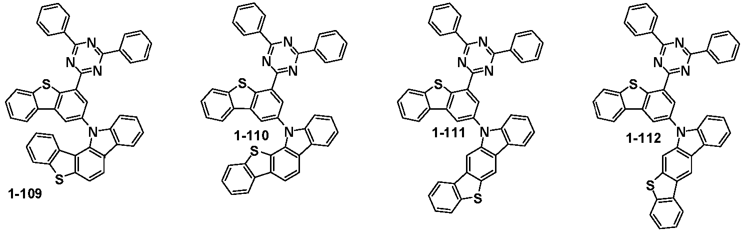

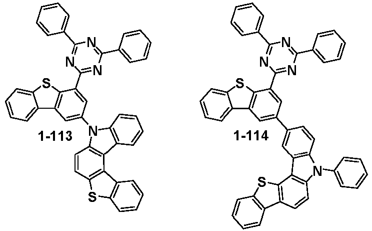

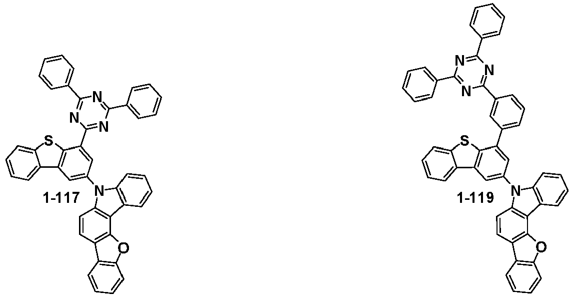

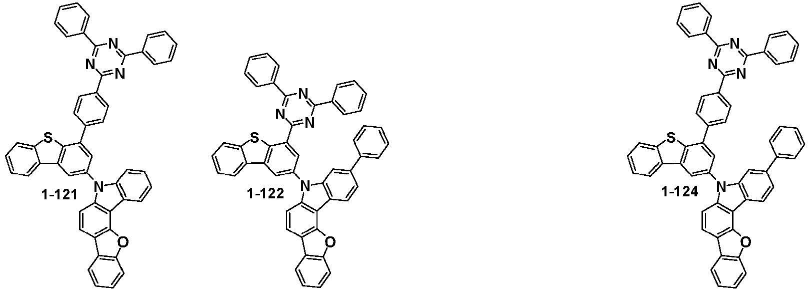

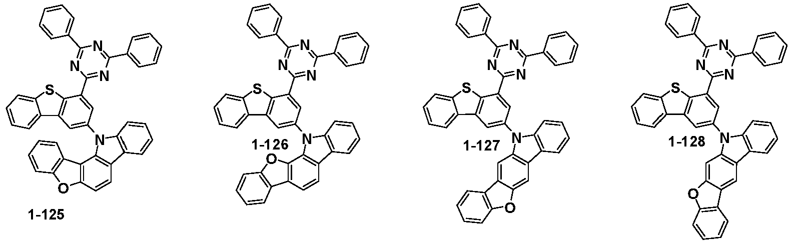

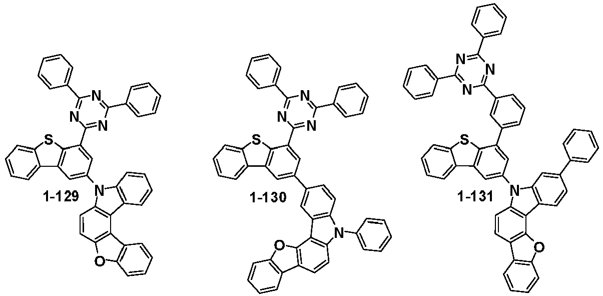

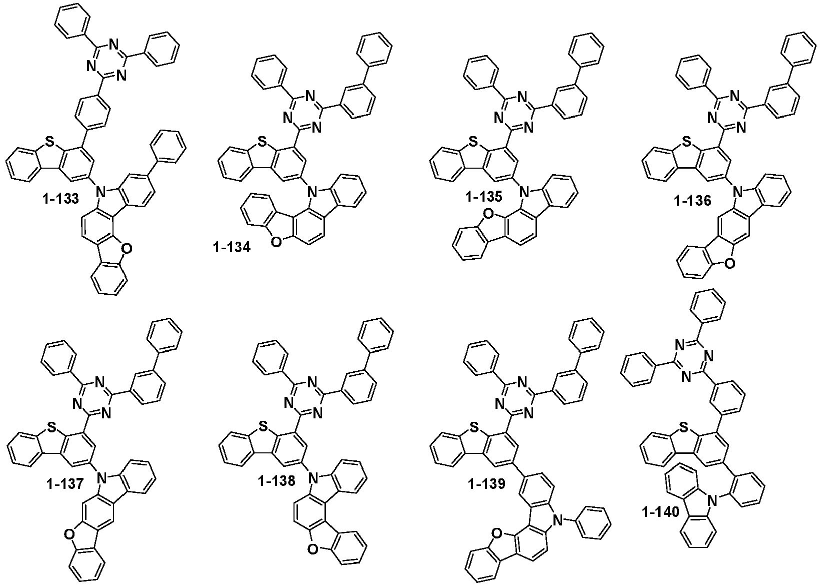

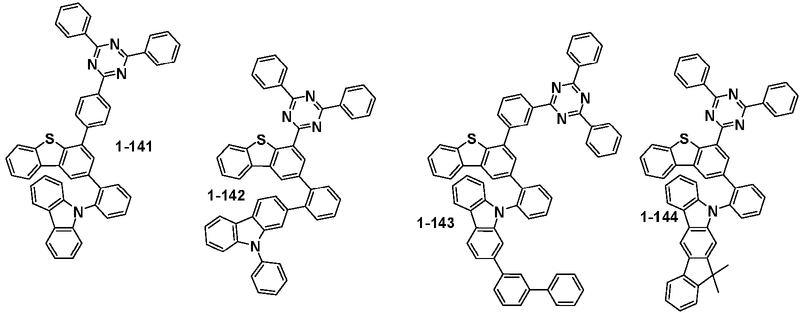

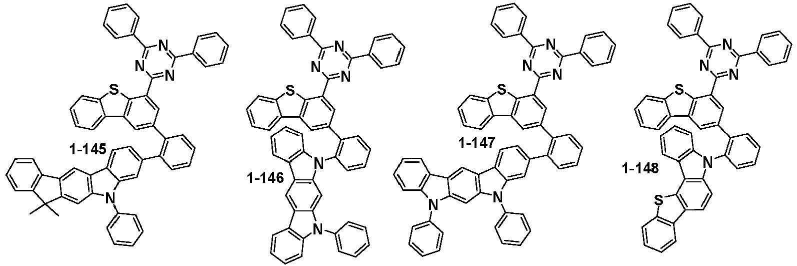

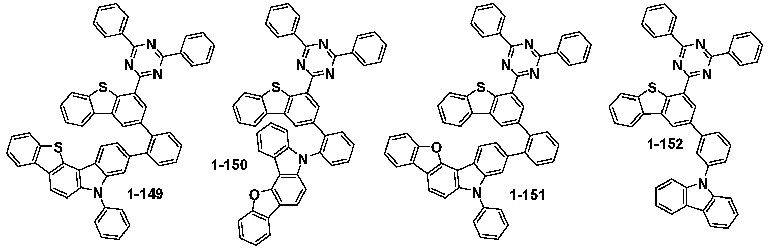

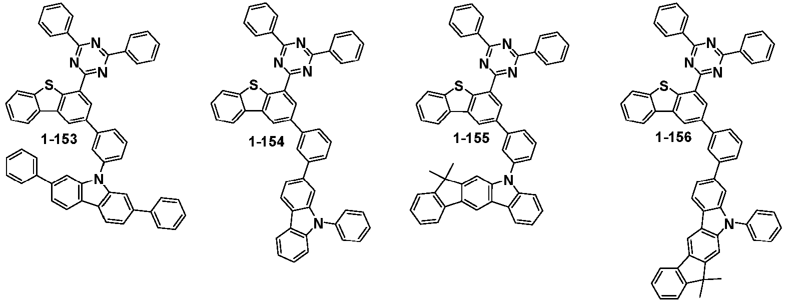

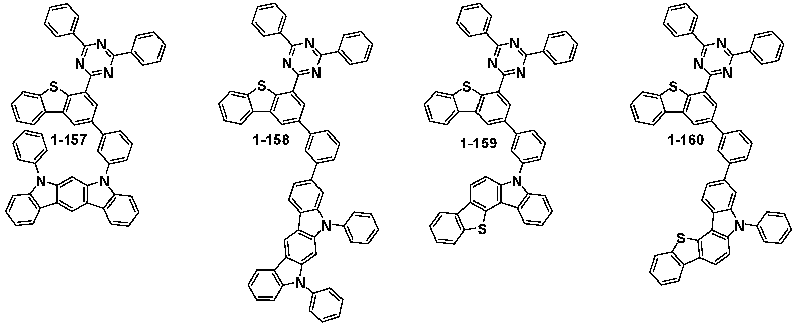

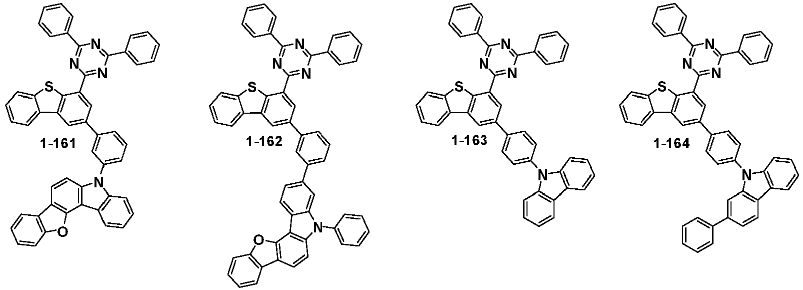

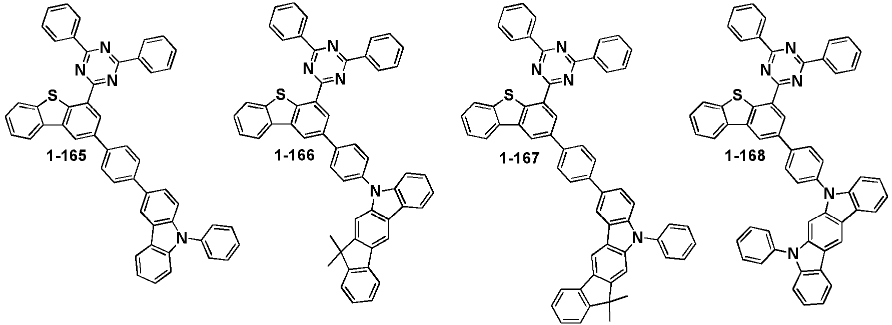

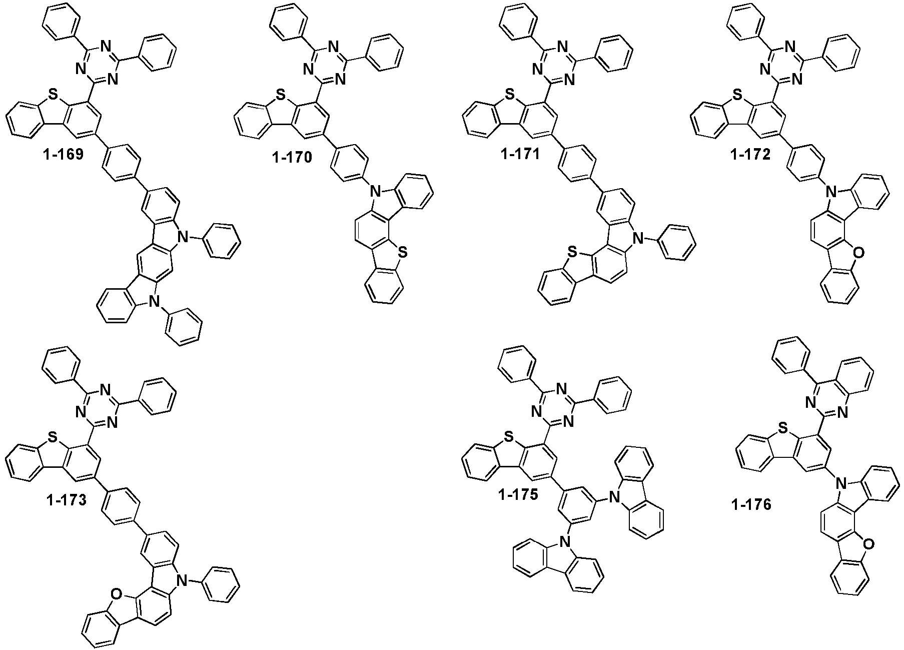

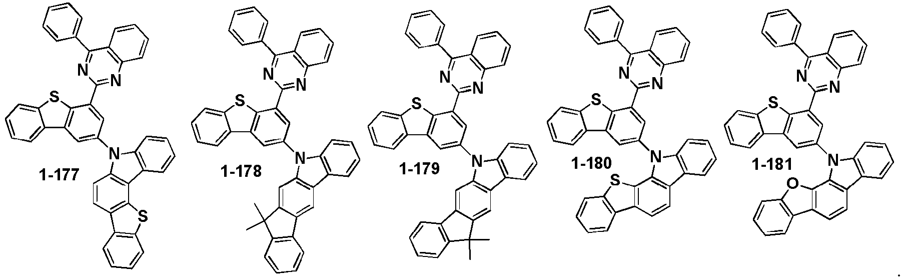

5. The organic light emitting device of Claim 1, wherein Chemical Formula 2 is represented by any one of the following compounds:

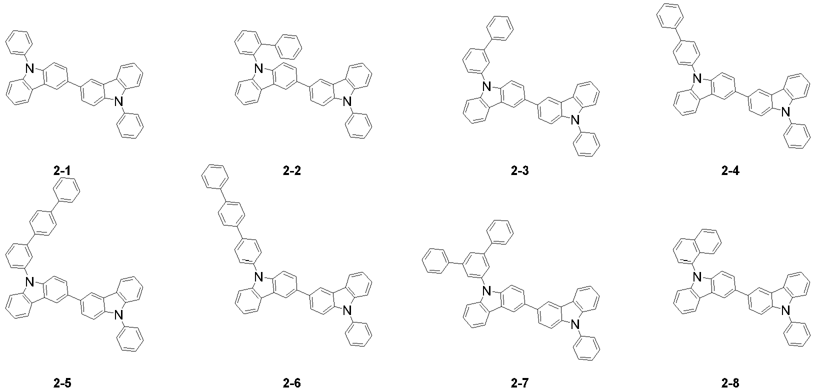

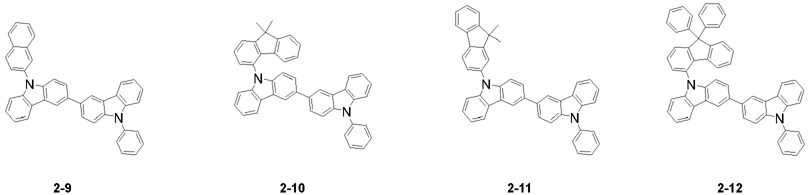

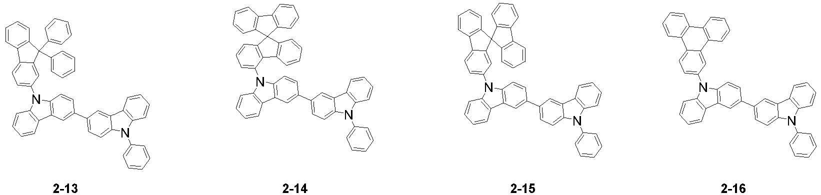

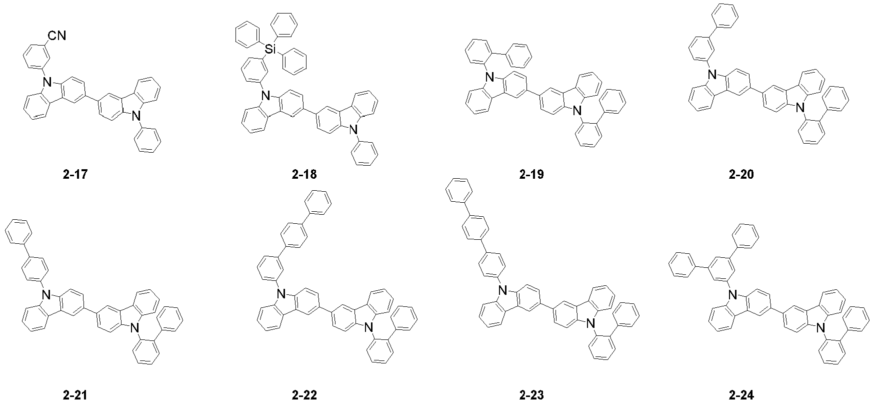

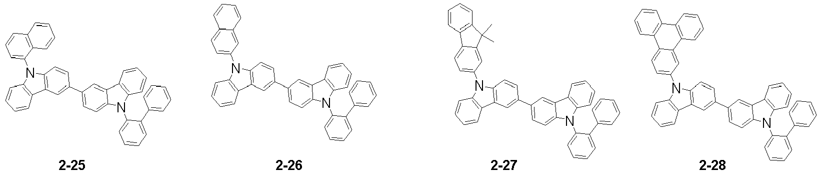

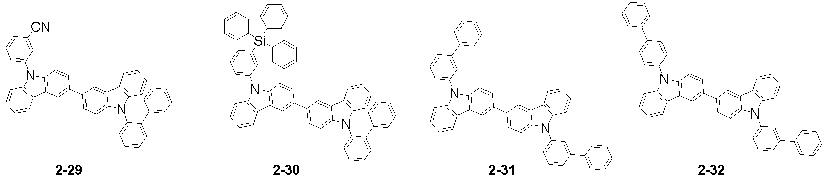

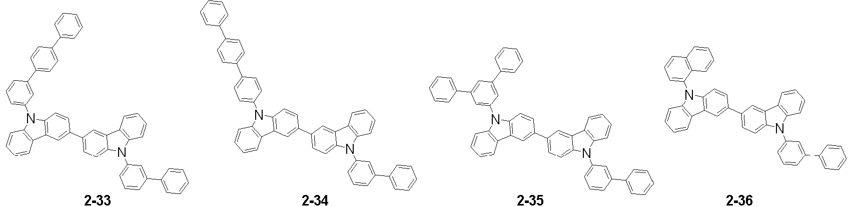

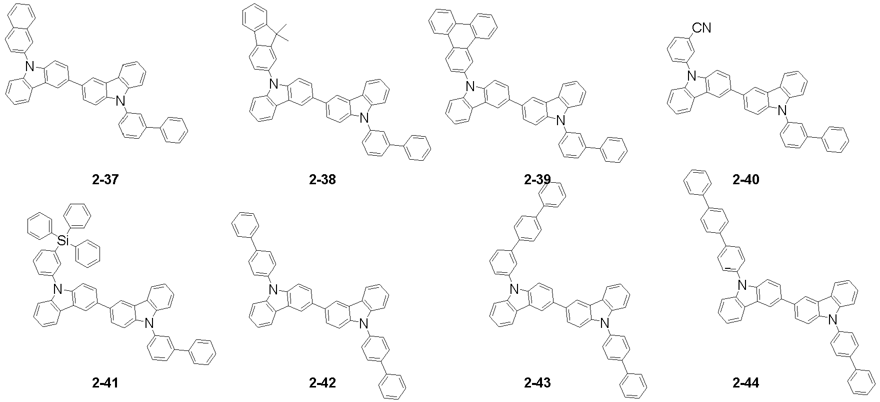

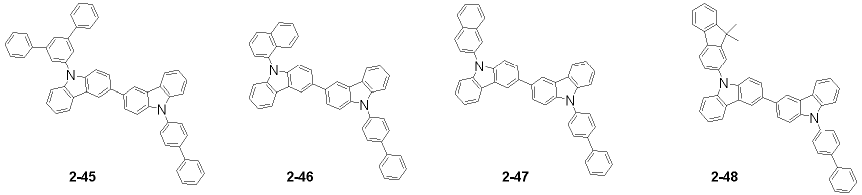

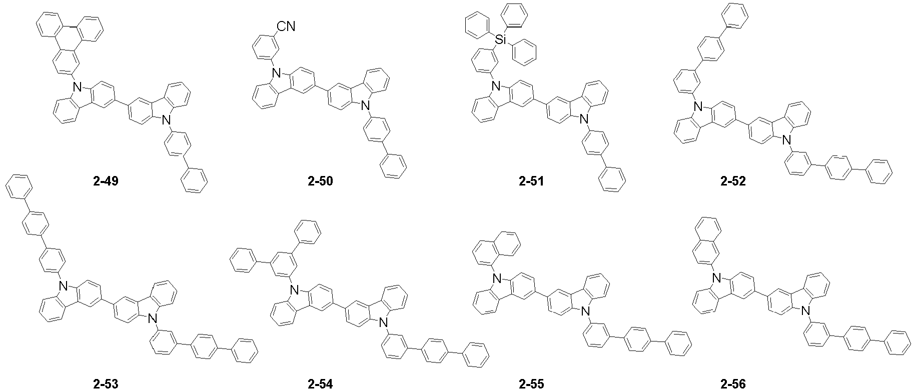

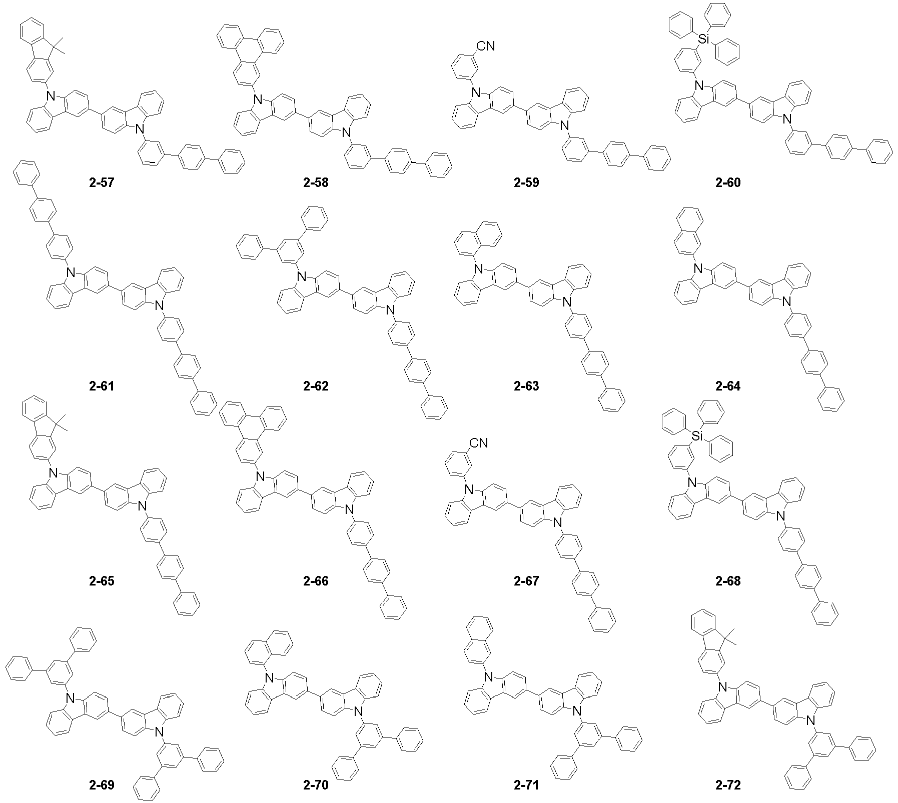

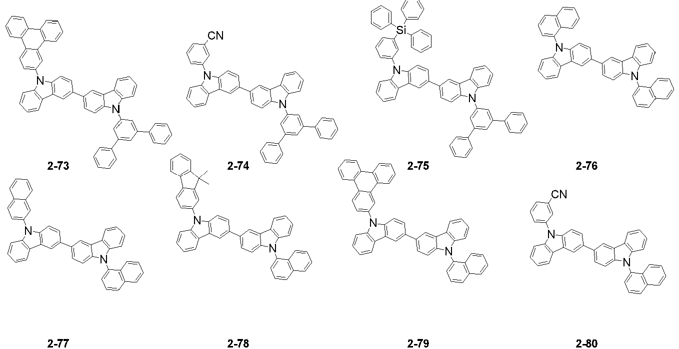

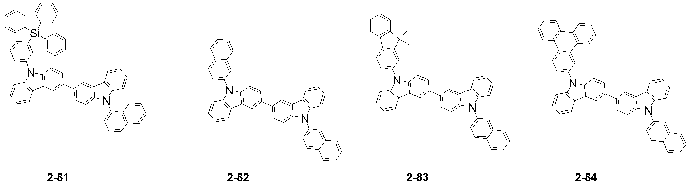

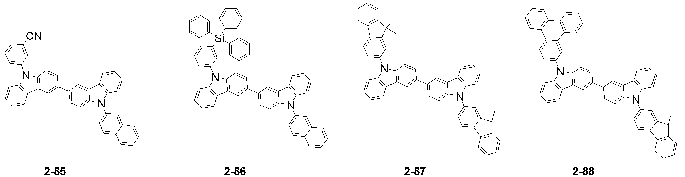

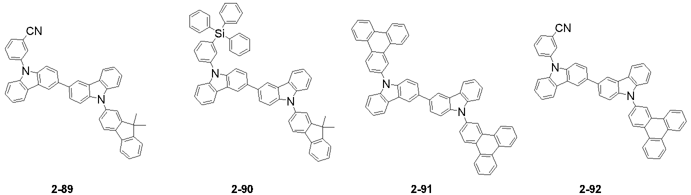

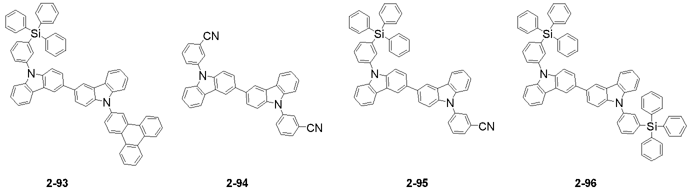

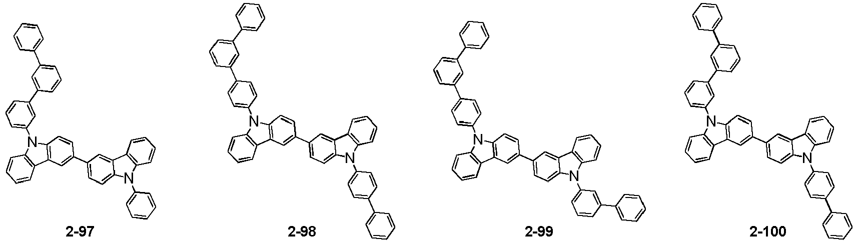

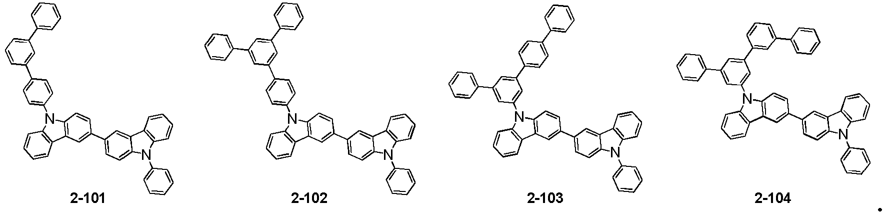

6. A composition for an organic material layer of an organic light emitting device, the composition comprising:
a hetero-cyclic compound represented by the following Chemical Formula 1; and
a compound represented by the following Chemical Formula 2 at the same time: 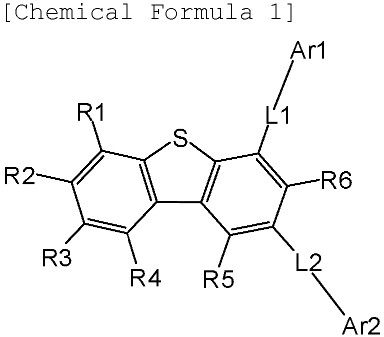 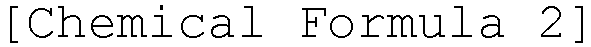 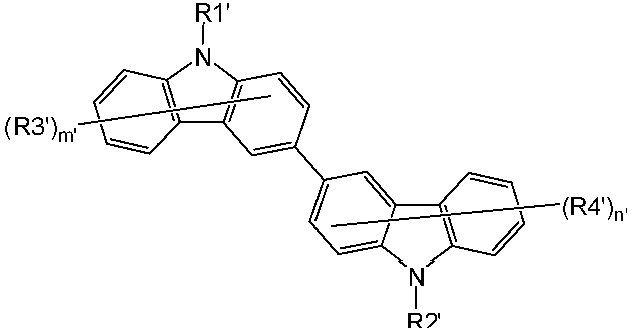
in Chemical Formulae 1 and 2,
L1 and L2 are the same as or different from each other, and each independently a direct bond, or a substituted or unsubstituted C₆ to C₆₀ arylene group;
Ar1 is a pyridine group unsubstituted or substituted with a phenyl group; a triazine group unsubstituted or substituted with one or more substituents selected from the group consisting of a phenyl group, a biphenyl group, a dibenzothiophene group and a dimethylfluorene group; a quinoline group unsubstituted or substituted with a phenyl group; a quinazoline group unsubstituted or substituted with a phenyl group or a biphenyl group; a phenanthroline group; or a benzimidazole group unsubstituted or substituted with a phenyl group,
Ar2 is represented by any one of the following Chemical Formulae 3 and 4; 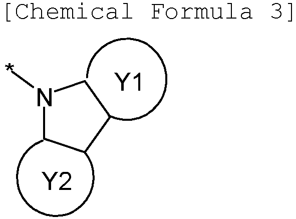 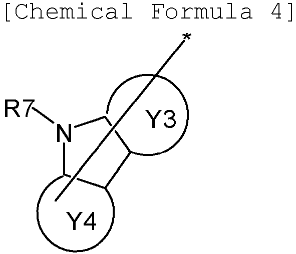
Y1 to Y4 are the same as or different from each other, and each independently a substituted or unsubstituted C₆ to C₆₀ aromatic hydrocarbon ring; or a substituted or unsubstituted C₂ to C₆₀ aromatic heteroring;
R1 to R7, R3' and R4' are the same as or different from each other, and each independently selected from the group consisting of hydrogen; deuterium; a halogen group; -CN; a substituted or unsubstituted C₁ to C₆₀ alkyl group; a substituted or unsubstituted C₂ to C₆₀ alkenyl group; a substituted or unsubstituted C₂ to C₆₀ alkynyl group; a substituted or unsubstituted C₁ to C₆₀ alkoxy group; a substituted or unsubstituted C₃ to C₆₀ cycloalkyl group; a substituted or unsubstituted C₂ to C₆₀ heterocycloalkyl group; a substituted or unsubstituted C₆ to C₆₀ aryl group; a substituted or unsubstituted C₂ to C₆₀ heteroaryl group; - SiRR'R"; -P(=O)RR' ; and an amine group unsubstituted or substituted with a C₁ to C₂₀ alkyl group, a substituted or unsubstituted C₆ to C₆₀ aryl group, or a C₂ to C₆₀ heteroaryl group, or two or more groups adjacent to each other bond to each other to form a substituted or unsubstituted aliphatic or aromatic hydrocarbon ring;
R1' is represented by -(L1')p'-(Ar1')q', and R2' is represented by -(L2')r'-(Ar2')s';
L1' and L2' are each independently a direct bond, or a substituted or unsubstituted C₆ to C₆₀ arylene group;
Ar1' and Ar2' are each independently -CN; a substituted or unsubstituted C₁ to C₆₀ alkyl group; -SiRR'R"; a substituted or unsubstituted C₆ to C₆₀ aryl group,
wherein Chemical Formula 3 is represented by any one of the following structural formulae: 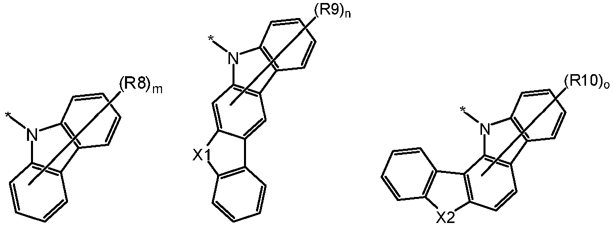 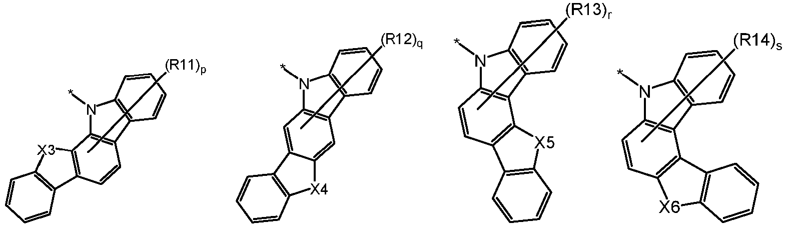
in the structural formulae, X1 to X6 are the same as or different from each other, and each independently NR, S, O or CR'R";
R8 to R14 are the same as or different from each other, and each independently selected from the group consisting of hydrogen; deuterium; a halogen group; -CN; a substituted or unsubstituted C₁ to C₆₀ alkyl group; a substituted or unsubstituted C₂ to C₆₀ alkenyl group; a substituted or unsubstituted C₂ to C₆₀ alkynyl group; a substituted or unsubstituted C₁ to C₆₀ alkoxy group; a substituted or unsubstituted C₃ to C₆₀ cycloalkyl group; a substituted or unsubstituted C₂ to C₆₀ heterocycloalkyl group; a substituted or unsubstituted C₆ to C₆₀ aryl group; a substituted or unsubstituted C₂ to C₆₀ heteroaryl group; -SiRR'R"; -P(=O)RR'; and an amine group unsubstituted or substituted with a C₁ to C₂₀ alkyl group, a substituted or unsubstituted C₆ to C₆₀ aryl group, or a C₂ to C₆₀ heteroaryl group, or two or more groups adjacent to each other bond to each other to form a substituted or unsubstituted aliphatic or aromatic hydrocarbon ring;
R, R' and R" are the same as or different from each other, and each independently hydrogen; deuterium; -CN; a substituted or unsubstituted C₁ to C₆₀ alkyl group; a substituted or unsubstituted C₃ to C₆₀ cycloalkyl group; a substituted or unsubstituted C₆ to C₆₀ aryl group; or a substituted or unsubstituted C₂ to C₆₀ heteroaryl group;
p' and r' are each independently an integer of 0 to 4;
q' and s' are each independently an integer of 1 to 4; and
m' and n' are each independently an integer of 0 to 7,
m is an integer of 0 to 8, and n, o, p, q, r and s are each independently an integer of 0 to 6.

7. The composition for an organic material layer of an organic light emitting device of Claim 6, wherein a weight ratio of the hetero-cyclic compound represented by Chemical Formula 1:the compound represented by Chemical Formula 2 in the composition is from 1:10 to 10:1.

8. The composition for an organic material layer of an organic light emitting device of Claim 6, wherein Chemical Formula 1 is represented by any one of the following Chemical Formulae 5 to 10: 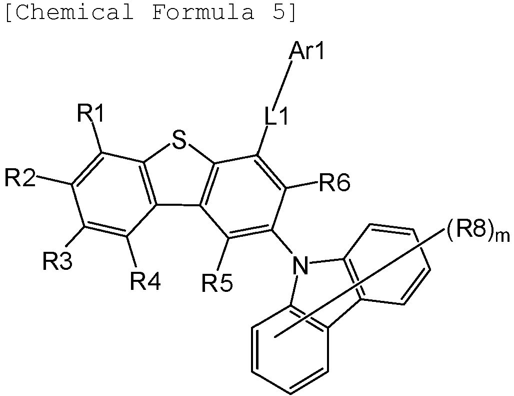 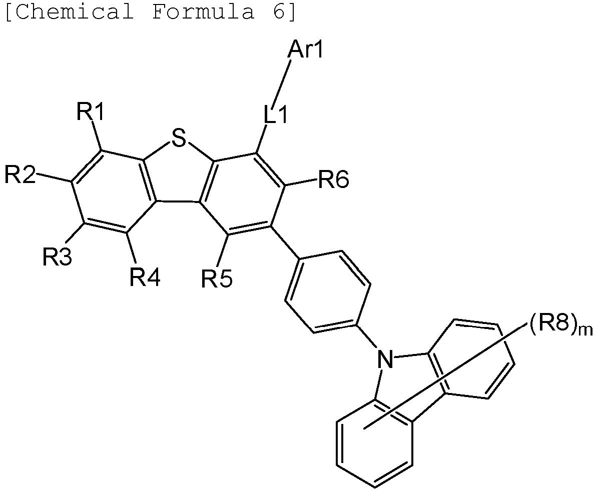 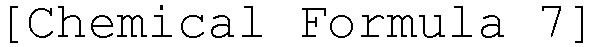 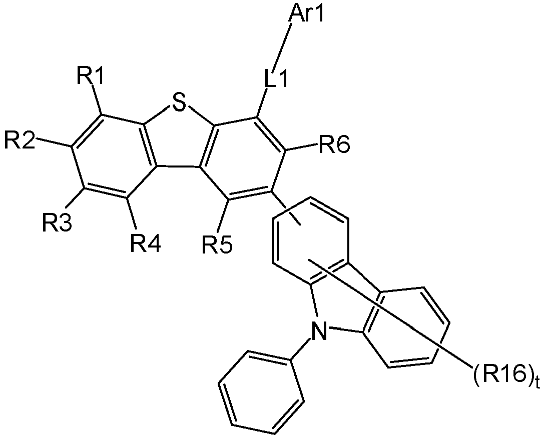 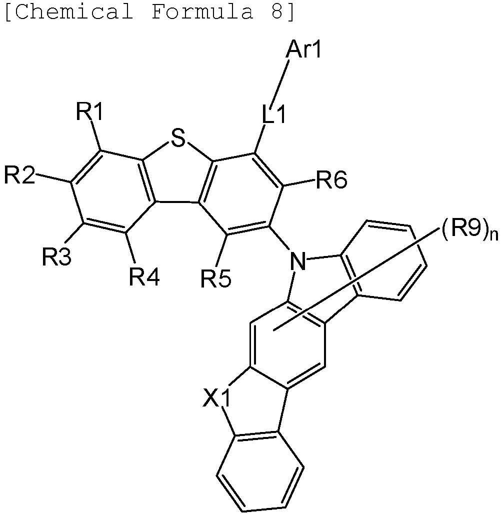 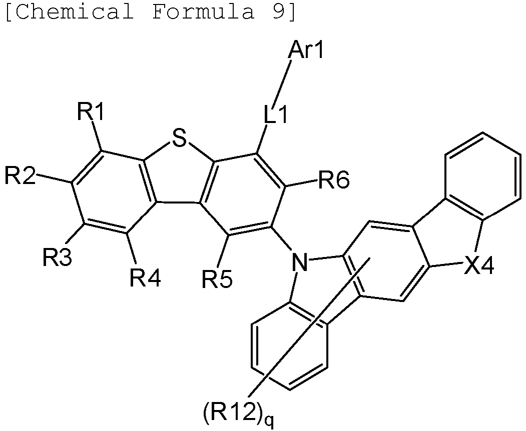 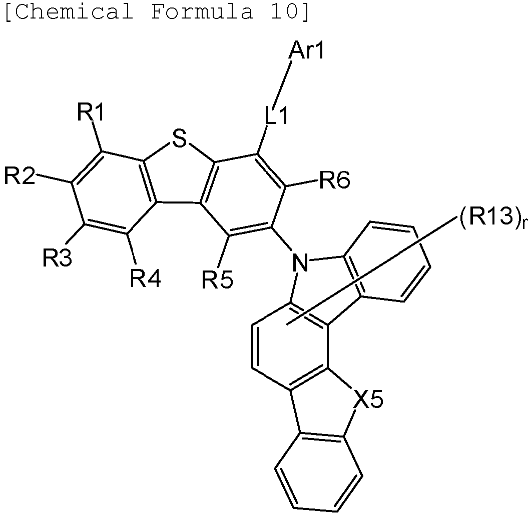 in Chemical Formulae 5 to 10,
R1 to R6, L1 and Ar1 have the same definitions as in Chemical Formula 1;
X1, X4 and X5 are the same as or different from each other, and each independently NR, S, O or CR'R";
R8, R9, R12, R13 and R16 are the same as or different from each other, and each independently selected from the group consisting of hydrogen; deuterium; a halogen group; -CN; a substituted or unsubstituted C₁ to C₆₀ alkyl group; a substituted or unsubstituted C₂ to C₆₀ alkenyl group; a substituted or unsubstituted C₂ to C₆₀ alkynyl group; a substituted or unsubstituted C₁ to C₆₀ alkoxy group; a substituted or unsubstituted C₃ to C₆₀ cycloalkyl group; a substituted or unsubstituted C₂ to C₆₀ heterocycloalkyl group; a substituted or unsubstituted C₆ to C₆₀ aryl group; a substituted or unsubstituted C₂ to C₆₀ heteroaryl group; - SiRR'R"; -P(=O)RR'; and an amine group unsubstituted or substituted with a C₁ to C₂₀ alkyl group, a substituted or unsubstituted C₆ to C₆₀ aryl group, or a C₂ to C₆₀ heteroaryl group, or two or more groups adjacent to each other bond to each other to form a substituted or unsubstituted aliphatic or aromatic hydrocarbon ring;
R, R' and R" are the same as or different from each other, and each independently hydrogen; deuterium; -CN; a substituted or unsubstituted C₁ to C₆₀ alkyl group; a substituted or unsubstituted C₃ to C₆₀ cycloalkyl group; a substituted or unsubstituted C₆ to C₆₀ aryl group; or a substituted or unsubstituted C₂ to C₆₀ heteroaryl group; and
m is an integer of 0 to 8, n, q and r are each independently an integer of 0 to 6, and t is an integer of 0 to 7.

## Patentansprüche

1. Organische lichtemittierende Vorrichtung, umfassend:
eine Anode (200);
eine Kathode (400); und
eine lichtemittierende Schicht (303), die zwischen der Anode und der Kathode vorgesehen ist,
wobei die lichtemittierende Schicht (303) eine heterocyclische Verbindung, die durch die folgende chemische Formel 1 dargestellt ist, und eine Verbindung, die durch die folgende chemische Formel 2 dargestellt ist, gleichzeitig umfasst: 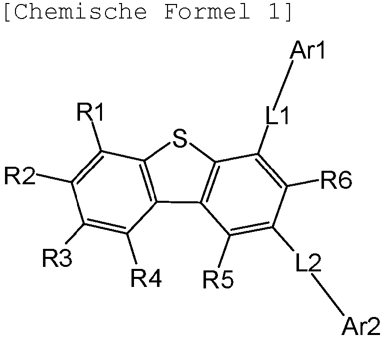 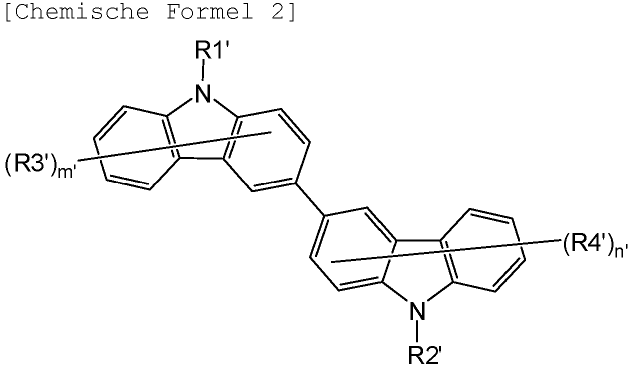
wobei in den chemischen Formeln 1 und 2
L1 und L2 gleich oder verschieden voneinander sind und jeweils unabhängig eine direkte Bindung oder eine substituierte oder unsubstituierte C₆- bis C₆₀-Arylengruppe sind;
Ar1 eine Pyridingruppe, unsubstituiert oder substituiert mit einer Phenylgruppe; eine Triazingruppe, unsubstituiert oder substituiert mit einem oder mehreren Substituenten ausgewählt aus der Gruppe bestehend aus einer Phenylgruppe, einer Biphenylgruppe, einer Dibenzothiophengruppe und einer Dimethylfluorengruppe ; eine Chinolingruppe, unsubstituiert oder substituiert mit einer Phenylgruppe; eine Chinazolingruppe, unsubstituiert oder substituiert mit einer Phenylgruppe oder einer Biphenylgruppe; eine Phenanthrolingruppe; oder eine Benzimidazolgruppe, unsubstituiert oder substituiert mit einer Phenylgruppe, ist;
Ar2 durch eine der folgenden chemischen Formeln 3 und 4 dargestellt ist; 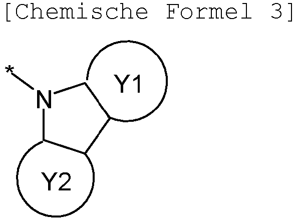 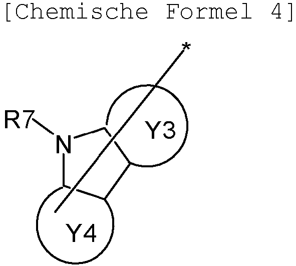
wobei Y1 bis Y4 gleich oder verschieden voneinander sind und jeweils unabhängig ein substituierter oder unsubstituierter aromatischer C₆- bis C₆₀-Kohlenwasserstoffring; oder ein substituierter oder unsubstituierter aromatischer C₂- bis C₆₀-Heteroring sind;
R1 bis R7, R3' und R4' gleich oder verschieden voneinander sind und jeweils unabhängig ausgewählt sind aus der Gruppe bestehend aus Wasserstoff; Deuterium; einer Halogengruppe; -CN; einer substituierten oder unsubstituierten C₁- bis C₆₀-Alkylgruppe; einer substituierten oder unsubstituierten C₂- bis C₆₀-Alkenylgruppe; einer substituierten oder unsubstituierten C₂-bis C₆₀-Alkinylgruppe; einer substituierten oder unsubstituierten C₁- bis C₆₀-Alkoxygruppe; einer substituierten oder unsubstituierten C₃- bis C₆₀-Cycloalkylgruppe; einer substituierten oder unsubstituierten C₂- bis C₆₀-Heterocycloalkylgruppe; einer substituierten oder unsubstituierten C₆- bis C₆₀-Arylgruppe; einer substituierten oder unsubstituierten C₂- bis C₆₀-Heteroarylgruppe; -SiRR'R"; - P(=O)RR'; und einer Amingruppe, unsubstituiert oder substituiert mit einer C₁- bis C₂₀-Alkylgruppe, einer substituierten oder unsubstituierten C₆- bis C₆₀-Arylgruppe oder einer C₂- bis C₆₀-Heteroarylgruppe, oder zwei oder mehr Gruppen, die aneinander angrenzen, aneinander binden, um einen substituierten oder unsubstituierten aliphatischen oder aromatischen Kohlenwasserstoffring zu bilden;
R1' durch -(L1')p'-(Ar1')q' dargestellt ist und R2' durch -(L2')r'-(Ar2')s' dargestellt ist;
L1' und L2' jeweils unabhängig eine direkte Bindung oder eine substituierte oder unsubstituierte C₆ - bis C₆₀ - Arylengruppe sind;
Ar1' und Ar2' jeweils unabhängig -CN; eine substituierte oder unsubstituierte C₁- bis C₆₀-Alkylgruppe; -SiRR'R"; eine substituierte oder unsubstituierte C₆- bis C₆₀-Arylgruppe sind,
wobei die chemische Formel 3 durch eine der folgenden Strukturformeln dargestellt ist: 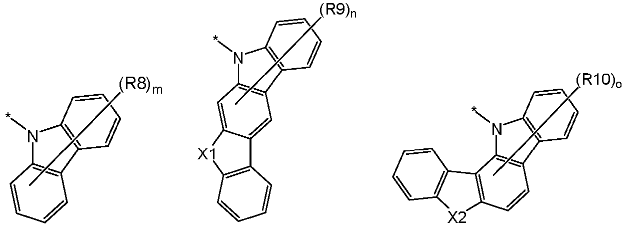 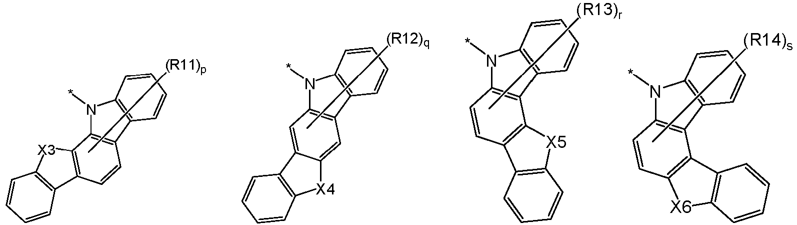
wobei in den Strukturformeln X1 bis X6 gleich oder verschieden voneinander und jeweils unabhängig NR, S, O oder CR'R" sind;
R8 bis R14 gleich oder verschieden voneinander sind und jeweils unabhängig ausgewählt sind aus der Gruppe bestehend aus Wasserstoff; Deuterium; einer Halogengruppe; -CN; einer substituierten oder unsubstituierten C₁- bis C₆₀-Alkylgruppe; einer substituierten oder unsubstituierten C₂- bis C₆₀-Alkenylgruppe; einer substituierten oder unsubstituierten C₂-bis C₆₀-Alkinylgruppe; einer substituierten oder unsubstituierten C₁- bis C₆₀-Alkoxygruppe; einer substituierten oder unsubstituierten C₃- bis C₆₀-Cycloalkylgruppe; einer substituierten oder unsubstituierten C₂- bis C₆₀-Heterocycloalkylgruppe; einer substituierten oder unsubstituierten C₆- bis C₆₀-Arylgruppe; einer substituierten oder unsubstituierten C₂- bis C₆₀-Heteroarylgruppe; -SiRR'R"; - P(=O)RR'; und einer Amingruppe, unsubstituiert oder substituiert mit einer C₁- bis C₂₀-Alkylgruppe, einer substituierten oder unsubstituierten C₆- bis C₆₀-Arylgruppe oder einer C₂- bis C₆₀-Heteroarylgruppe, oder zwei oder mehr Gruppen, die aneinander angrenzen, aneinander binden, um einen substituierten oder unsubstituierten aliphatischen oder aromatischen Kohlenwasserstoffring zu bilden;
R, R' und R" gleich oder verschieden voneinander sind und jeweils unabhängig Wasserstoff; Deuterium; -CN; eine substituierte oder unsubstituierte C₁- bis C₆₀-Alkylgruppe; eine substituierte oder unsubstituierte C₃- bis C₆₀-Cycloalkylgruppe; eine substituierte oder unsubstituierte C₆- bis C₆₀-Arylgruppe; oder eine substituierte oder unsubstituierte C₂- bis C₆₀-Heteroarylgruppe sind;
p' und r' jeweils unabhängig eine ganze Zahl von 0 bis 4 sind;
q' und s' jeweils unabhängig eine ganze Zahl von 1 bis 4 sind; und
m' und n' jeweils unabhängig eine ganze Zahl von 0 bis 7 sind,
m eine ganze Zahl von 0 bis 8 ist und n, o, p, q, r und s jeweils unabhängig eine ganze Zahl von 0 bis 6 sind.

2. Organische lichtemittierende Vorrichtung nach Anspruch 1, wobei die chemische Formel 4 durch eine der folgenden Strukturformeln dargestellt ist: 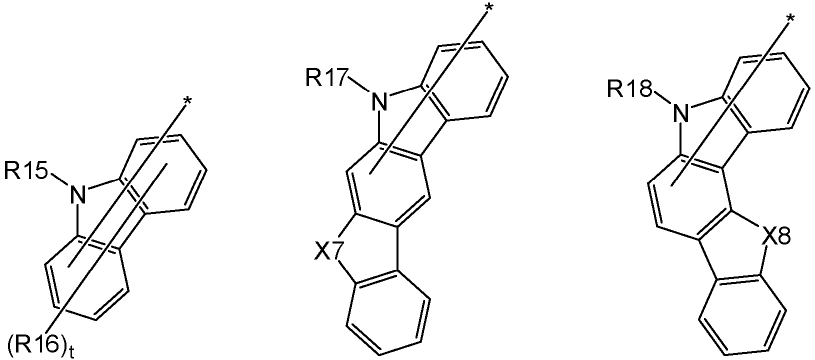
wobei in den Strukturformeln X7 und X8 gleich oder verschieden voneinander und jeweils unabhängig NR, S, O oder CR'R" sind;
R15 bis R18 gleich oder verschieden voneinander sind und jeweils unabhängig ausgewählt sind aus der Gruppe bestehend aus Wasserstoff; Deuterium; einer Halogengruppe; -CN; einer substituierten oder unsubstituierten C₁- bis C₆₀-Alkylgruppe; einer substituierten oder unsubstituierten C₂- bis C₆₀-Alkenylgruppe; einer substituierten oder unsubstituierten C₂-bis C₆₀-Alkinylgruppe; einer substituierten oder unsubstituierten C₁- bis C₆₀-Alkoxygruppe; einer substituierten oder unsubstituierten C₃- bis C₆₀-Cycloalkylgruppe; einer substituierten oder unsubstituierten C₂- bis C₆₀-Heterocycloalkylgruppe; einer substituierten oder unsubstituierten C₆- bis C₆₀-Arylgruppe; einer substituierten oder unsubstituierten C₂- bis C₆₀-Heteroarylgruppe; -SiRR'R"; - P(=O)RR'; und einer Amingruppe, unsubstituiert oder substituiert mit einer C₁- bis C₂₀-Alkylgruppe, einer substituierten oder unsubstituierten C₆- bis C₆₀-Arylgruppe oder einer C₂- bis C₆₀-Heteroarylgruppe, oder zwei oder mehr Gruppen, die aneinander angrenzen, aneinander binden, um einen substituierten oder unsubstituierten aliphatischen oder aromatischen Kohlenwasserstoffring zu bilden;
R, R' und R" gleich oder verschieden voneinander sind und jeweils unabhängig Wasserstoff; Deuterium; -CN; eine substituierte oder unsubstituierte C₁- bis C₆₀-Alkylgruppe; eine substituierte oder unsubstituierte C₃- bis C₆₀-Cycloalkylgruppe; eine substituierte oder unsubstituierte C₆- bis C₆₀-Arylgruppe; oder eine substituierte oder unsubstituierte C₂- bis C₆₀-Heteroarylgruppe sind; und
t eine ganze Zahl von 0 bis 7 ist.

3. Organische lichtemittierende Vorrichtung nach Anspruch 1, wobei die chemische Formel 1 durch eine der folgenden chemischen Formeln 5 bis 10 dargestellt ist: 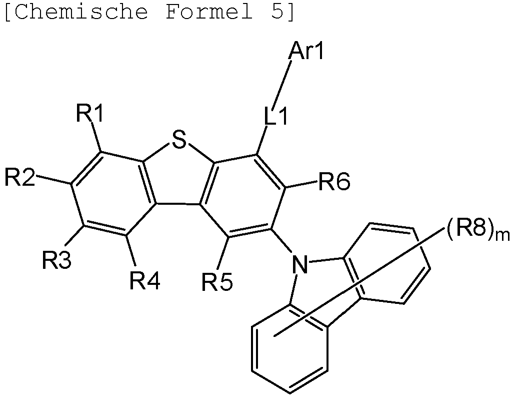 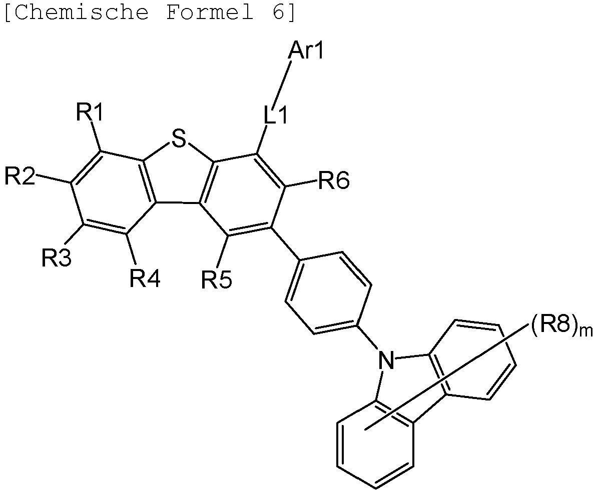 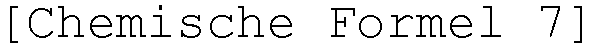 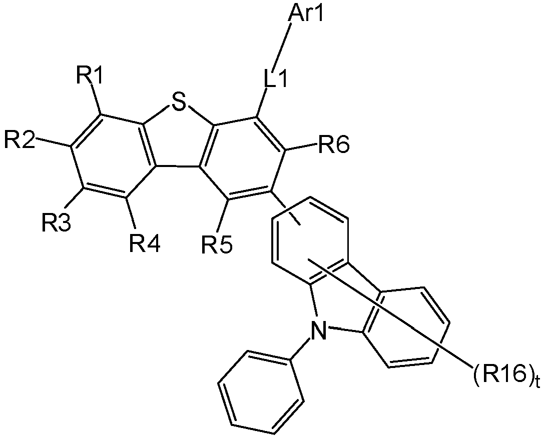 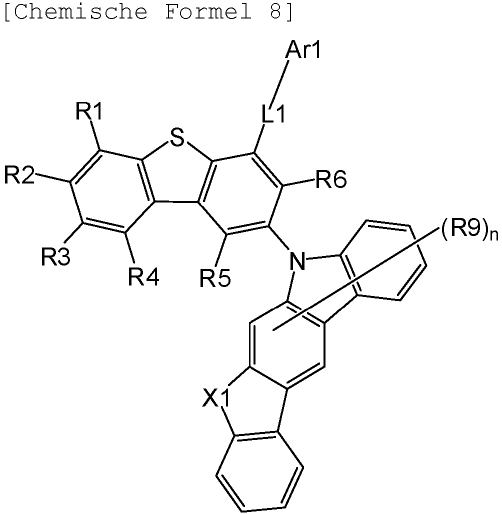 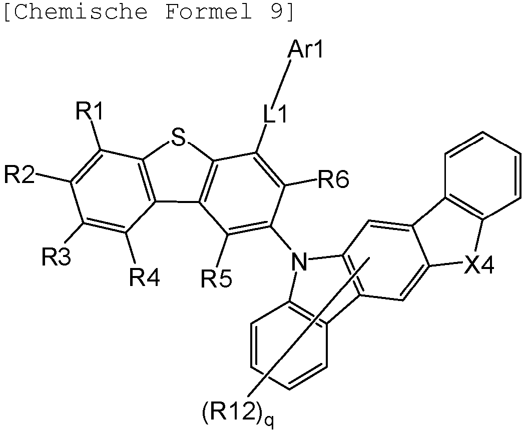 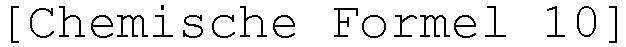 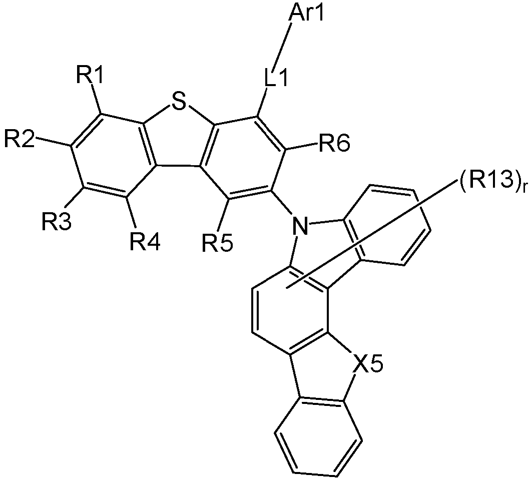 wobei in den Chemischen Formeln 5 bis 10
R1 bis R6, L1 und Ar1 die gleichen Definitionen wie in der Chemischen Formel 1 haben;
X1, X4 und X5 gleich oder verschieden voneinander und jeweils unabhängig NR, S, O oder CR'R" sind;
R8, R9, R12, R13 und R16 gleich oder verschieden voneinander sind und jeweils unabhängig ausgewählt sind aus der Gruppe bestehend aus Wasserstoff; Deuterium; einer Halogengruppe; -CN; einer substituierten oder unsubstituierten C₁- bis C₆₀-Alkylgruppe; einer substituierten oder unsubstituierten C₂- bis C₆₀-Alkenylgruppe; einer substituierten oder unsubstituierten C₂- bis C₆₀-Alkinylgruppe; einer substituierten oder unsubstituierten C₁- bis C₆₀-Alkoxygruppe; einer substituierten oder unsubstituierten C₃- bis C₆₀-Cycloalkylgruppe; einer substituierten oder unsubstituierten C₂-bis C₆₀-Heterocycloalkylgruppe; einer substituierten oder unsubstituierten C₆- bis C₆₀-Arylgruppe; einer substituierten oder unsubstituierten C₂- bis C₆₀-Heteroarylgruppe; -SiRR'R"; - P(=O)RR'; und einer Amingruppe, unsubstituiert oder substituiert mit einer C₁- bis C₂₀-Alkylgruppe, einer substituierten oder unsubstituierten C₆- bis C₆₀-Arylgruppe oder einer C₂- bis C₆₀-Heteroarylgruppe, oder zwei oder mehr Gruppen, die aneinander angrenzen, aneinander binden, um einen substituierten oder unsubstituierten aliphatischen oder aromatischen Kohlenwasserstoffring zu bilden;
R, R' und R" gleich oder verschieden voneinander sind und jeweils unabhängig Wasserstoff; Deuterium; -CN; eine substituierte oder unsubstituierte C₁- bis C₆₀-Alkylgruppe; eine substituierte oder unsubstituierte C₃- bis C₆₀-Cycloalkylgruppe; eine substituierte oder unsubstituierte C₆- bis C₆₀-Arylgruppe; oder eine substituierte oder unsubstituierte C₂- bis C₆₀-Heteroarylgruppe sind; und
m eine ganze Zahl von 0 bis 8 ist, n, q und r jeweils unabhängig eine ganze Zahl von 0 bis 6 sind und t eine ganze Zahl von 0 bis 7 ist.

4. Organische lichtemittierende Vorrichtung nach Anspruch 1, wobei die chemische Formel 1 durch eine der folgenden Verbindungen dargestellt ist:

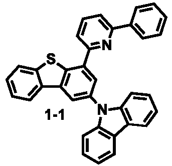

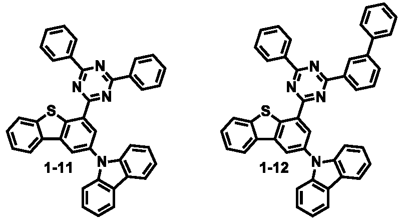

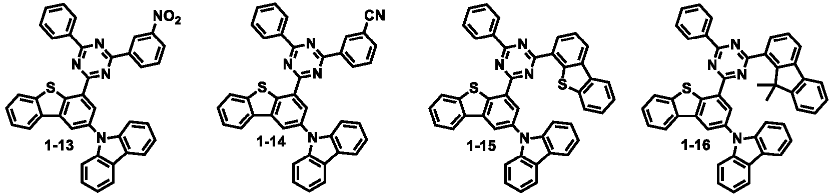

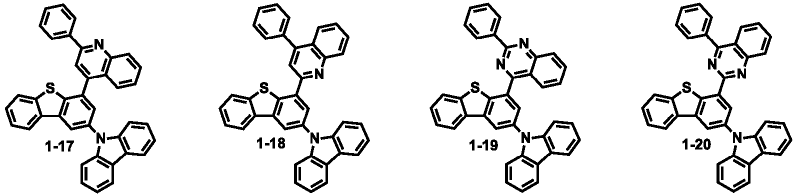

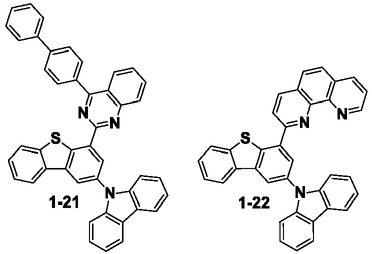

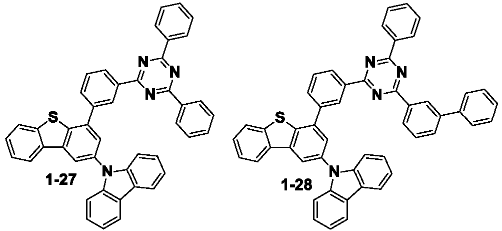

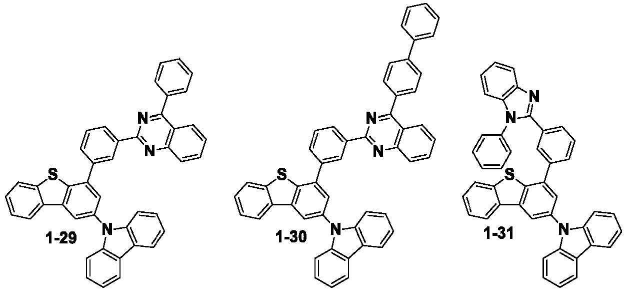

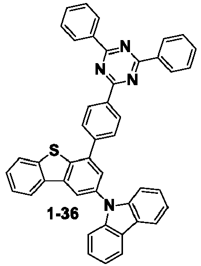

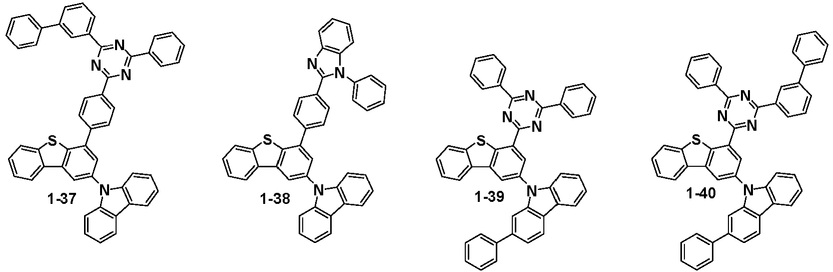

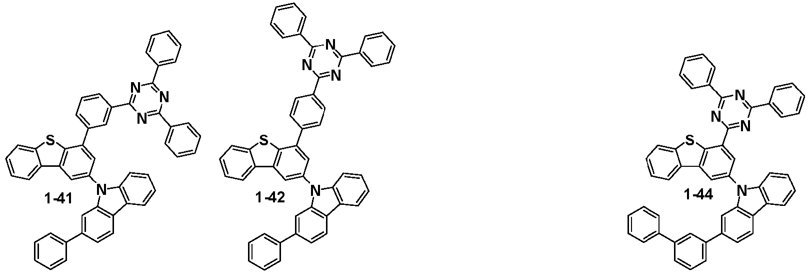

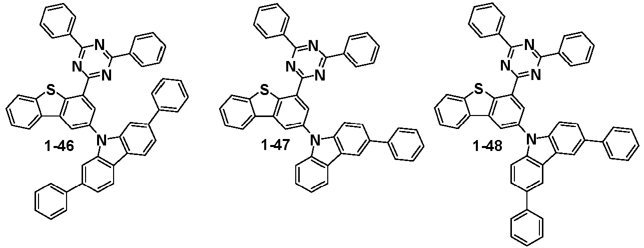

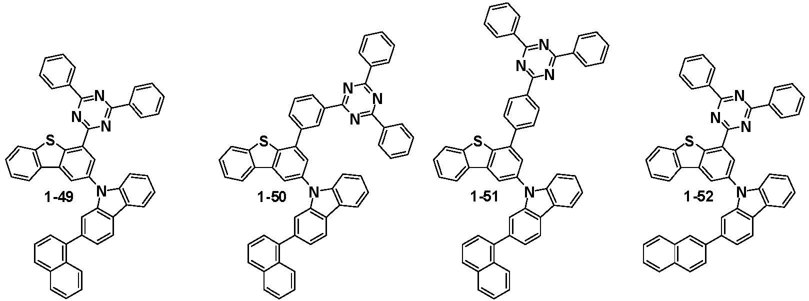

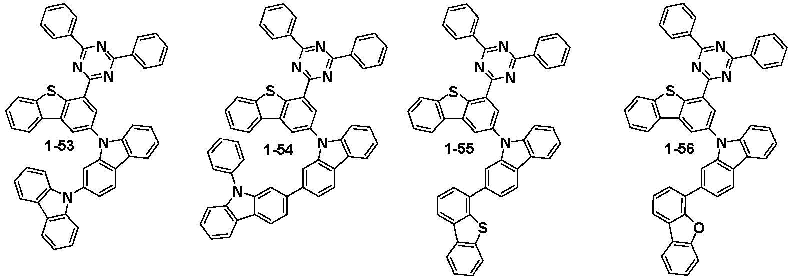

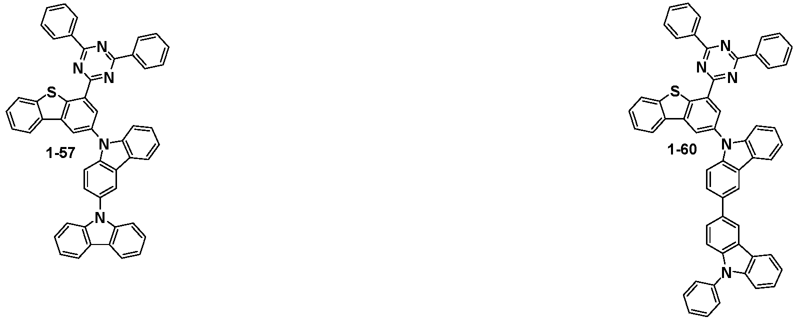

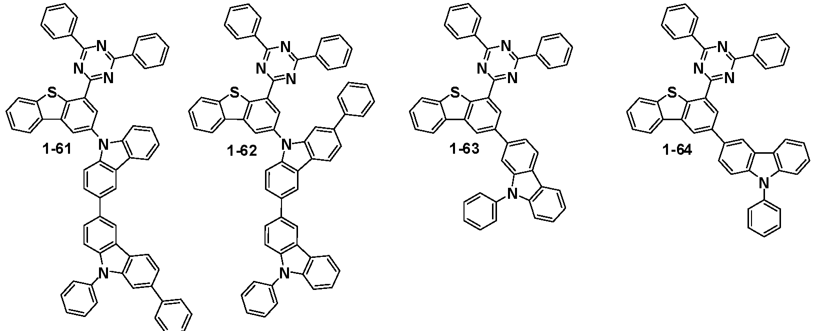

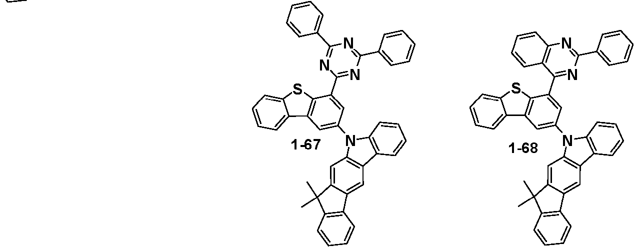

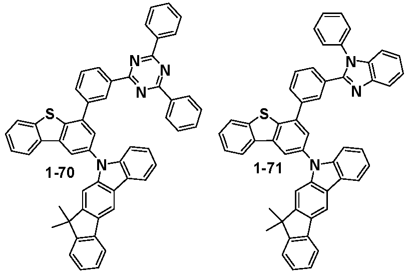

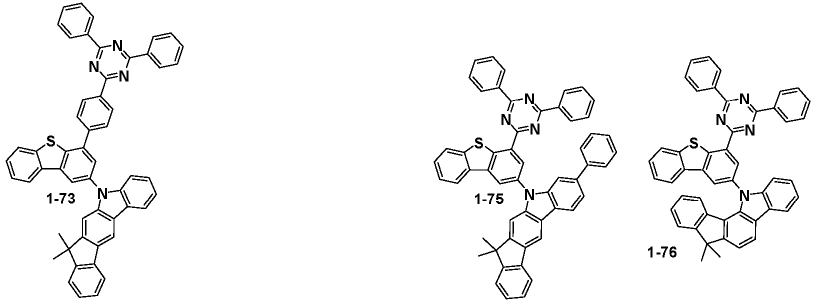

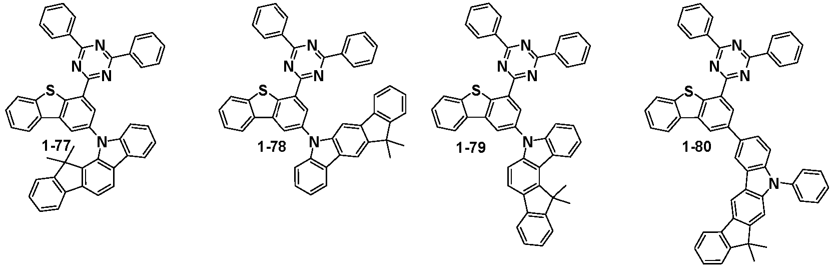

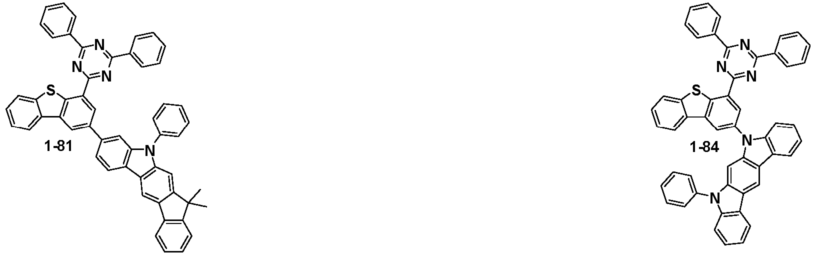

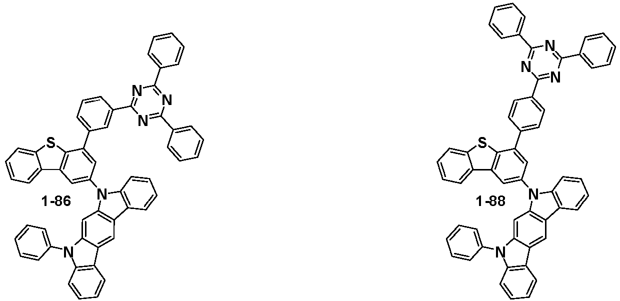

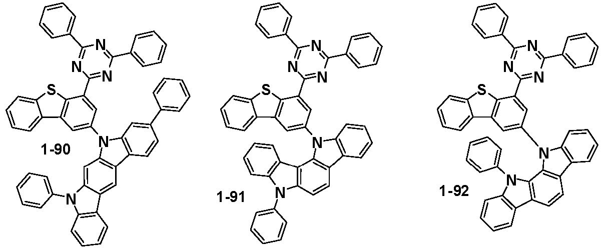

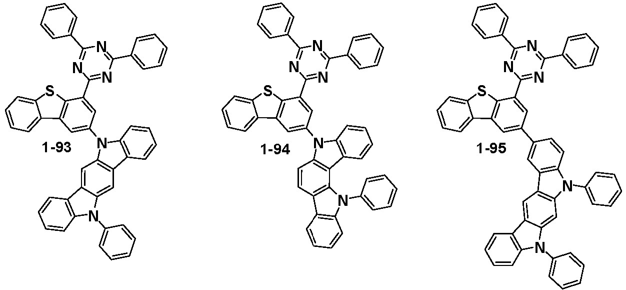

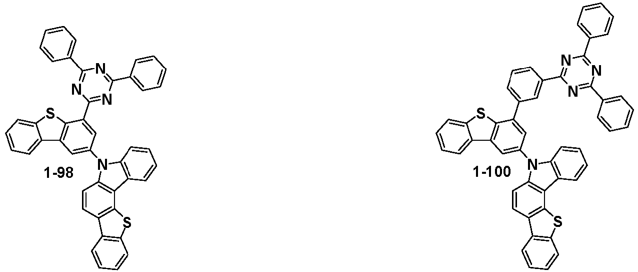

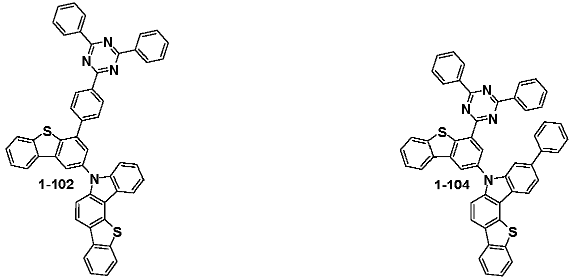

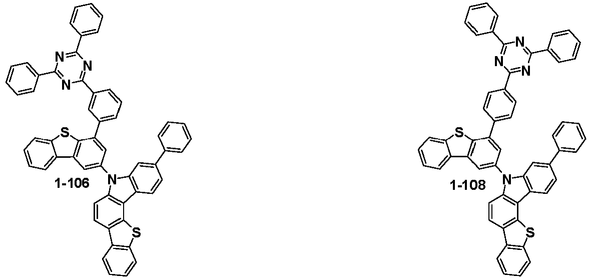

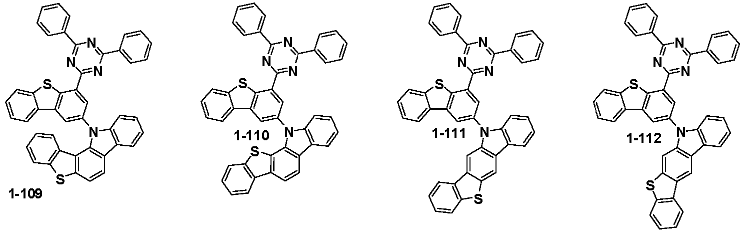

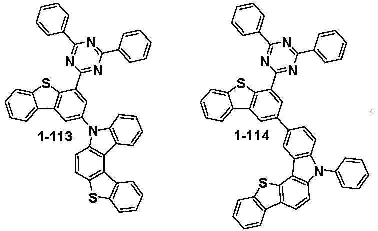

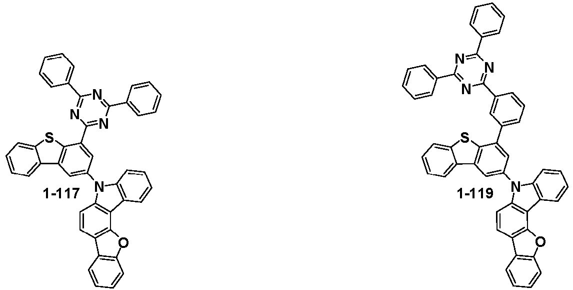

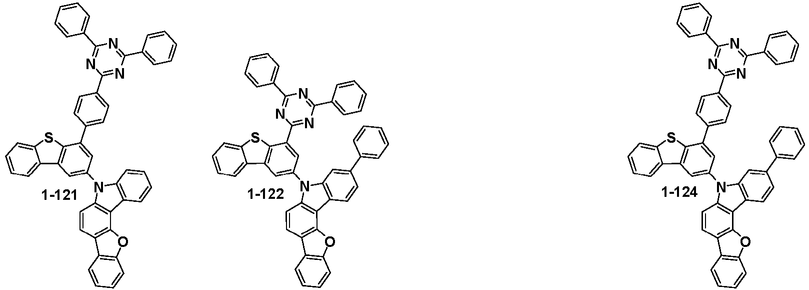

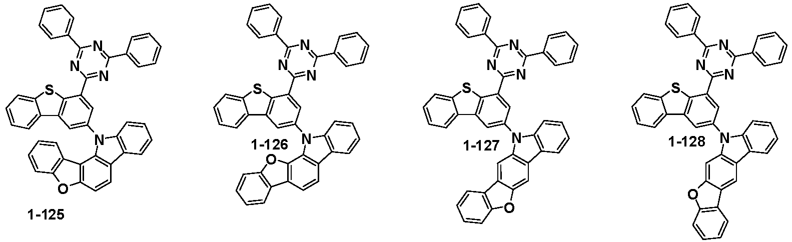

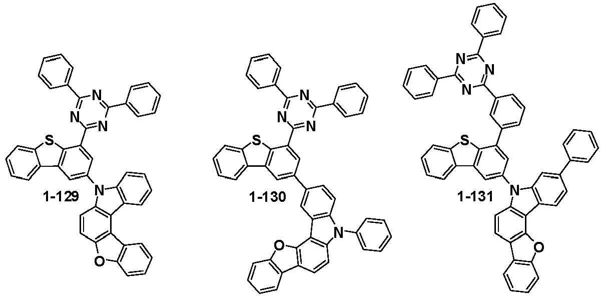

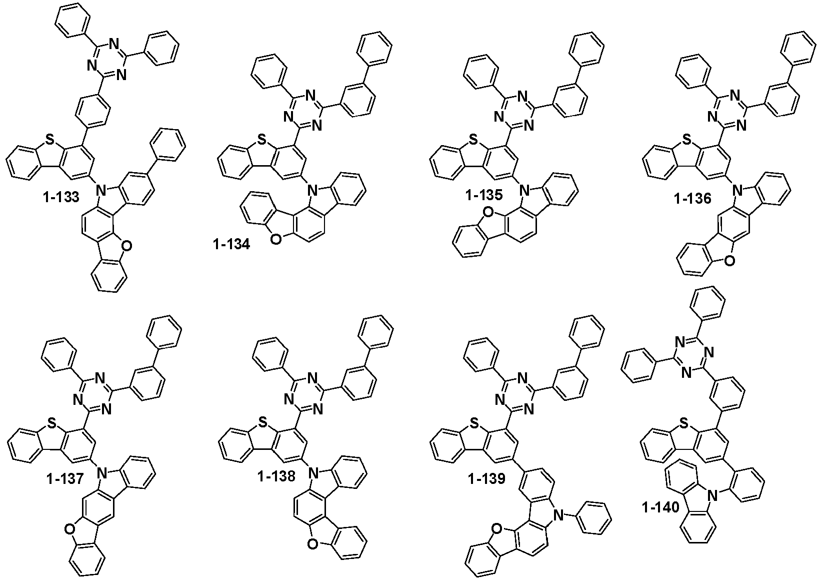

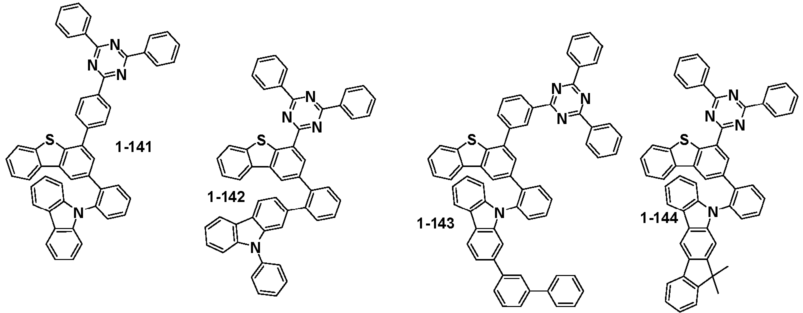

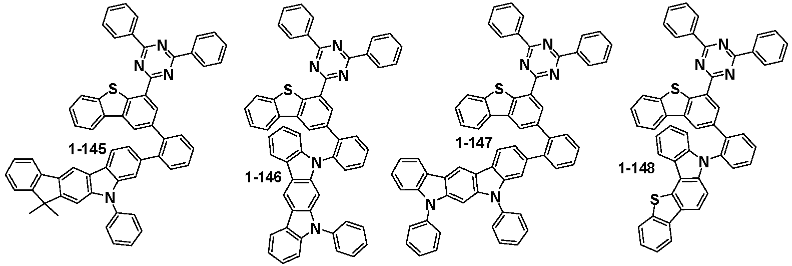

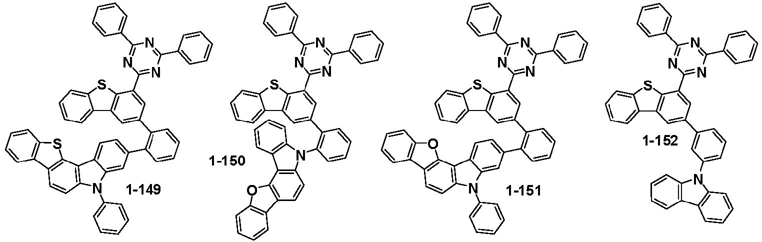

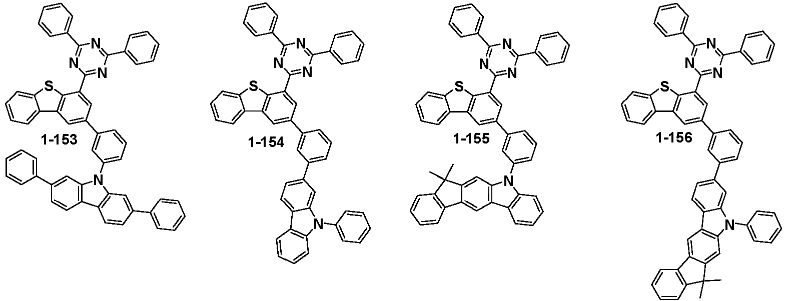

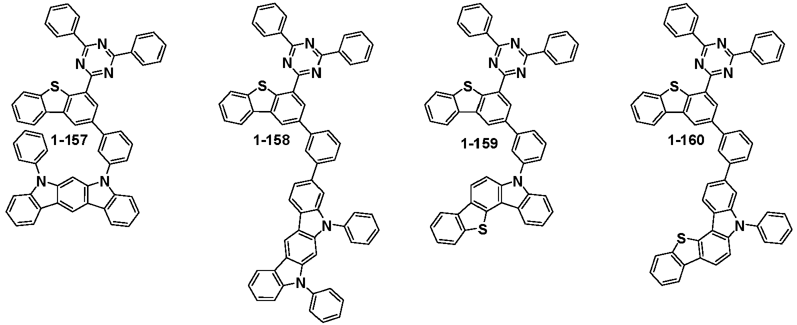

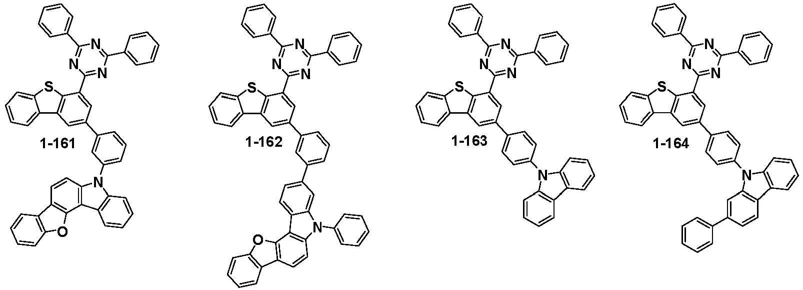

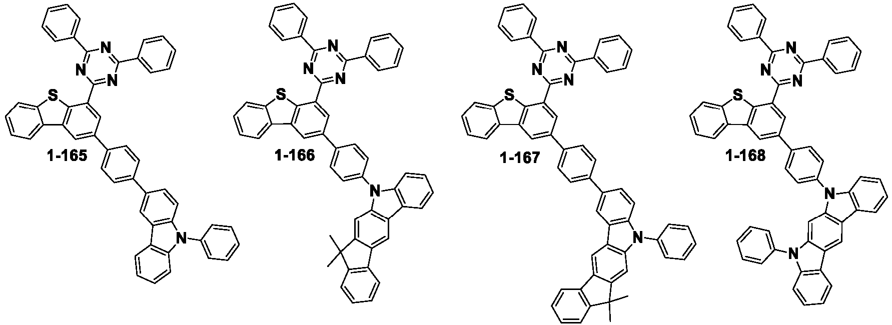

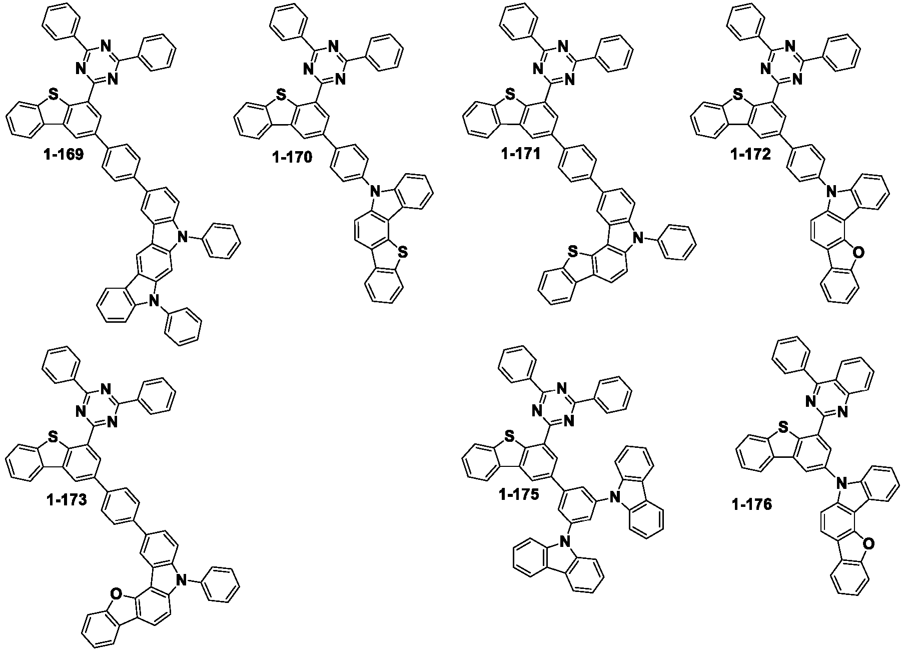

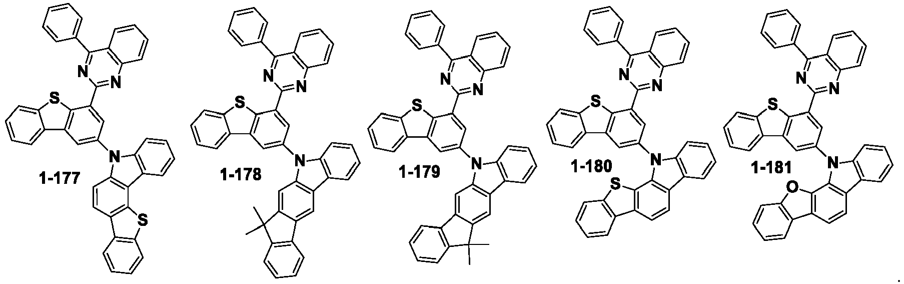

5. Organische lichtemittierende Vorrichtung nach Anspruch 1, wobei die chemische Formel 2 durch eine der folgenden Verbindungen dargestellt ist:

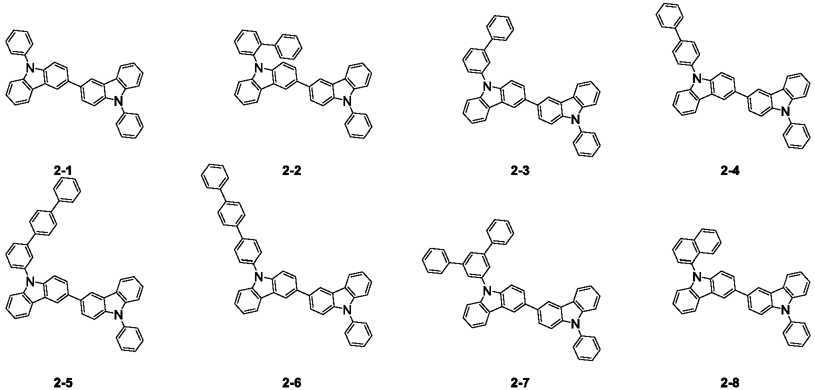

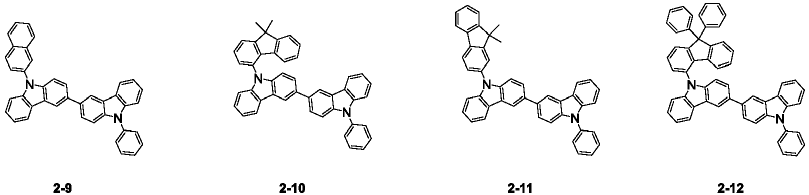

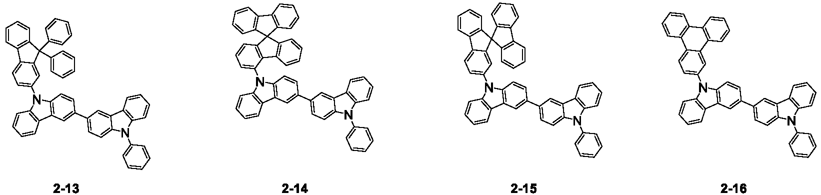

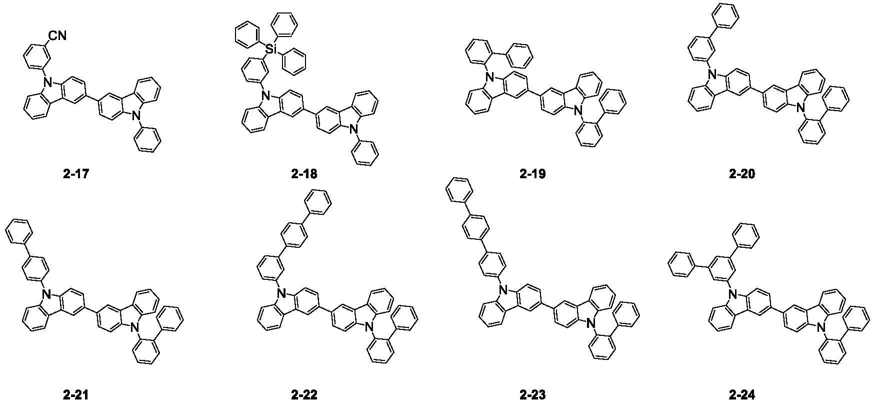

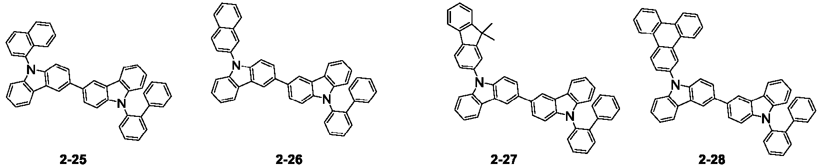

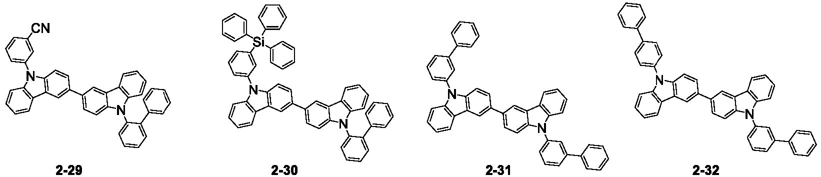

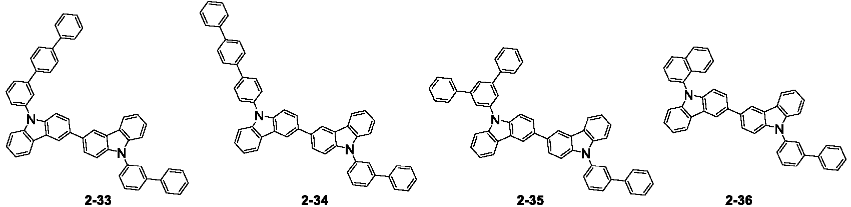

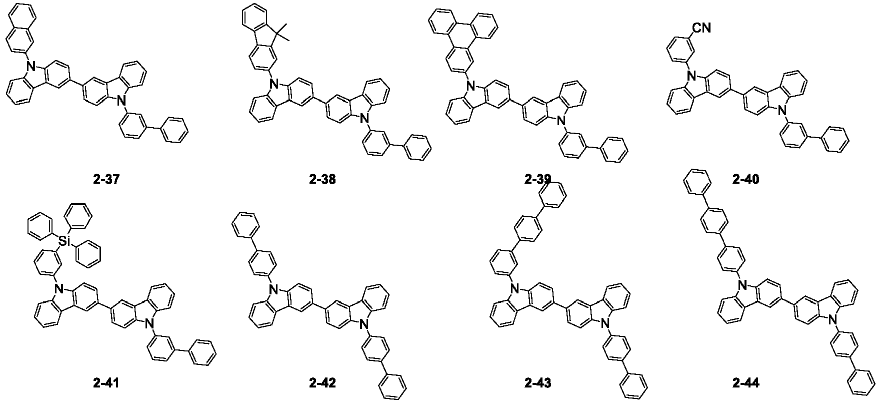

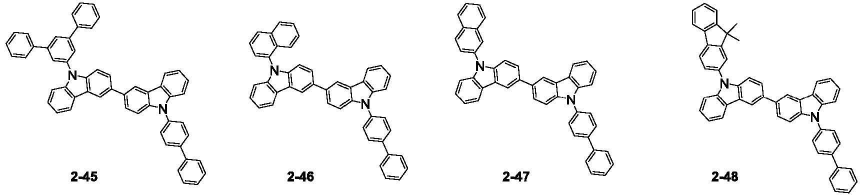

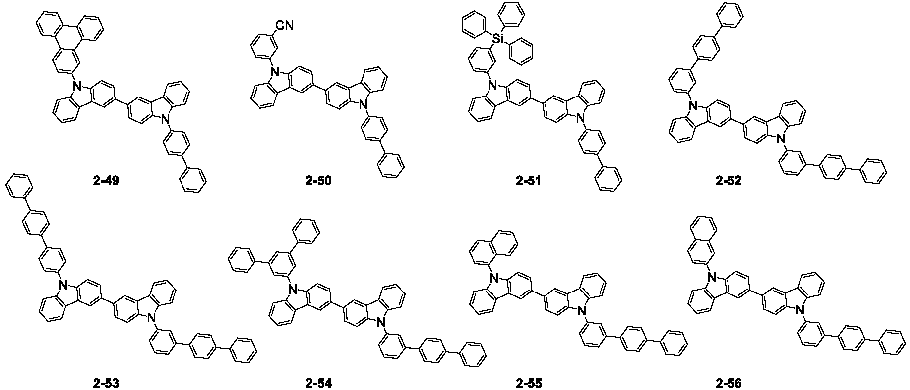

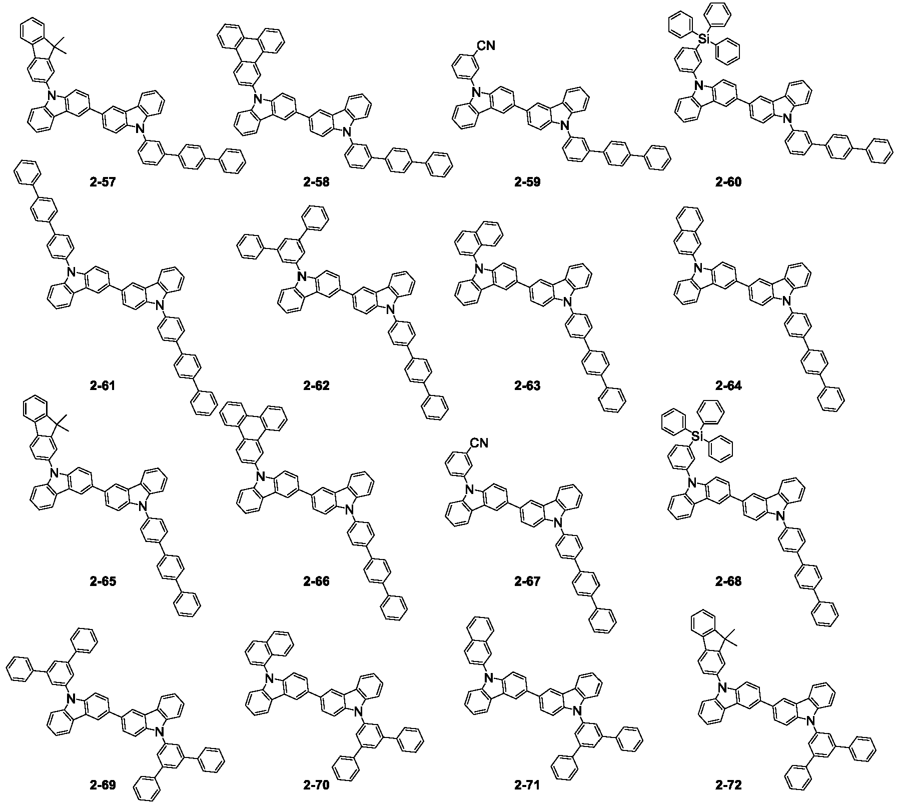

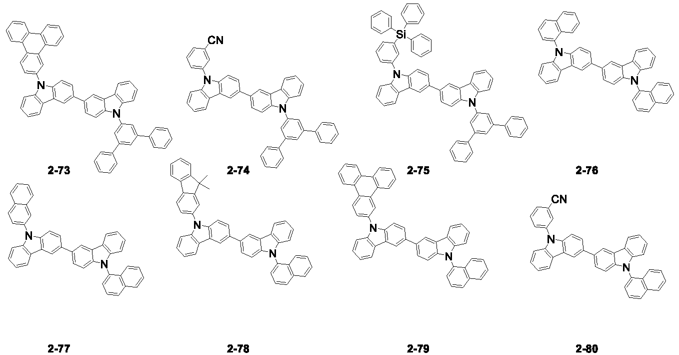

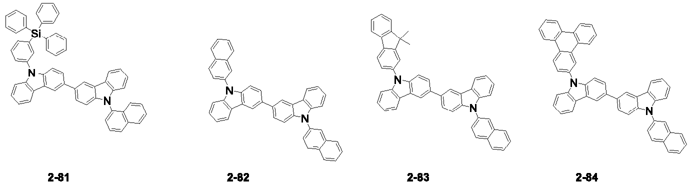

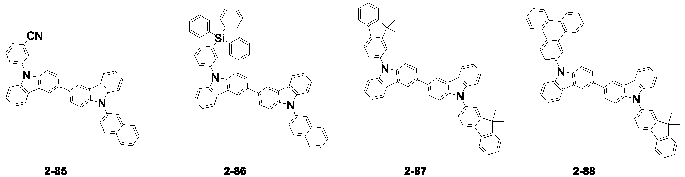

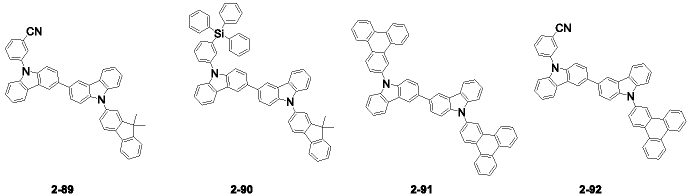

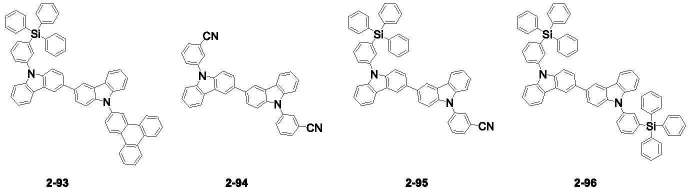

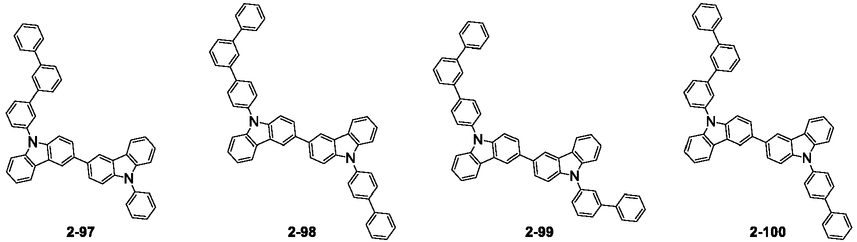

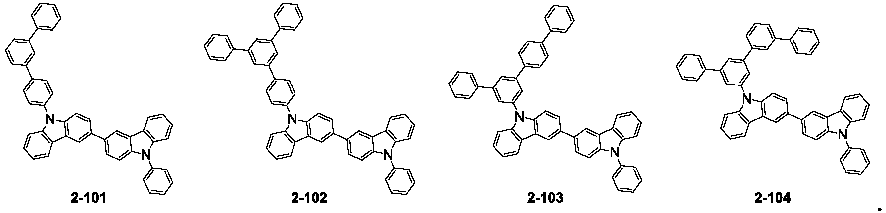

6. Zusammensetzung für eine organische Materialschicht einer organischen lichtemittierenden Vorrichtung, wobei die Zusammensetzung umfasst:
eine heterocyclische Verbindung, die durch die folgende chemische Formel 1 dargestellt ist; und
eine Verbindung, die durch die folgende chemische Formel 2 dargestellt ist, gleichzeitig: 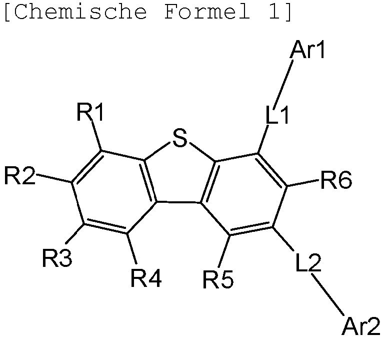 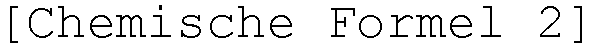 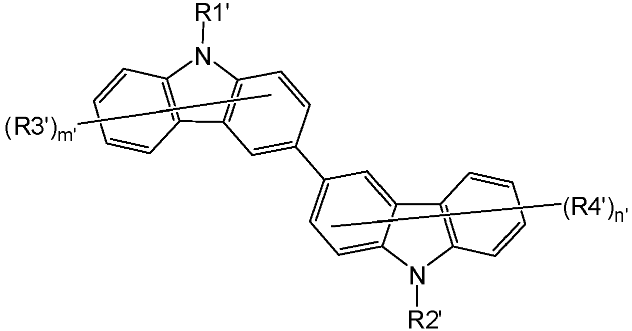
wobei in den chemischen Formeln 1 und 2
L1 und L2 gleich oder verschieden voneinander sind und jeweils unabhängig eine direkte Bindung oder eine substituierte oder unsubstituierte C₆ - bis C₆₀ -Arylengruppe sind;
Ar1 eine Pyridingruppe, unsubstituiert oder substituiert mit einer Phenylgruppe; eine Triazingruppe, unsubstituiert oder substituiert mit einem oder mehreren Substituenten ausgewählt aus der Gruppe bestehend aus einer Phenylgruppe, einer Biphenylgruppe, einer Dibenzothiophengruppe und einer Dimethylfluorengruppe; eine Chinolingruppe, unsubstituiert oder substituiert mit einer Phenylgruppe; eine Chinazolingruppe, unsubstituiert oder substituiert mit einer Phenylgruppe oder einer Biphenylgruppe; eine Phenanthrolingruppe; oder eine Benzimidazolgruppe, unsubstituiert oder substituiert mit einer Phenylgruppe, ist;
Ar2 durch eine der folgenden chemischen Formeln 3 und 4 dargestellt ist; 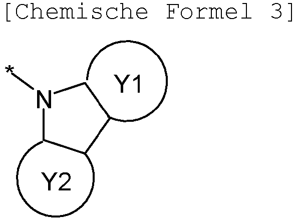 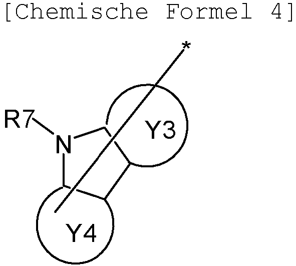
wobei Y1 bis Y4 gleich oder verschieden voneinander sind und jeweils unabhängig ein substituierter oder unsubstituierter aromatischer C₆- bis C₆₀-Kohlenwasserstoffring; oder ein substituierter oder unsubstituierter aromatischer C₂- bis C₆₀-Heteroring sind;
R1 bis R7, R3' und R4' gleich oder verschieden voneinander sind und jeweils unabhängig ausgewählt sind aus der Gruppe bestehend aus Wasserstoff; Deuterium; einer Halogengruppe; -CN; einer substituierten oder unsubstituierten C₁- bis C₆₀-Alkylgruppe; einer substituierten oder unsubstituierten C₂- bis C₆₀-Alkenylgruppe; einer substituierten oder unsubstituierten C₂-bis C₆₀-Alkinylgruppe; einer substituierten oder unsubstituierten C₁- bis C₆₀-Alkoxygruppe; einer substituierten oder unsubstituierten C₃- bis C₆₀-Cycloalkylgruppe; einer substituierten oder unsubstituierten C₂- bis C₆₀-Heterocycloalkylgruppe; einer substituierten oder unsubstituierten C₆- bis C₆₀-Arylgruppe; einer substituierten oder unsubstituierten C₂- bis C₆₀-Heteroarylgruppe; -SiRR'R"; - P(=O)RR'; und einer Amingruppe, unsubstituiert oder substituiert mit einer C₁- bis C₂₀-Alkylgruppe, einer substituierten oder unsubstituierten C₆- bis C₆₀-Arylgruppe oder einer C₂- bis C₆₀-Heteroarylgruppe, oder zwei oder mehr Gruppen, die aneinander angrenzen, aneinander binden, um einen substituierten oder unsubstituierten aliphatischen oder aromatischen Kohlenwasserstoffring zu bilden;
R1' durch -(L1')p'-(Ar1')q' dargestellt ist und R2' durch -(L2')r'-(Ar2')s' dargestellt ist;
L1' und L2' jeweils unabhängig eine direkte Bindung oder eine substituierte oder unsubstituierte C₆- bis C₆₀-Arylengruppe sind;
Ar1' und Ar2' jeweils unabhängig -CN; eine substituierte oder unsubstituierte C₁- bis C₆₀-Alkylgruppe; -SiRR'R"; eine substituierte oder unsubstituierte C₆- bis C₆₀-Arylgruppe sind,
wobei die chemische Formel 3 durch eine der folgenden Strukturformeln dargestellt ist: 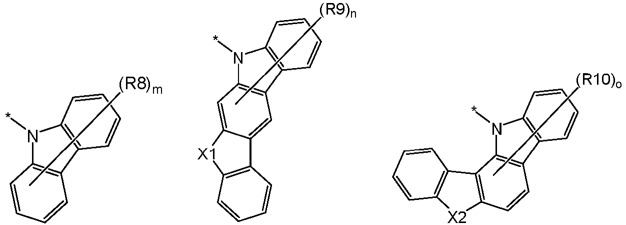 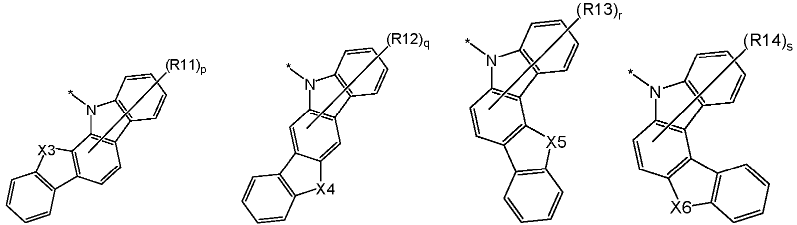
wobei in den Strukturformeln X1 bis X6 gleich oder verschieden voneinander und jeweils unabhängig NR, S, O oder CR'R" sind;
R8 bis R14 gleich oder verschieden voneinander sind und jeweils unabhängig ausgewählt sind aus der Gruppe bestehend aus Wasserstoff; Deuterium; einer Halogengruppe; -CN; einer substituierten oder unsubstituierten C₁- bis C₆₀-Alkylgruppe; einer substituierten oder unsubstituierten C₂- bis C₆₀-Alkenylgruppe; einer substituierten oder unsubstituierten C₂-bis C₆₀-Alkinylgruppe; einer substituierten oder unsubstituierten C₁- bis C₆₀-Alkoxygruppe; einer substituierten oder unsubstituierten C₃- bis C₆₀-Cycloalkylgruppe; einer substituierten oder unsubstituierten C₂- bis C₆₀-Heterocycloalkylgruppe; einer substituierten oder unsubstituierten C₆ - bis C₆₀-Arylgruppe; einer substituierten oder unsubstituierten C₂- bis C₆₀-Heteroarylgruppe; -SiRR'R"; - P(=O)RR'; und einer Amingruppe, unsubstituiert oder substituiert mit einer C₁- bis C₂₀-Alkylgruppe, einer substituierten oder unsubstituierten C₆- bis C₆₀-Arylgruppe oder einer C₂- bis C₆₀-Heteroarylgruppe, oder zwei oder mehr Gruppen, die aneinander angrenzen, aneinander binden, um einen substituierten oder unsubstituierten aliphatischen oder aromatischen Kohlenwasserstoffring zu bilden;
R, R' und R" gleich oder verschieden voneinander sind und jeweils unabhängig Wasserstoff; Deuterium; -CN; eine substituierte oder unsubstituierte C₁ - bis C₆₀-Alkylgruppe; eine substituierte oder unsubstituierte C₃- bis C₆₀-Cycloalkylgruppe; eine substituierte oder unsubstituierte C₆- bis C₆₀-Arylgruppe; oder eine substituierte oder unsubstituierte C₂- bis C₆₀-Heteroarylgruppe sind;
p' und r' jeweils unabhängig eine ganze Zahl von 0 bis 4 sind;
q' und s' jeweils unabhängig eine ganze Zahl von 1 bis 4 sind; und
m' und n' jeweils unabhängig eine ganze Zahl von 0 bis 7 sind,
m eine ganze Zahl von 0 bis 8 ist und n, o, p, q, r und s jeweils unabhängig eine ganze Zahl von 0 bis 6 sind.

7. Zusammensetzung für eine organische Materialschicht einer organischen lichtemittierenden Vorrichtung nach Anspruch 6, wobei ein Gewichtsverhältnis der heterocyclischen Verbindung, die durch die chemische Formel 1 dargestellt ist:der Verbindung, die durch die chemische Formel 2 dargestellt ist, in der Zusammensetzung von 1:10 bis 10:1 beträgt.

8. Zusammensetzung für eine organische Materialschicht einer organischen lichtemittierenden Vorrichtung nach Anspruch 6, wobei die chemische Formel 1 durch eine der folgenden chemischen Formeln 5 bis 10 dargestellt ist: 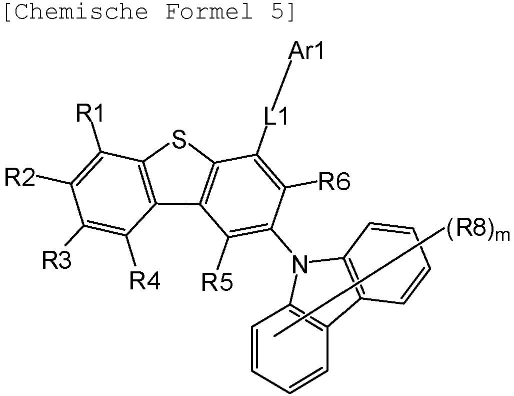 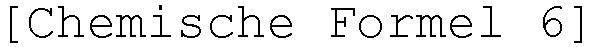 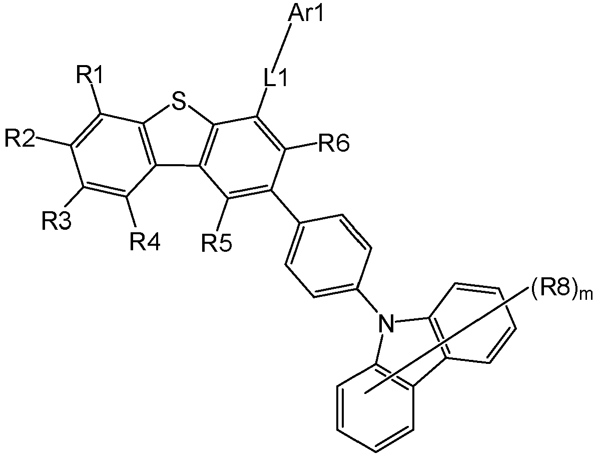 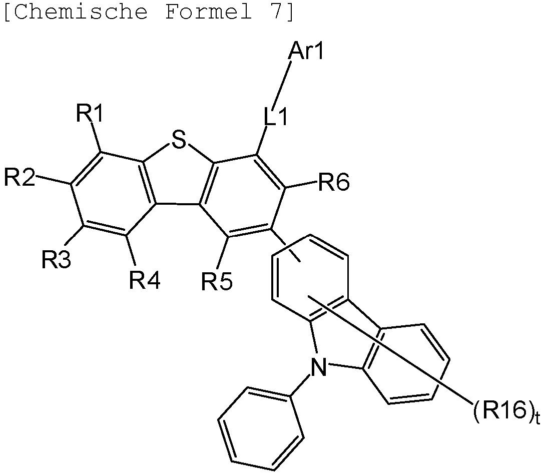 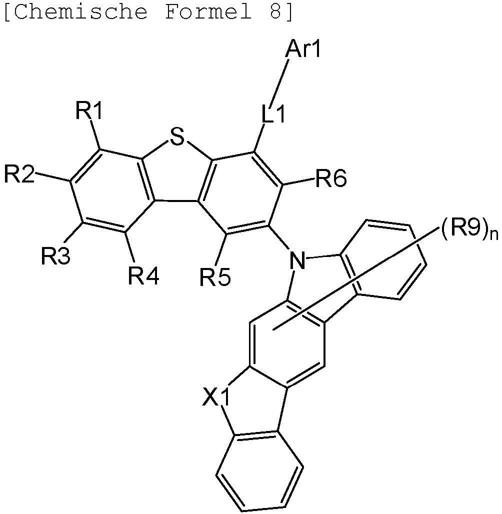 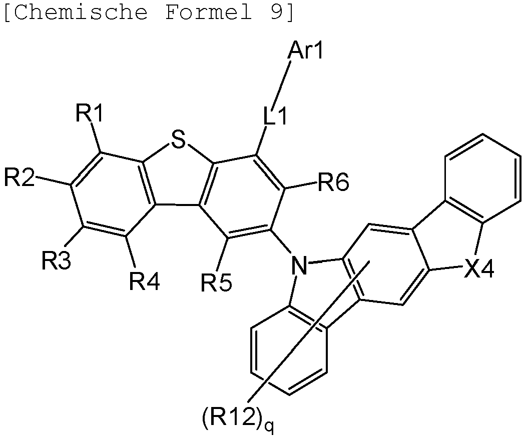 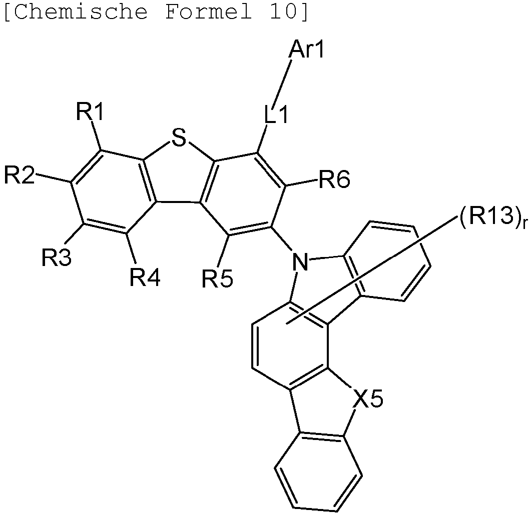 wobei in den Chemischen Formeln 5 bis 10
R1 bis R6, L1 und Ar1 die gleichen Definitionen wie in der Chemischen Formel 1 haben;
X1, X4 und X5 gleich oder verschieden voneinander und jeweils unabhängig NR, S, O oder CR'R" sind;
R8, R9, R12, R13 und R16 gleich oder verschieden voneinander sind und jeweils unabhängig ausgewählt sind aus der Gruppe bestehend aus Wasserstoff; Deuterium; einer Halogengruppe; -CN; einer substituierten oder unsubstituierten C₁- bis C₆₀-Alkylgruppe; einer substituierten oder unsubstituierten C₂- bis C₆₀-Alkenylgruppe; einer substituierten oder unsubstituierten C₂- bis C₆₀-Alkinylgruppe; einer substituierten oder unsubstituierten C₁- bis C₆₀-Alkoxygruppe; einer substituierten oder unsubstituierten C₃- bis C₆₀-Cycloalkylgruppe; einer substituierten oder unsubstituierten C₂-bis C₆₀-Heterocycloalkylgruppe; einer substituierten oder unsubstituierten C₆- bis C₆₀-Arylgruppe; einer substituierten oder unsubstituierten C₂- bis C₆₀-Heteroarylgruppe; -SiRR'R"; - P(=O)RR'; und einer Amingruppe, unsubstituiert oder substituiert mit einer C₁- bis C₂₀-Alkylgruppe, einer substituierten oder unsubstituierten C₆- bis C₆₀-Arylgruppe oder einer C₂- bis C₆₀-Heteroarylgruppe, oder zwei oder mehr Gruppen, die aneinander angrenzen, aneinander binden, um einen substituierten oder unsubstituierten aliphatischen oder aromatischen Kohlenwasserstoffring zu bilden;
R, R' und R" gleich oder verschieden voneinander sind und jeweils unabhängig Wasserstoff; Deuterium; -CN; eine substituierte oder unsubstituierte C₁- bis C₆₀-Alkylgruppe; eine substituierte oder unsubstituierte C₃- bis C₆₀-Cycloalkylgruppe; eine substituierte oder unsubstituierte C₆- bis C₆₀-Arylgruppe; oder eine substituierte oder unsubstituierte C₂- bis C₆₀-Heteroarylgruppe sind; und
m eine ganze Zahl von 0 bis 8 ist, n, q und r jeweils unabhängig eine ganze Zahl von 0 bis 6 sind und t eine ganze Zahl von 0 bis 7 ist.

## Revendications

1. Dispositif électroluminescent organique comprenant :
une anode ;
une cathode ; et
une couche électroluminescente fournie entre l'anode et la cathode,
dans lequel une couche électroluminescente comprend un composé hétérocyclique représenté par la Formule chimique 1 ci-après et un composé représenté par la Formule chimique 2 ci-après en même temps : 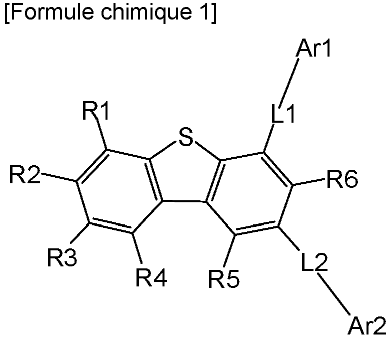 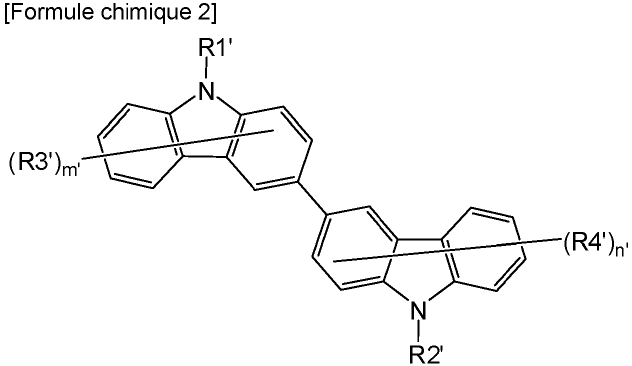
dans les Formules chimiques 1 et 2,
L1 et L2 sont identiques ou différents l'un de l'autre, et chacun indépendamment une liaison directe, ou un groupe arylène en C₆ à C₆₀ non substitué ou substitué ;
Ar1 est un groupe pyridine non substitué ou substitué ; un groupe triazine non substitué ou substitué ; un groupe quinoline non substitué ou substitué ; un groupe quinazoline non substitué ou substitué ; un groupe phénanthroline non substitué ou substitué ; ou un groupe benzimidazole non substitué ou substitué,
Ar2 est représenté par l'une des Formules chimiques 3 et 4 ci-après ; 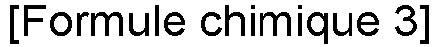 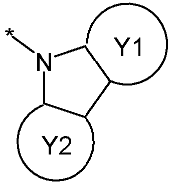 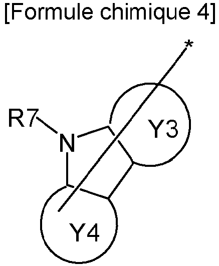
Y1 à Y4 sont identiques ou différents l'un de l'autre, et chacun indépendamment un cycle hydrocarboné aromatique en C₆ à C₆₀ en ; ou un hétérocycle aromatique en C₂ à C₆₀ en ;
R1 à R7, R3' et R4' sont identiques ou différents l'un de l'autre, et chacun indépendamment sélectionnés dans le groupe constitué de un hydrogène ; un deutérium ; un groupe halogène ; -CN ; un groupe alkyle en C₁ à C₆₀ non substitué ou substitué ; un groupe alkényle en C₂ à C₆₀ non substitué ou substitué ; un groupe alkynyle en C₂ à C₆₀ non substitué ou substitué ; un groupe alkoxy en C₁ à C₆₀ non substitué ou substitué ; un groupe cycloalkyle en C₃ à C₆₀ ; un groupe hétérocycloalkyle C₂ à C₆₀ ; un groupe aryle en C₆ à C₆₀ ; un groupe hétéroaryle en C₂ à C₆₀ ; -SiRR'R" ; -P(=O)RR' ; et un groupe amino non substitué ou substitué par un groupe alkyle en C₁ à C₂₀, un groupe aryle en C₆ à C₆₀, ou un groupe hétéroaryle C₂ à C₆₀, ou deux groupes ou plus adjacents les uns aux autres se lient les uns aux autres pour former un cycle hydrocarboné aromatique ou aliphatique non substitué ou substitué ;
R1' est représenté par -(L1')p'-(Ar1')q', et R2' est représenté par -(L2')r'-(Ar2')s' ;
L1' et L2' sont chacun indépendamment une liaison directe, ou un groupe arylène en C₆ à C₆₀ non substitué ou substitué ;
Ar1' et Ar2' sont chacun indépendamment -CN ; un groupe alkyle en C₁ à C₆₀ ; - SiRR'R" ; un groupe aryle en C₆ à C₆₀ non substitué ou substitué,
dans lequel la Formule chimique 3 est représentée par l'une quelconque des formules structurelles ci-après : 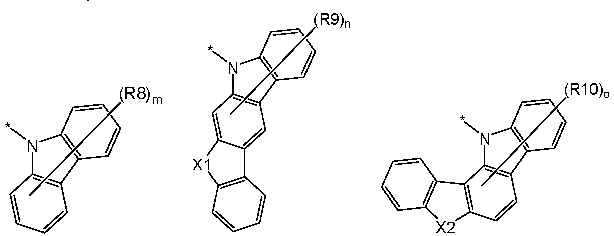 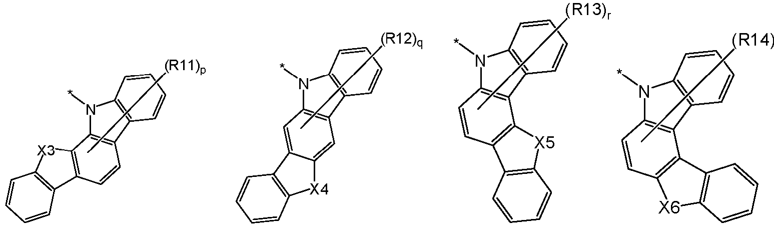
dans les formules structurelles, X1 à X6 sont identiques ou différents l'un de l'autre, et chacun indépendamment NR, S, O ou CR'R" ;
R8 à R14 sont identiques ou différents l'un de l'autre, et chacun indépendamment sélectionnés dans le groupe constitué de un hydrogène ; un deutérium ; un groupe halogène ; -CN ; un groupe alkyle en C₁ à C₆₀ non substitué ou substitué ; un groupe alkényle C₂ à C₆₀ non substitué ou substitué ; un groupe alkynyle C₂ à C₆₀ non substitué ou substitué ; un groupe alkoxy en C₁ à C₆₀ non substitué ou substitué ; un groupe cycloalkyle en C₃ à C₆₀ non substitué ou substitué ; un groupe hétérocycloalkyle C₂ à C₆₀ non substitué ou substitué ; un groupe aryle en C₆ à C₆₀ non substitué ou substitué ; un groupe hétéroaryle en C₆ à C₆₀ non substitué ou substitué ; -SiRR'R" ; -P(=O)RR' ; et un groupe amino non substitué ou substitué par un groupe alkyle en C₁ à C₂₀, un groupe aryle en C₆ à C₆₀, ou un groupe hétéroaryle en C₆ à C₆₀, ou deux groupes ou plus adjacents les uns aux autres se lient les uns aux autres pour former un cycle hydrocarboné aromatique ou aliphatique non substitué ou substitué ;
R, R' et R" sont identiques ou différents l'un de l'autre, et chacun indépendamment un hydrogène ; un deutérium ; -CN ; un groupe alkyle en C₁ à C₆₀ non substitué ou substitué ; un groupe cycloalkyle en C₃ à C₆₀ non substitué ou substitué ; un groupe aryle en C₆ à C₆₀ non substitué ou substitué ; ou un groupe hétéroaryle en C₂ à C₆₀ non substitué ou substitué ;
p' et r' sont chacun indépendamment un nombre entier de 0 à 4 ;
q' et s' sont chacun indépendamment un nombre entier de 1 à 4 ; et
m' et n' sont chacun indépendamment un nombre entier de 0 à 7,
m est un nombre entier de 0 à 8, et n, o, p, q, r et s sont chacun indépendamment un nombre entier de 0 à 6.

2. Dispositif électroluminescent organique selon la revendication 1, dans lequel la Formule chimique 4 est représentée par l'une quelconque des formules structurelles ci-après : 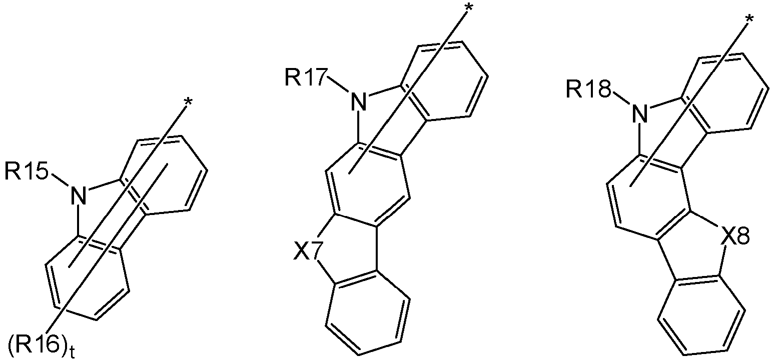
dans les formules structurelles, X7 et X8 sont identiques ou différents l'un de l'autre, et chacun indépendamment NR, S, O ou CR'R" ;
R15 à R18 sont identiques ou différents l'un de l'autre, et chacun indépendamment sélectionnés dans le groupe constitué de un hydrogène ; un deutérium ; un groupe halogène ; -CN ; un groupe alkyle en C₁ à C₆₀ non substitué ou substitué ; un groupe alkényle en C₂ à C₆₀ non substitué ou substitué ; un groupe alkynyle en C₂ à C₆₀ non substitué ou substitué ; un groupe alkoxy en C₁ à C₆₀ non substitué ou substitué ; un groupe cycloalkyle en C₃ à C₆₀ non substitué ou substitué ; un groupe hétérocycloalkyle en C₂ à C₆₀ non substitué ou substitué ; un groupe aryle en C₆ à C₆₀ non substitué ou substitué ; un groupe hétéroaryle en C₂ à C₆₀ non substitué ou substitué ; -SiRR'R" ; -P(=O)RR' ; et un groupe amino non substitué ou substitué par un groupe alkyle en C₁ à C₂₀, un groupe aryle en C₆ à C₆₀ non substitué ou substitué, ou un groupe hétéroaryle en C₂ à C₆₀, ou deux groupes ou plus adjacents les uns aux autres se lient les uns aux autres pour former un cycle hydrocarboné aromatique ou aliphatique non substitué ou substitué ;
R, R' et R" sont identiques ou différents l'un de l'autre, et chacun indépendamment un hydrogène ; un deutérium ; -CN ; un groupe alkyle en C₁ à C₆₀ non substitué ou substitué ; un groupe cycloalkyle en C₃ à C₆₀ non substitué ou substitué ; un groupe aryle en C₆ à C₆₀ non substitué ou substitué ; ou un groupe hétéroaryle en C₂ à C₆₀ non substitué ou substitué ; et
t est un nombre entier de 0 à 7.

3. Dispositif électroluminescent organique selon la revendication 1, dans lequel la Formule chimique 1 est représentée par l'une des Formules chimiques 5 à 10 ci-après : 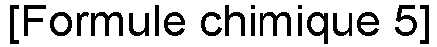 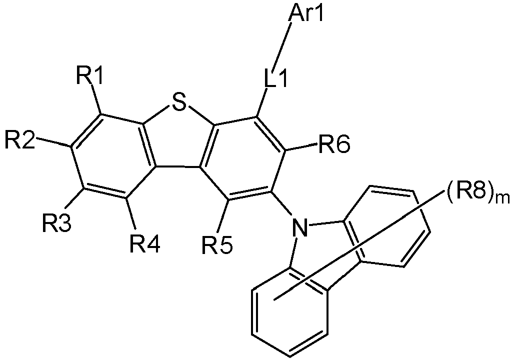 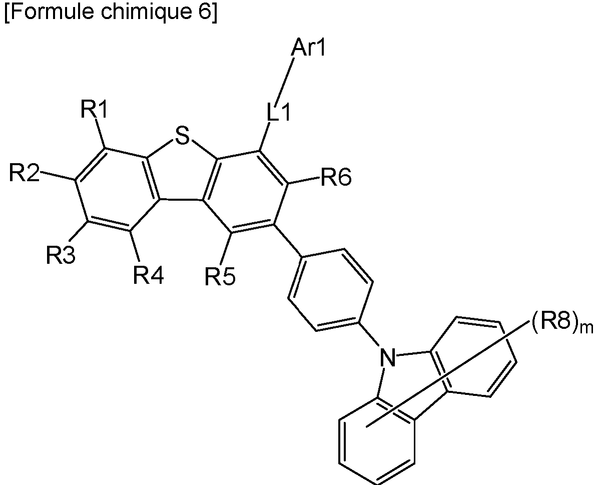 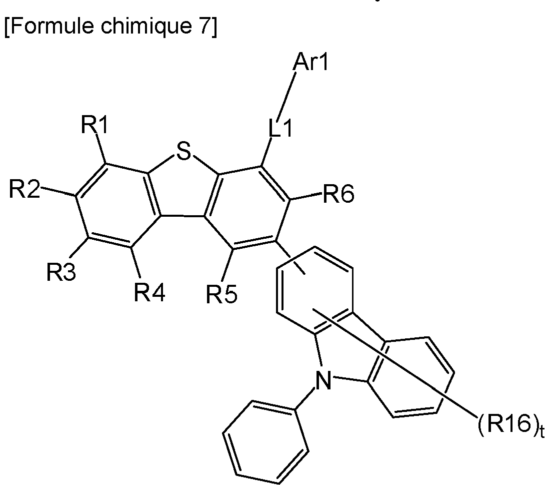 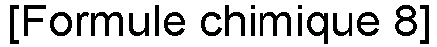 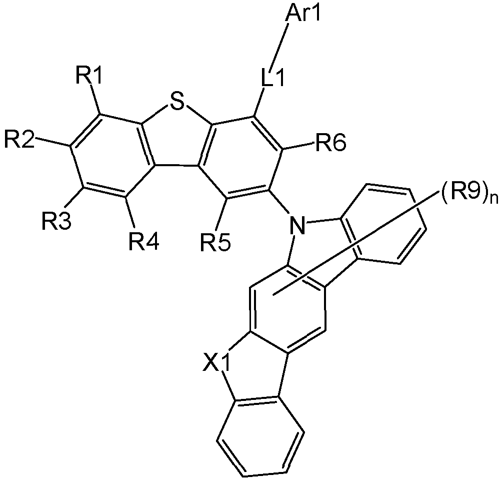 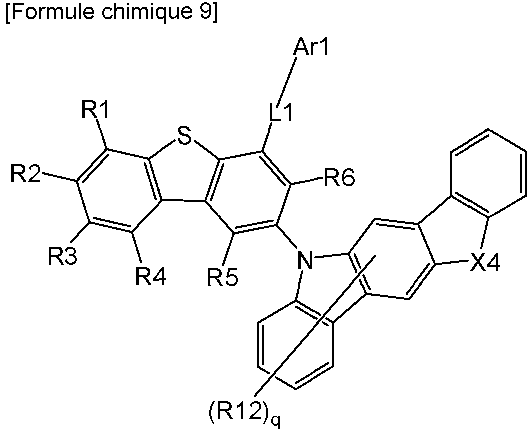 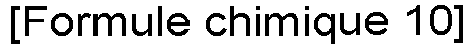 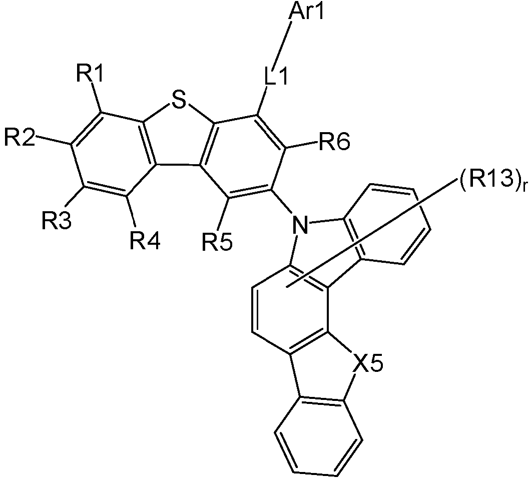 dans les Formules chimiques 5 à 10,
R1 à R6, L1 et Ar1 ont les mêmes définitions que dans la Formule chimique 1 ;
X1, X4 et X5 sont identiques ou différents l'un de l'autre, et chacun indépendamment NR, S, O ou CR'R" ;
R8, R9, R12, R13 et R16 sont identiques ou différents l'un de l'autre, et chacun indépendamment sélectionnés dans le groupe constitué de un hydrogène ; un deutérium ; un groupe halogène ; -CN ; un groupe alkyle en C₁ à C₆₀ non substitué ou substitué ; un groupe alkényle en C₂ à C₆₀ non substitué ou substitué ; un groupe alkynyle en C₂ à C₆₀ non substitué ou substitué ; un groupe alkoxy en C₁ à C₆₀ non substitué ou substitué ; un groupe cycloalkyle en C₃ à C₆₀ non substitué ou substitué ; un groupe hétérocycloalkyle en C₂ à C₆₀ non substitué ou substitué ; un groupe aryle en C₆ à C₆₀ non substitué ou substitué ; un groupe hétéroaryle en C₂ à C₆₀ ; -SiRR'R" ; -P(=O)RR' ; et un groupe amino non substitué ou substitué par un groupe alkyle en C₁ à C₂₀, un groupe aryle en C₆ à C₆₀ non substitué ou substitué, ou un groupe hétéroaryle en C₂ à C₆₀ non substitué ou substitué, ou deux groupes ou plus adjacents les uns aux autres se lient les uns aux autres pour former un cycle hydrocarboné aromatique ou aliphatique non substitué ou substitué ;
R, R' et R" sont identiques ou différents l'un de l'autre, et chacun indépendamment un hydrogène ; un deutérium ; -CN ; un groupe alkyle en C₁ à C₆₀ non substitué ou substitué ; un groupe cycloalkyle en C₃ à C₆₀ non substitué ou substitué ; un groupe aryle en C₆ à C₆₀ non substitué ou substitué ; ou un groupe hétéroaryle en C₂ à C₆₀ non substitué ou substitué ; et
m est un nombre entier de 0 à 8, n, q et r sont chacun indépendamment un nombre entier de 0 à 6 et t est un nombre entier de 0 à 7.

4. Dispositif électroluminescent organique selon la revendication 1, dans lequel la Formule chimique 1 est représentée par l'un quelconque des composés suivants :

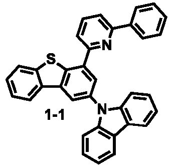

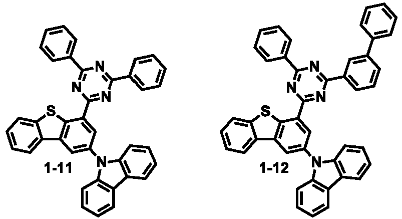

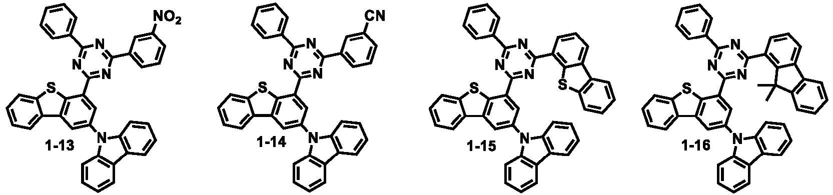

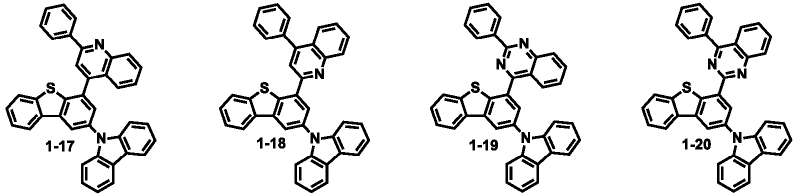

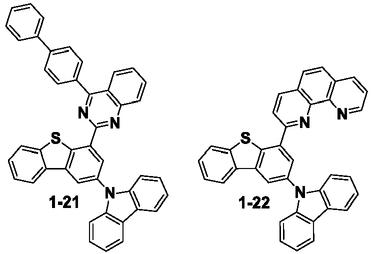

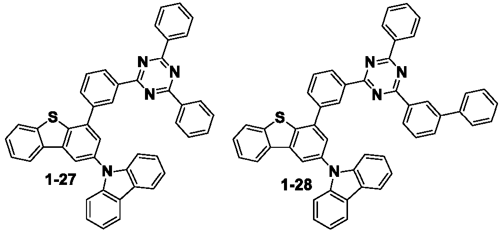

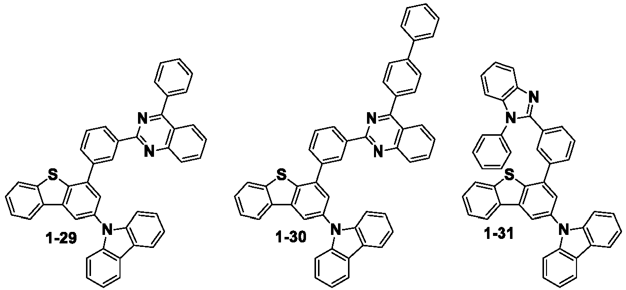

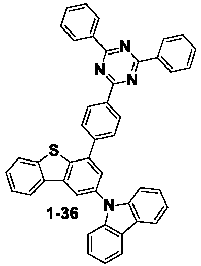

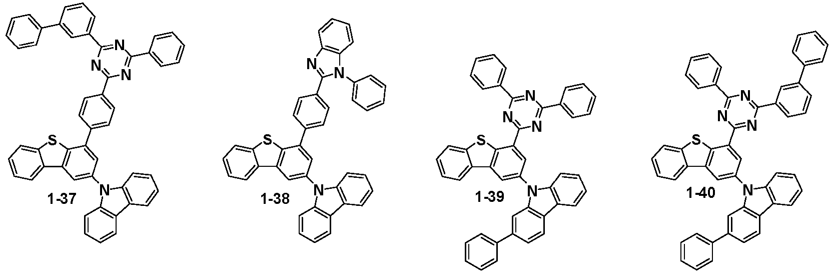

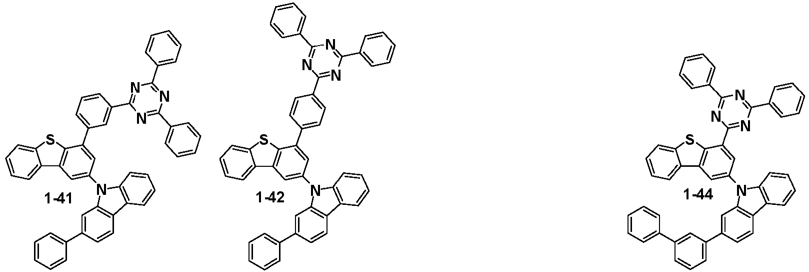

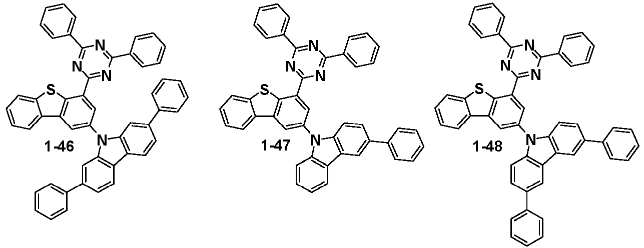

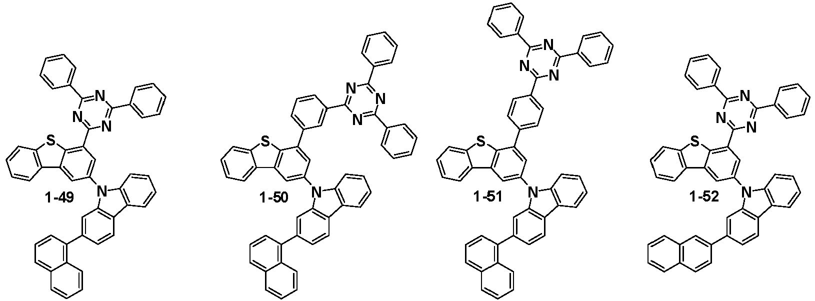

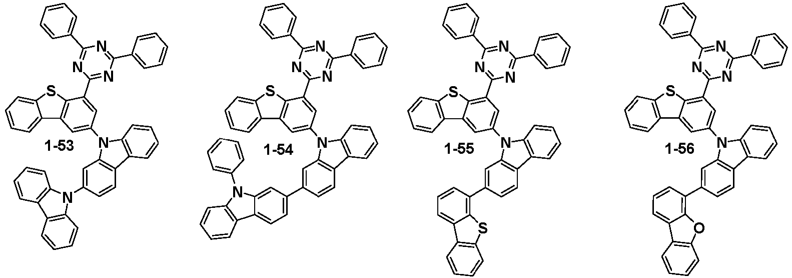

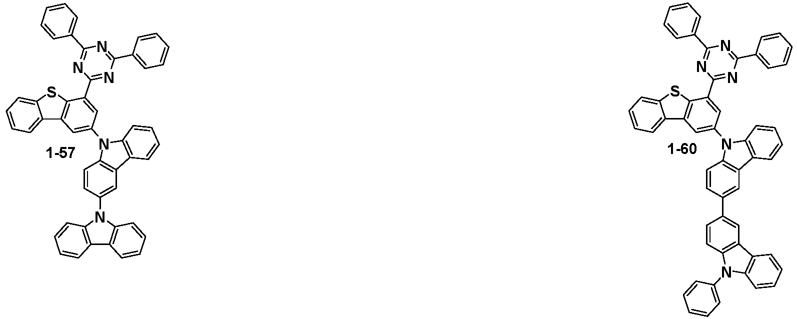

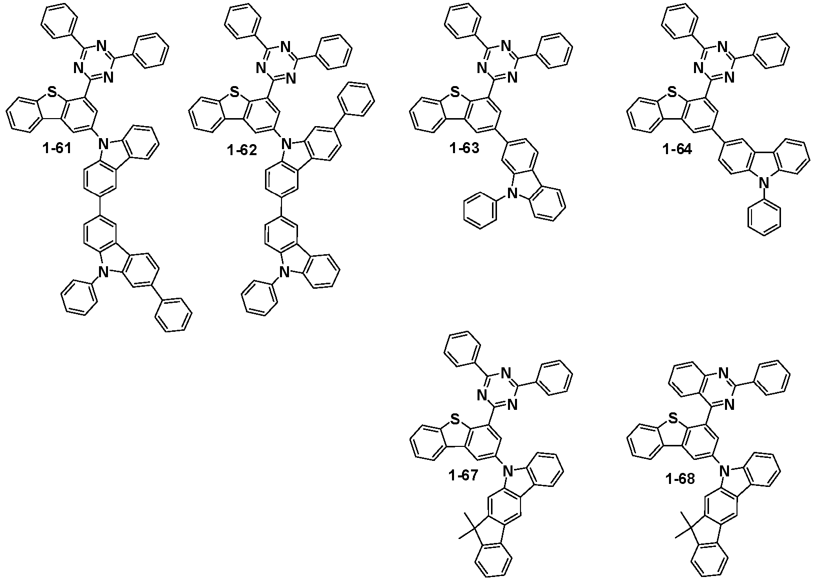

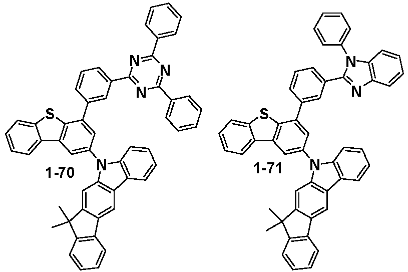

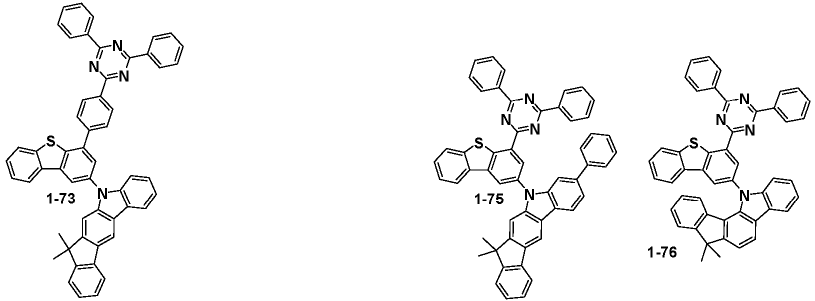

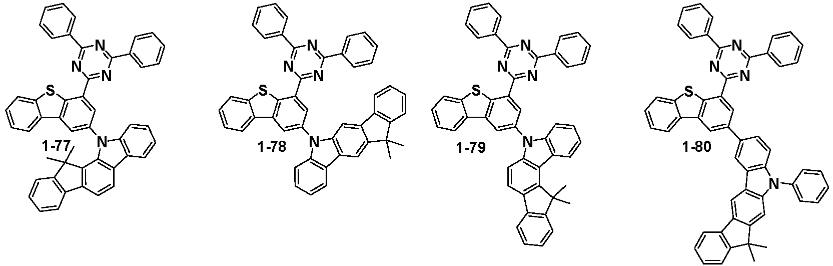

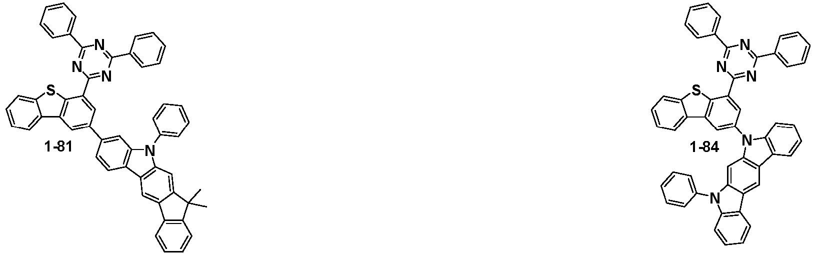

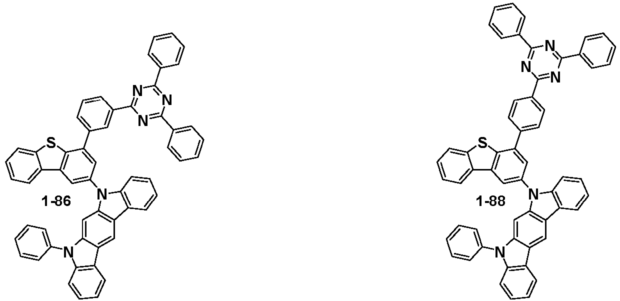

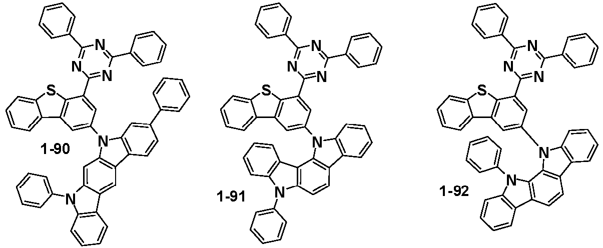

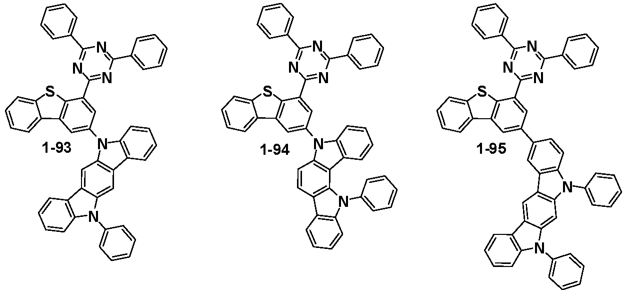

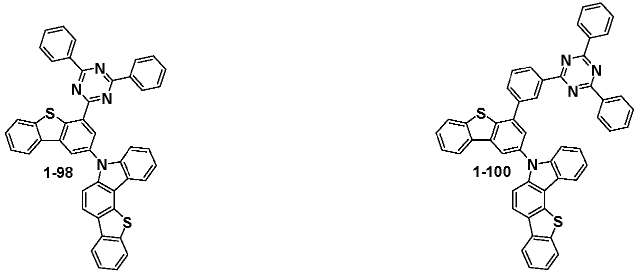

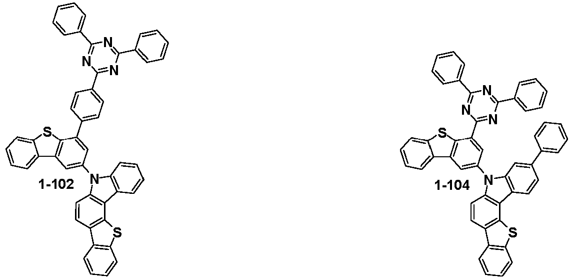

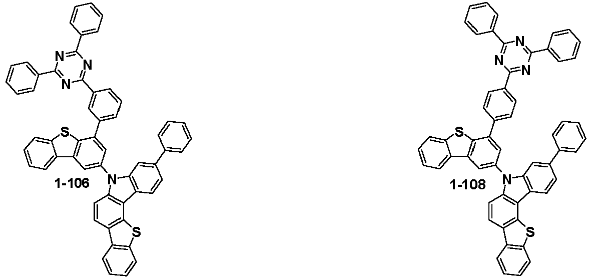

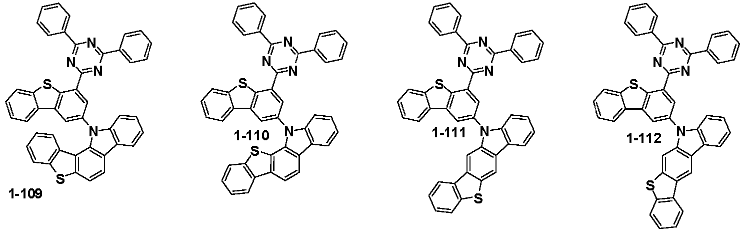

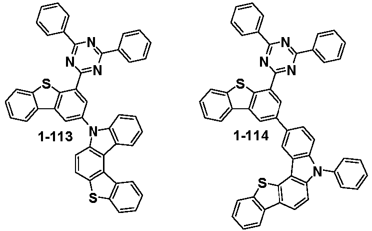

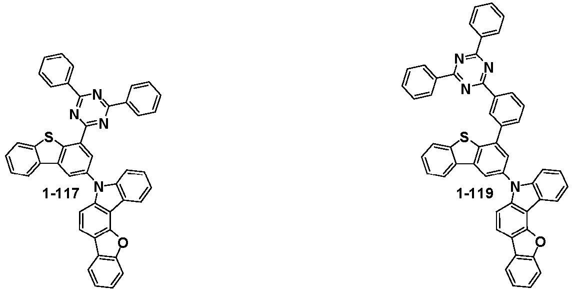

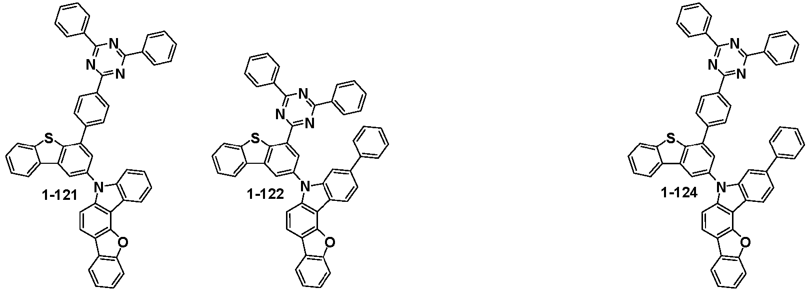

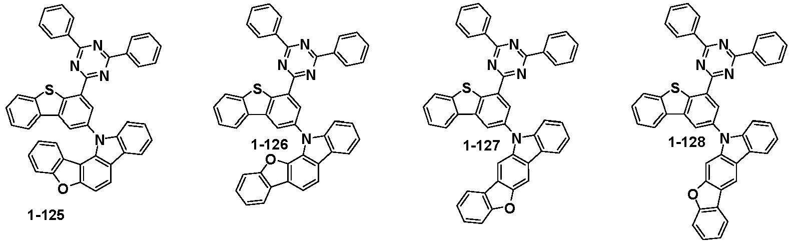

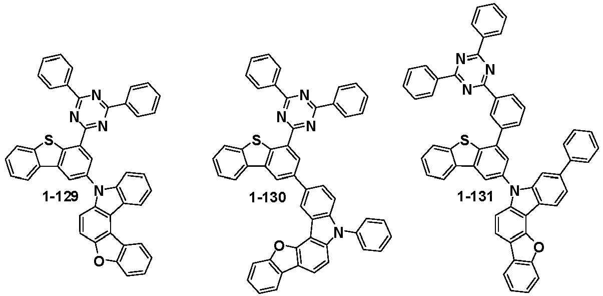

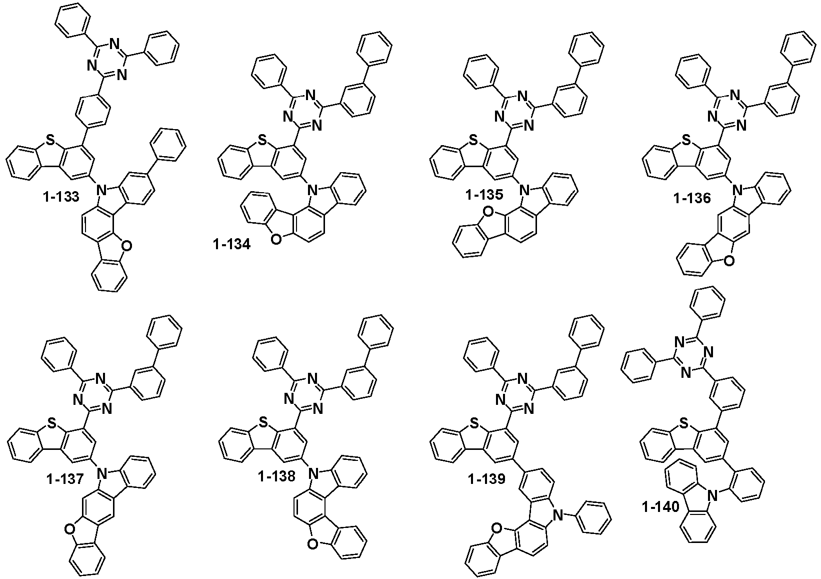

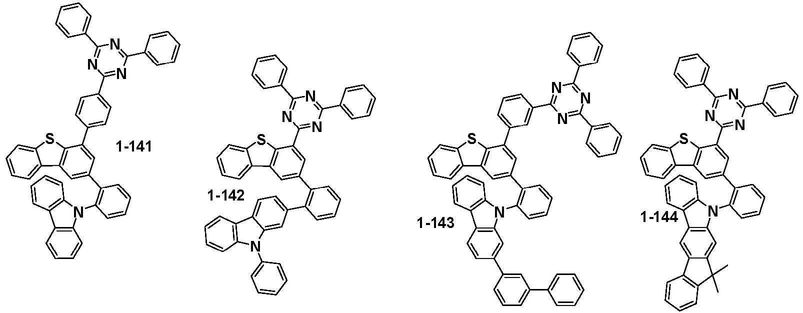

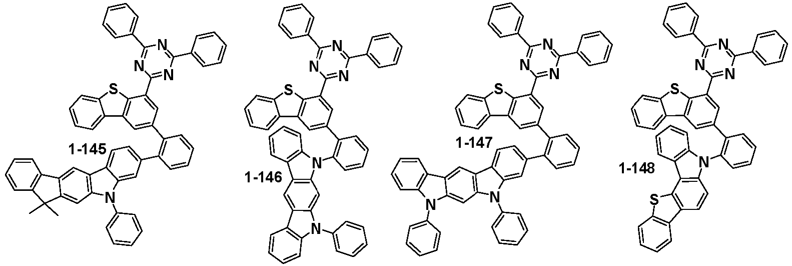

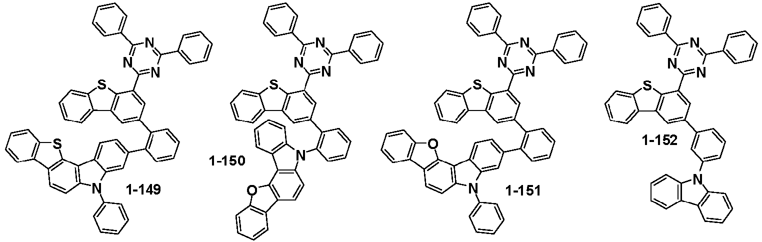

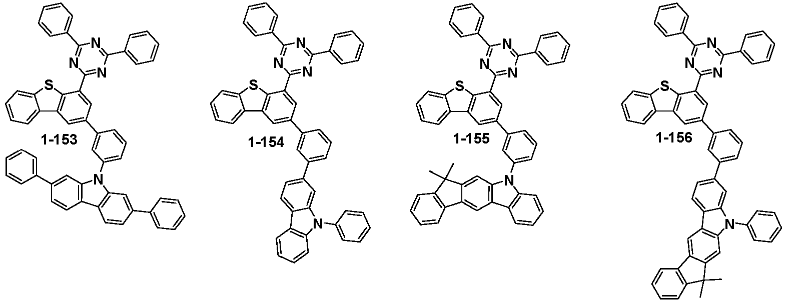

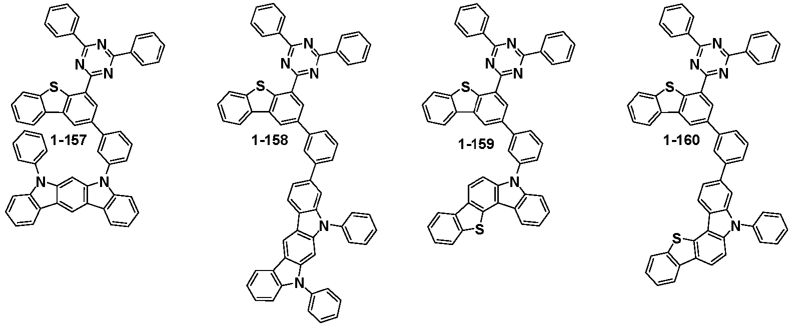

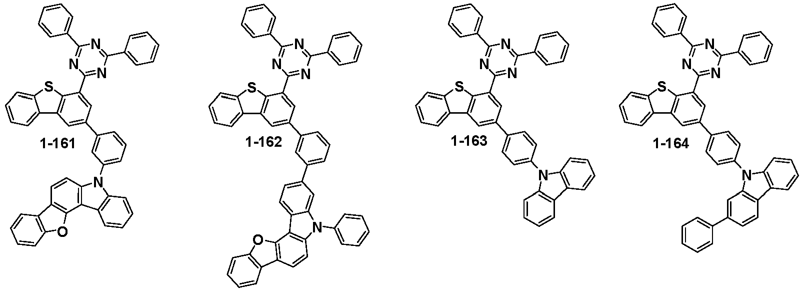

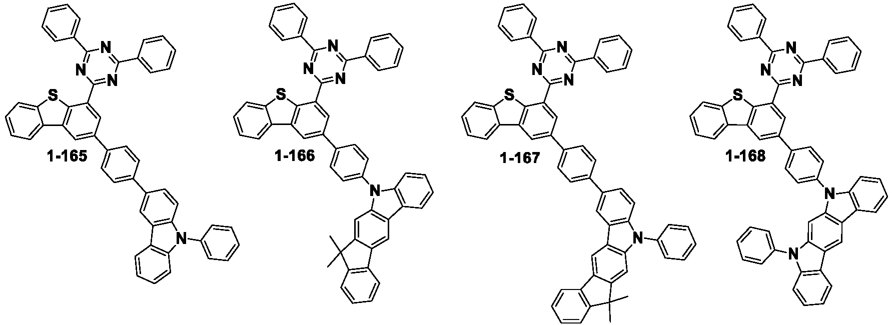

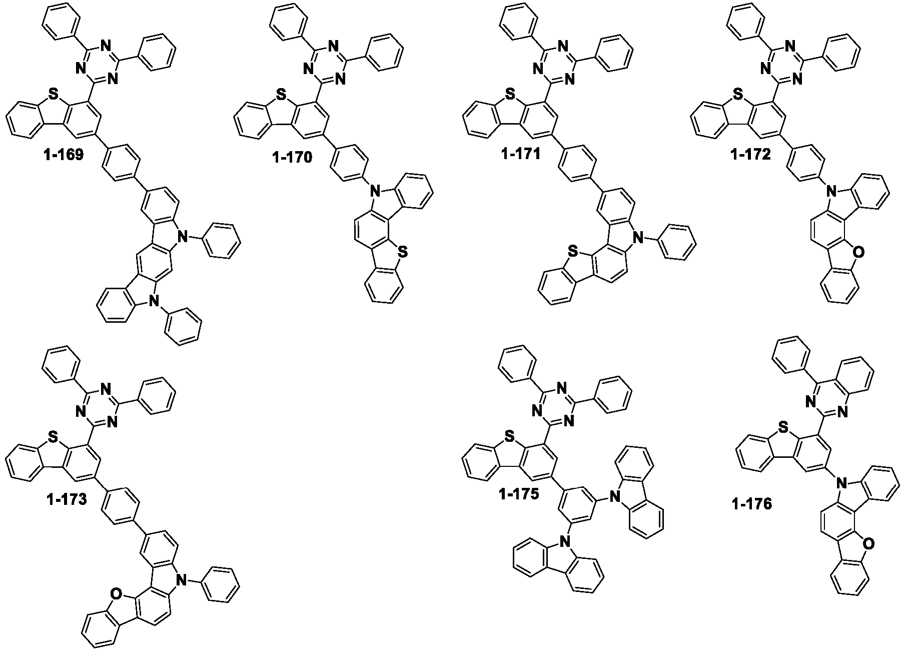

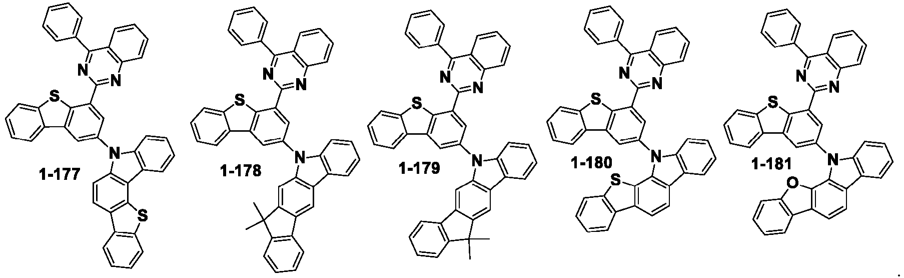

5. Dispositif électroluminescent organique selon la revendication 1, dans lequel la Formule chimique 2 est représentée par l'un quelconque des composés suivants :

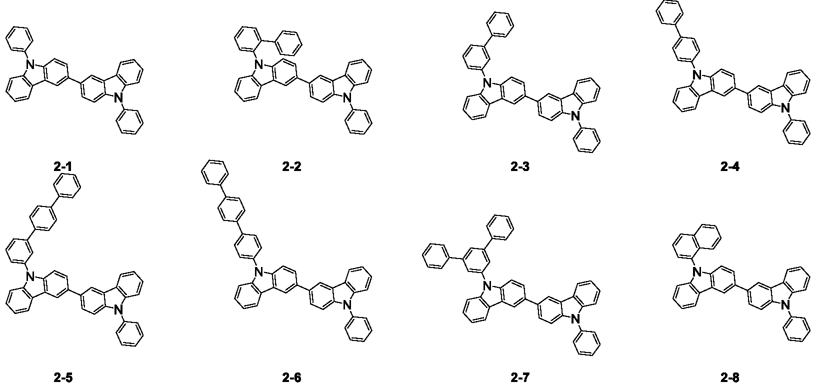

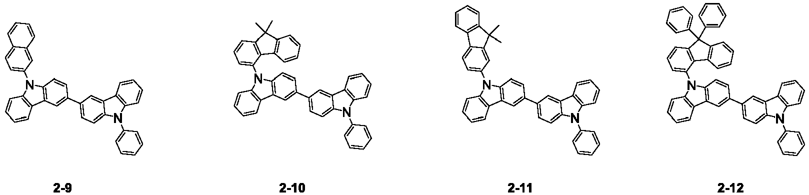

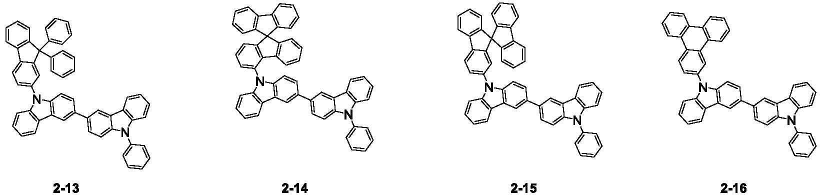

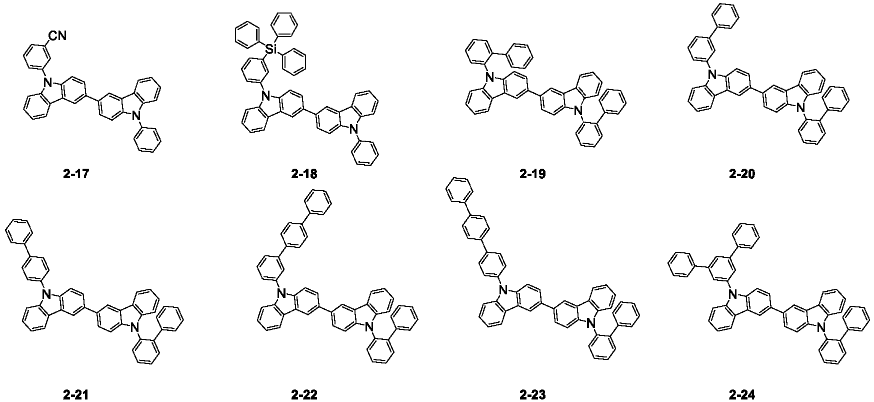

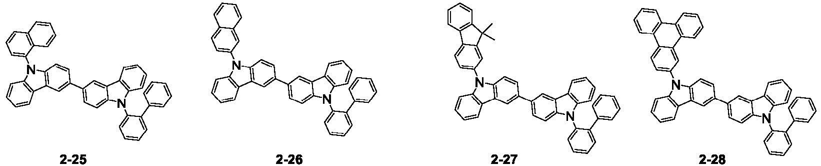

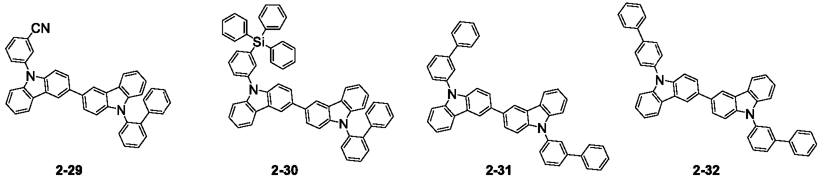

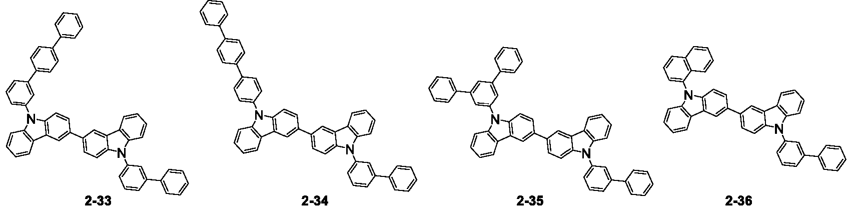

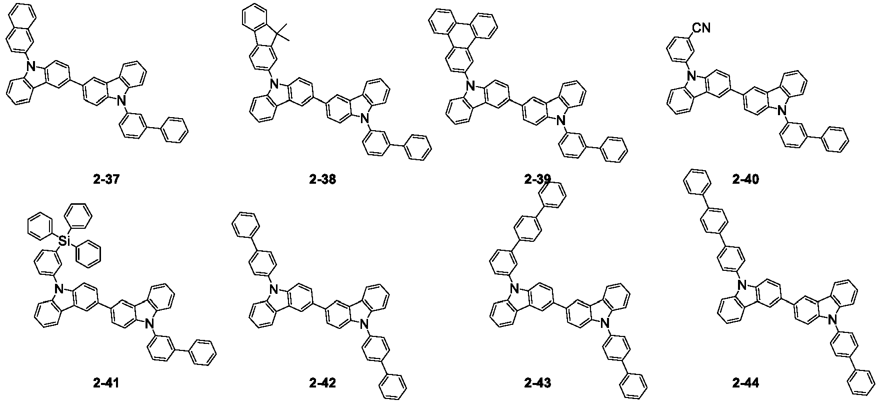

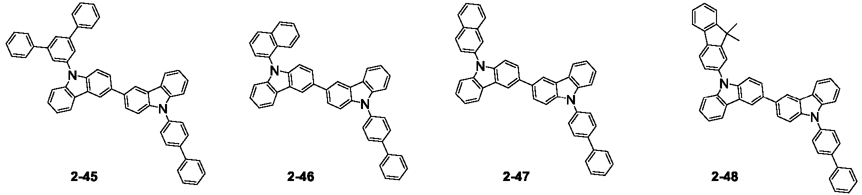

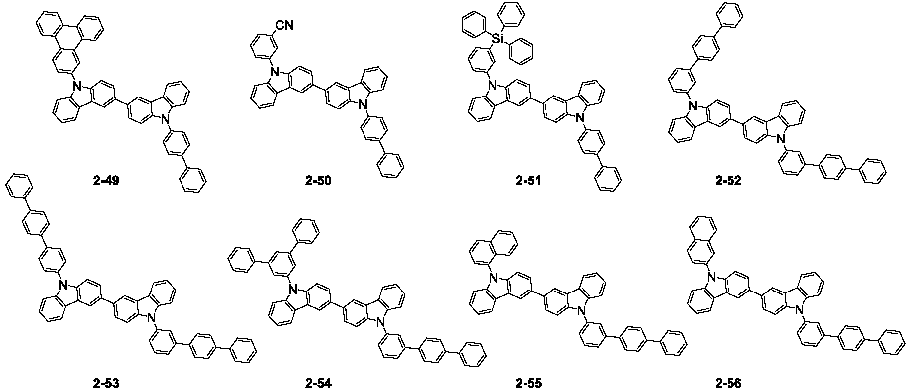

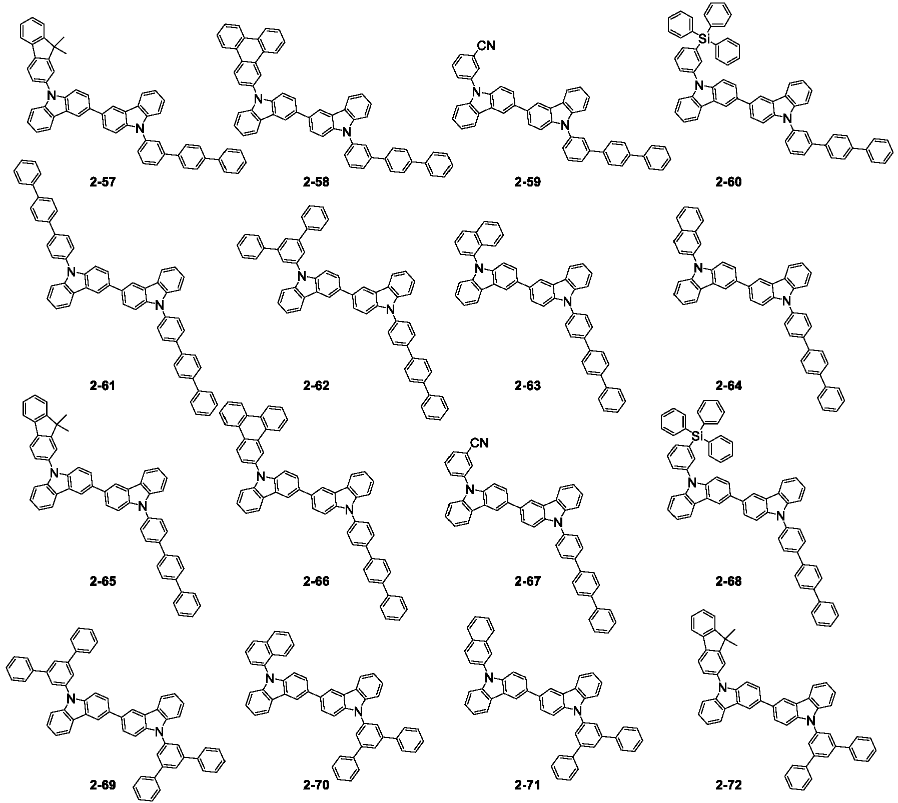

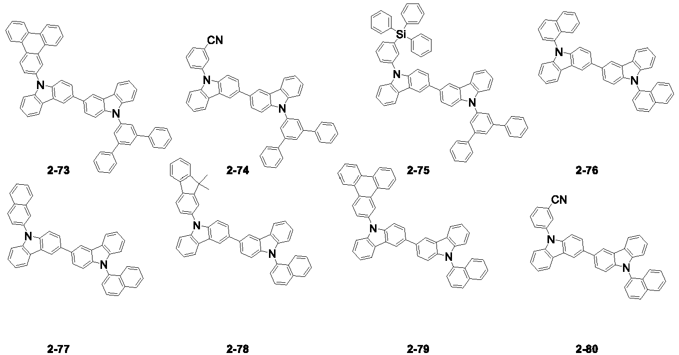

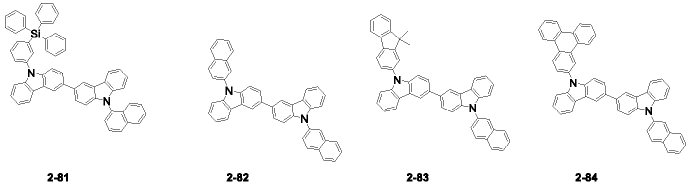

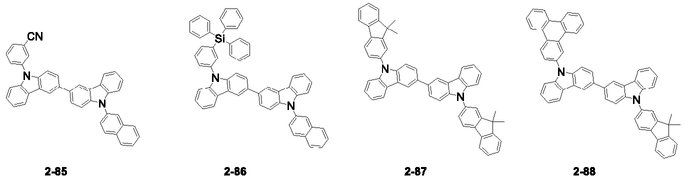

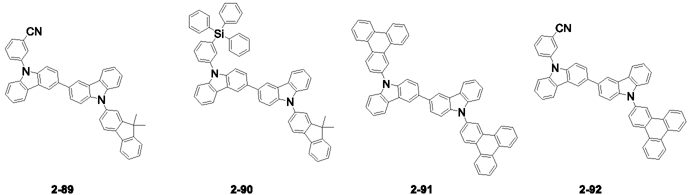

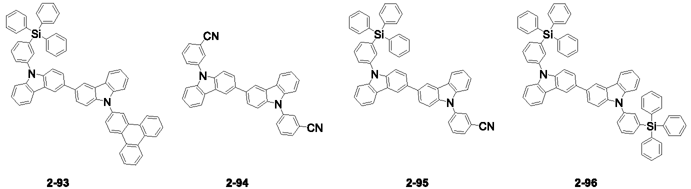

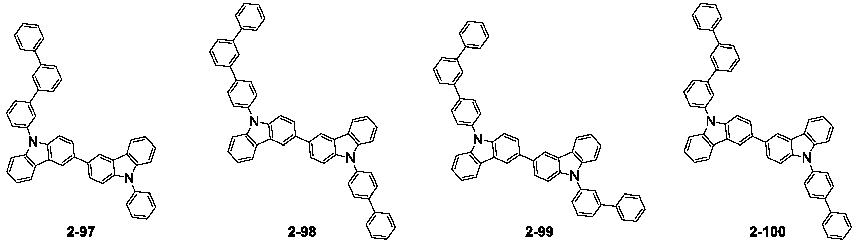

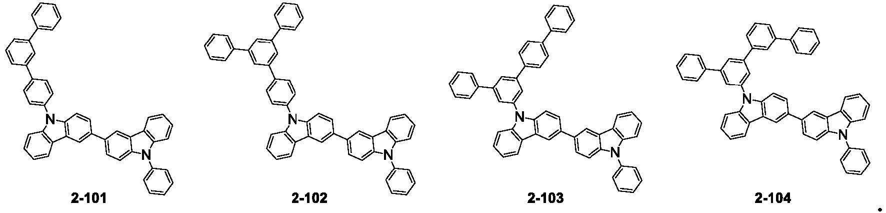

6. Composition pour une couche de matériau organique d'un dispositif électroluminescent organique, la composition comprenant :
un composé hétérocyclique représenté par la Formule chimique 1 ci-après ; et
un composé représenté par la Formule chimique 2 ci-après en même temps : 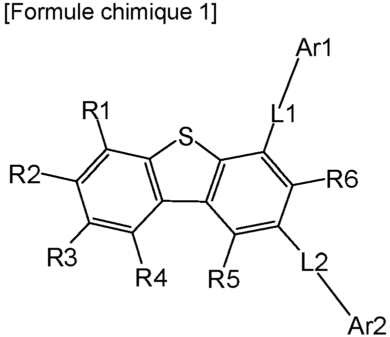 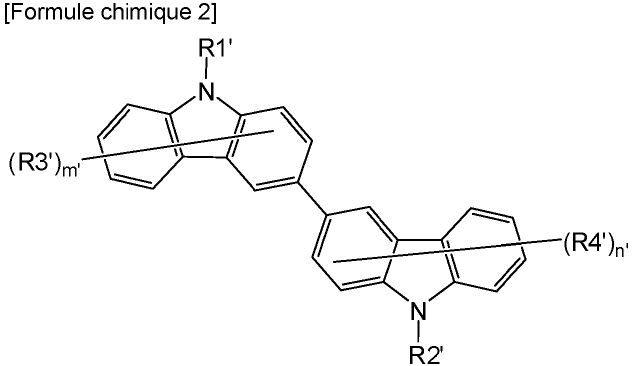
dans les Formules chimiques 1 et 2,
L1 et L2 sont identiques ou différents l'un de l'autre, et chacun indépendamment une liaison directe, ou un groupe arylène en C₆ à C₆₀ non substitué ou substitué ;
Ar1 est un groupe pyridine non substitué ou substitué ; un groupe triazine non substitué ou substitué ; un groupe quinoline non substitué ou substitué ; un groupe quinazoline non substitué ou substitué ; un groupe phénanthroline non substitué ou substitué ; ou un groupe benzimidazole non substitué ou substitué,
Ar2 est représenté par l'une des Formules chimiques 3 et 4 ci-après ; 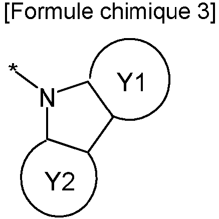 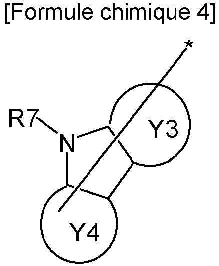
Y1 à Y4 sont identiques ou différents l'un de l'autre, et chacun indépendamment un cycle hydrocarboné aromatique en C₆ à C₆₀ non substitué ou substitué ; ou un hétérocycle aromatique en C₂ à C₆₀ non substitué ou substitué ;
R1 à R7, R3' et R4' sont identiques ou différents l'un de l'autre, et chacun indépendamment sélectionnés dans le groupe constitué de un hydrogène ; un deutérium ; un groupe halogène ; -CN ; un groupe alkyle en C₁ à C₆₀ non substitué ou substitué ; un groupe alkényle en C₂ à C₆₀ non substitué ou substitué ; un groupe alkynyle en C₂ à C₆₀ non substitué ou substitué ; un groupe alkoxy en C₁ à C₆₀ non substitué ou substitué ; un groupe cycloalkyle en C₃ à C₆₀ ; un groupe hétérocycloalkyle en C₂ à C₆₀ non substitué ou substitué ; un groupe aryle en C₆ à C₆₀ non substitué ou substitué ; un groupe hétéroaryle en C₂ à C₆₀ non substitué ou substitué ; -SiRR'R" ; -P(=O)RR' ; et un groupe amino non substitué ou substitué par un groupe alkyle en C₁ à C₂₀, un groupe aryle en C₆ à C₆₀ non substitué ou substitué, ou un groupe hétéroaryle en C₂ à C₆₀, ou deux groupes ou plus adjacents les uns aux autres se lient les uns aux autres pour former un cycle hydrocarboné aromatique ou aliphatique non substitué ou substitué ;
R1' est représenté par -(L1')p'-(Ar1')q', et R2' est représenté par -(L2')r'-(Ar2')s' ;
L1' et L2' sont chacun indépendamment une liaison directe, ou un groupe arylène en C₆ à C₆₀ non substitué ou substitué ;
Ar1' et Ar2' sont chacun indépendamment -CN ; un groupe alkyle en C₁ à C₆₀ non substitué ou substitué ; -SiRR'R" ; un groupe aryle en C₆ à C₆₀ non substitué ou substitué,
dans lequel la Formule chimique 3 est représentée par l'une quelconque des formules structurelles ci-après : 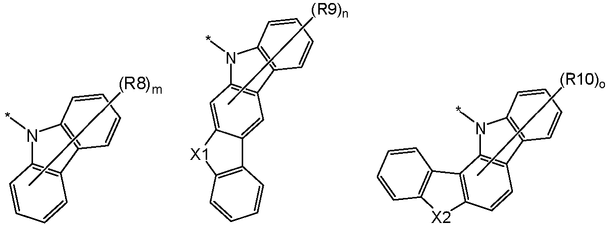 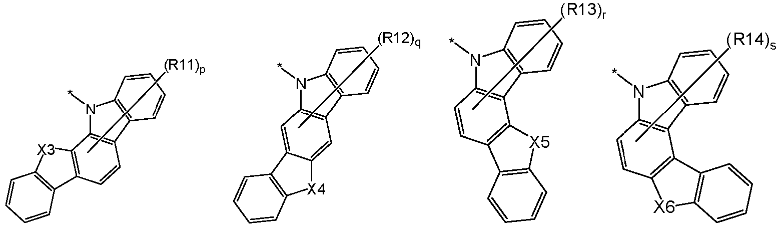
dans les formules structurelles, X1 à X6 sont identiques ou différents l'un de l'autre, et chacun indépendamment NR, S, O ou CR'R" ;
R8 à R14 sont identiques ou différents l'un de l'autre, et chacun indépendamment sélectionnés dans le groupe constitué de un hydrogène ; un deutérium ; un groupe halogène ; -CN ; un groupe alkyle en C₁ à C₆₀ non substitué ou substitué ; un groupe alkényle en C₂ à C₆₀ non substitué ou substitué ; un groupe alkynyle en C₂ à C₆₀ non substitué ou substitué ; un groupe alkoxy en C₁ à C₆₀ ; un groupe cycloalkyle en C₃ à C₆₀ non substitué ou substitué ; un groupe hétérocycloalkyle en C₂ à C₆₀ non substitué ou substitué ; un groupe aryle en C₆ à C₆₀ non substitué ou substitué ; un groupe hétéroaryle en C₂ à C₆₀ non substitué ou substitué ; -SiRR'R" ; -P(=O)RR' ; et un groupe amino non substitué ou substitué par un groupe alkyle en C₁ à C₂₀, un groupe aryle en C₆ à C₆₀ non substitué ou substitué, ou un groupe hétéroaryle en C₂ à C₆₀, ou deux groupes ou plus adjacents les uns aux autres se lient les uns aux autres pour former un cycle hydrocarboné aromatique ou aliphatique non substitué ou substitué ;
R, R' et R" sont identiques ou différents l'un de l'autre, et chacun indépendamment un hydrogène ; un deutérium ; -CN ; un groupe alkyle en C₁ à C₆₀ non substitué ou substitué ; un groupe cycloalkyle en C₃ à C₆₀ non substitué ou substitué ; un groupe aryle en C₆ à C₆₀ non substitué ou substitué ; ou un groupe hétéroaryle en C₂ à C₆₀ non substitué ou substitué ;
p' et r' sont chacun indépendamment un nombre entier de 0 à 4 ;
q' et s' sont chacun indépendamment un nombre entier de 1 à 4 ; et
m' et n' sont chacun indépendamment un nombre entier de 0 à 7,
m est un nombre entier de 0 à 8, et n, o, p, q, r et s sont chacun indépendamment un nombre entier de 0 à 6.

7. Composition pour une couche de matériau organique d'un dispositif électroluminescent organique selon la revendication 6, dans laquelle un rapport en poids de le composé hétérocyclique représenté par Formule chimique 1 :le composé représenté par Formule chimique 2 dans la composition est de 1 :10 à 10 :1.

8. Composition pour une couche de matériau organique d'un dispositif électroluminescent organique selon la revendication 6, dans laquelle la Formule chimique 1 est représentée par l'une des Formules chimiques 5 à 10 ci-après : 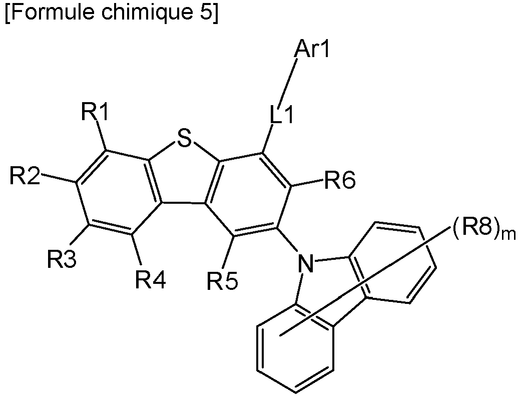 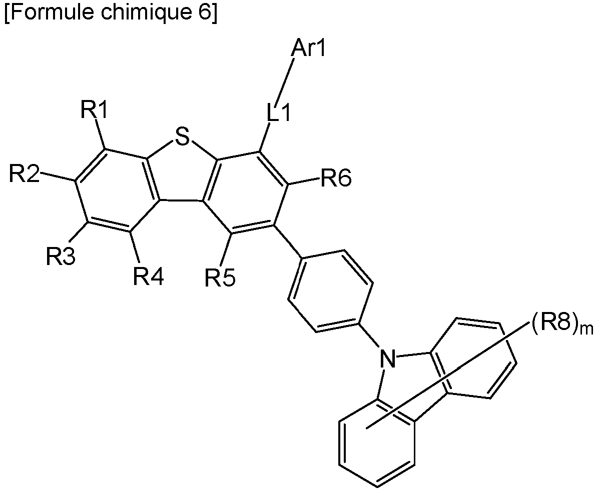 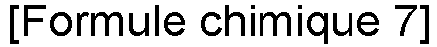 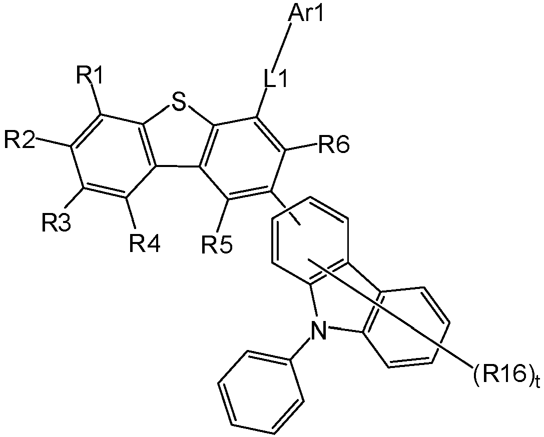 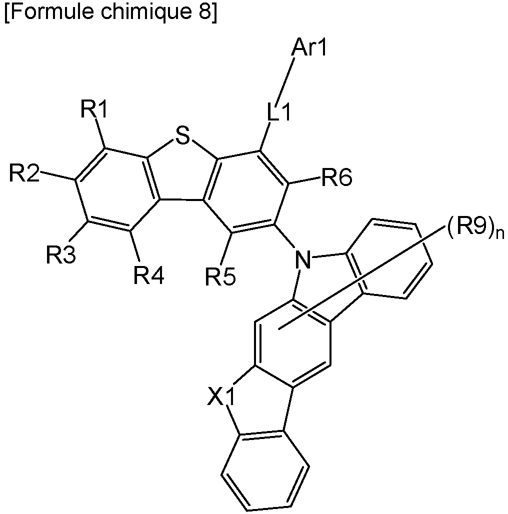 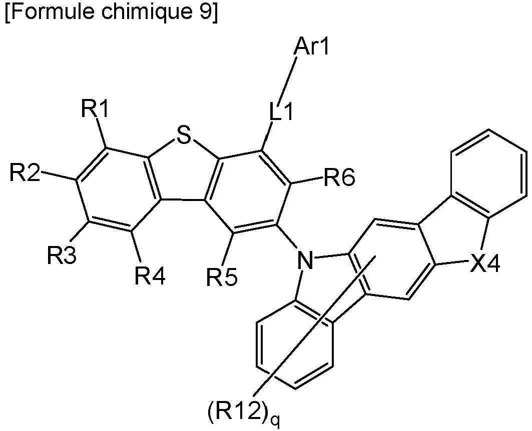 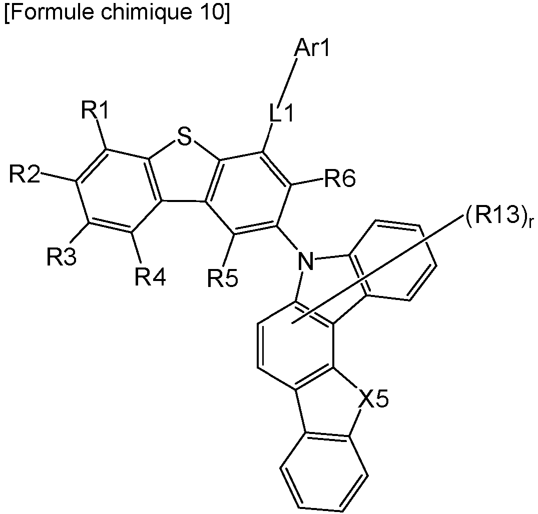 dans les Formules chimiques 5 à 10,
R1 à R6, L1 et Ar1 ont les mêmes définitions que dans la Formule chimique 1 ;
X1, X4 et X5 sont identiques ou différents l'un de l'autre, et chacun indépendamment NR, S, O ou CR'R" ;
R8, R9, R12, R13 et R16 sont identiques ou différents l'un de l'autre, et chacun indépendamment sélectionnés dans le groupe constitué de un hydrogène ; un deutérium ; un groupe halogène ; -CN ; un groupe alkyle en C₁ à C₆₀ non substitué ou substitué ; un groupe alkényle en C₂ à C₆₀ non substitué ou substitué ; un groupe alkynyle en C₂ à C₆₀ non substitué ou substitué ; un groupe alkoxy en C₁ à C₆₀ non substitué ou substitué ; un groupe cycloalkyle en C₃ à C₆₀ non substitué ou substitué ; un groupe hétérocycloalkyle en C₂ à C₆₀ ; un groupe aryle en C₆ à C₆₀ non substitué ou substitué ; un groupe hétéroaryle en C₂ à C₆₀ non substitué ou substitué ; -SiRR'R" ; -P(=O)RR' ; et un groupe amino non substitué ou substitué par un groupe alkyle en C₁ à C₂₀, un groupe aryle en C₆ à C₆₀ non substitué ou substitué, ou un groupe hétéroaryle en C₂ à C₆₀, ou deux groupes ou plus adjacents les uns aux autres se lient les uns aux autres pour former un cycle hydrocarboné aromatique ou aliphatique non substitué ou substitué ;
R, R' et R" sont identiques ou différents l'un de l'autre, et chacun indépendamment un hydrogène ; un deutérium ; -CN ; un groupe alkyle en C₁ à C₆₀ non substitué ou substitué ; un groupe cycloalkyle en C₃ à C₆₀ ; un groupe aryle en C₆ à C₆₀ non substitué ou substitué ; ou un groupe hétéroaryle en C₂ à C₆₀ non substitué ou substitué ; et
m est un nombre entier de 0 à 8, n, q et r sont chacun indépendamment un nombre entier de 0 à 6, et t est un nombre entier de 0 à 7.
